(19) Europäisches Patentamt
European Patent Office
Office européen des brevets

(11) Veröffentlichungsnummer: **0 447 506 B1**

(12) # EUROPÄISCHE PATENTSCHRIFT

(45) Veröffentlichungstag der Patentschrift: **26.10.94**

(51) Int. Cl.5: **C07D 405/12**, C07D 409/12, A01N 43/54, A01N 43/66

(21) Anmeldenummer: **90913234.2**

(22) Anmeldetag: **19.09.90**

(86) Internationale Anmeldenummer:
**PCT/CH90/00222**

(87) Internationale Veröffentlichungsnummer:
**WO 91/05781 (02.05.91 91/10)**

(54) **PYRIMIDIN- UND TRIAZIN-DERIVATE MIT HERBIZIDER UND PFLANZENWACHSTUMSREGULIERENDER WIRKUNG.**

(30) Priorität: **12.10.89 CH 3716/89**

(43) Veröffentlichungstag der Anmeldung:
**25.09.91 Patentblatt 91/39**

(45) Bekanntmachung des Hinweises auf die Patenterteilung:
**26.10.94 Patentblatt 94/43**

(84) Benannte Vertragsstaaten:
**CH DE ES FR GB IT LI**

(56) Entgegenhaltungen:
**EP-A- 0 145 078**
**EP-A- 0 287 079**
**EP-A- 0 299 446**
**EP-A- 0 336 494**

(73) Patentinhaber: **CIBA-GEIGY AG**
**Klybeckstrasse 141**
**CH-4002 Basel (CH)**

(72) Erfinder: **LÜTHY, Christoph**
**Gfennstrasse 43**
**CH-8603 Schwerzenbach (CH)**
Erfinder: **OBRECHT, Jean-Pierre**
**Sempacherstrasse 50**
**CH-8032 Zürich (CH)**

(74) Vertreter: **Roth, Bernhard M. et al**
**Patentabteilung CIBA-Geigy AG,**
**Postfach**
**CH-4002 Basel (CH)**

EP 0 447 506 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft heterocyclische Verbindungen, und zwar 2-Heterocyclyloxy/thio-pyrimidine und -1,3,5-triazine der allgemeinen Formel

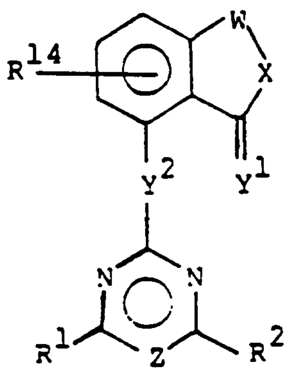

I

worin

W eine der Zweiwertigen Gruppen a) - d)

a) 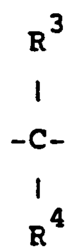

b) 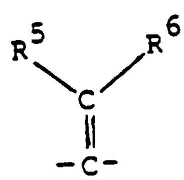

c) 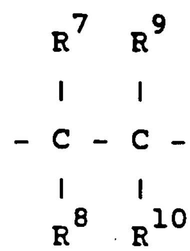

d) 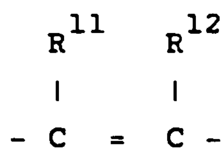

| | |
|---|---|
| X, $Y^1$ und $Y^2$ | jeweils Sauerstoff oder Schwefel. |
| Z | $CR^{13}$ oder Stickstoff. |
| $R^1$ | Wasserstoff, Fluor, Chlor, $C_{1-3}$-Alkyl, Halogenmethyl, Methoxymethyl, $C_{1-3}$-Alkoxy, Difluormethoxy oder Methylthio. |
| $R^2$ | Methyl, $C_{1-2}$-Alkoxy, $C_{1-2}$-Fluoralkoxy, $C_{1-2}$-Alkylamino, Di($C_{1-2}$-alkyl)amino oder N-Methoxymethylamino. |
| $R^3$ | Wasserstoff, Fluor, Chlor, Brom, gegebenenfalls substituiertes $C_{1-6}$-Alkyl. $C_{2-3}$-Alkenyl, $C_{2-3}$-Alkinyl, gegebenenfalls substituiertes Phenyl, Hydroxy, gegebenenfalls substituiertes $C_{1-6}$-Alkoxy, $C_{1-6}$-Alkylthio, Phenoxy, Phenylthio, Cyano, Rhodano, Formyl, Carboxy, $C_{2-5}$-Alkoxycarbonyl, Carbamoyl, Formyloxy, $C_{2-5}$-Alkanoyloxy, $C_{2-5}$-Alkoxycarbonyloxy, $C_{2-3}$-Alkylcarbamoyloxy, Di($C_{1-2}$-alkyl)carbamoyloxy oder Di($C_{1-2}$-alkoxy)phosphonyl, |
| $R^4$ | Wasserstoff, $C_{1-6}$-Alkyl oder Trifluormethyl, |
| $R^5$ | Wasserstoff, $C_{1-6}$-Alkyl oder gegebenenfalls substituiertes Phenyl, |
| $R^6$ | Wasserstoff oder Methyl, |
| $R^7$, $R^8$ und $R^9$ | unabhängig voneinander Wasserstoff oder $C_{1-3}$-Alkyl, |
| $R^{10}$ | Wasserstoff oder $C_{1-3}$-Alkoxy, |

$R^{11}$ und $R^{12}$ unabhängig voneinander Wasserstoff oder $C_{1-3}$-Alkyl,
$R^{13}$ Wasserstoff, Fluor, Chlor oder Methyl
und
$R^{14}$ Wasserstoff, Halogen, $C_{1-2}$-Alkyl oder $C_{1-2}$-Alkoxy bedeuten.

Die erfindungsgemässen Verbindungen, also die Verbindungen der Formel I, sind herbizid wirksam und eignen sich als Wirkstoffe von Unkrautbekämpfungsmitteln. Des weiteren haben die erfindungsgemässen Verbindungen pflanzenwachstumsregulierende Wirkung; sie eignen sich also u.a. als Mittel zur positiven Beeinflussung des Wachstums von Nutzpflanzen. Somit umfasst die Erfindung auch Unkrautbekämpfungsmittel und pflanzenwachstumsregulierende Mittel, welche erfindungsgemässe Verbindungen als Wirkstoffe enthalten, ein Verfahren zur Herstellung dieser Verbindungen sowie die Verwendung der Verbindungen bzw. Mittel zur Bekämpfung von Unkräutern bzw. zur Pflanzenwachstumsregulation.

In der obigen Formel I umfasst "Halogen" als solches oder als Teil einer komplexeren Gruppe, z.B. Halogenmethyl, Fluor, Chlor, Brom und Jod, wobei im allgemeinen Fluor und Chlor bevorzugt sind. Die Alkyl-, Alkenyl- und Alkinylreste können geradkettig oder verzweigt sein, wobei dies auch für den bzw. jeden Alkylteil der Alkoxy-, Alkylthio-, Alkoxycarbonyl- und weiterer Alkyl-enthaltener Gruppen gilt. Die bevorzugten $C_{2-3}$-Alkenyl- und -Alkinylgruppen sind Vinyl bzw. Aethinyl. Eine Halogenmethyl- oder Fluoralkoxygruppe kann ein oder mehrere Fluoratome aufweisen, wobei als Beispiele solcher Gruppen Chlormethyl, Trifluormethyl und Difluormethoxy genannt seien. Bei gegebenenfalls substituiertem $C_{1-6}$-Alkyl ($R^3$) handelt es sich insbesondere um eine Alkylgruppe, die mit Halogen (insbesondere Chlor), Hydroxy, Methoxy, Aethoxy, Nitro, Cyano, Vinyl, Aethinyl, Carboxy, $C_{2-5}$-Alkoxycarbonyl (insbesondere Methoxy- oder Aethoxycarbonyl) oder einer gegebenenfalls (insbesondere mit Methoxy) substituierten Phenylgruppe substituiert sein kann. Die bevorzugte gegebenenfalls substituierte Alkylgruppe ist gegebenenfalls substituiertes Methyl oder Aethyl, insbesondere die erstere Gruppe. Bei gegebenenfalls substituiertem $C_{1-6}$-Alkoxy ($R^3$) handelt es sich insbesondere um eine Alkoxygruppe, die mit Halogen (inbesondere Fluor oder Chlor), Vinyl, Aethinyl, Cyclopropyl, Phenyl, $C_{1-2}$-Alkoxy, $C_{1-2}$-Alkylthio, Cyano, Carboxy, $C_{2-5}$-Alkoxycarbonyl(insbesondere Methoxy- oder Aethoxycarbonyl), Carbamoyl, N-($C_{1-2}$-Alkyl)carbamoyl, N,N-Di($C_{1-2}$-alkyl)-carbamoyl oder $C_{3-5}$-Alkylideniminooxy substituiert sein kann. Eine gegebenenfalls substituierte Phenylgruppe ($R^3$, $R^5$) kann als Substituenten insbesondere Fluor, Chlor, Methyl, Methoxy oder Trifluormethyl aufweisen. Die bevorzugten $C_{2-5}$-Alkanoyloxy-, $C_{2-5}$-Alkoxycarbonyloxy-, $C_{2-3}$-Alkylcarbamoyloxy-,Di-($C_{1-2}$-alkyl)carbamoyloxy- und Di($C_{1-2}$-alkoxy)phosphonylgruppen ($R^3$) sind Acetyloxy oder Propionyloxy; Methoxycarbonyloxy oder Aethoxycarbonyloxy; Methylcarbamoyloxy; Dimethylcarbamoyloxy; bzw. Dimethoxy- phosphonyl.

Das allfällige Vorhandensein eines asymmetrischen Kohlenstoffatoms in den Verbindungen der Formel I hat zur Folge, dass die Verbindungen in optisch isomeren Formen auftreten können. Durch das Vorliegen einer allfälligen alipatischen C=C-Doppelbindung kann auch geometrische Isomerie auftreten. Die Formel I soll diese und allfällige weitere isomere Formen sowie Gemische davon umfassen.

Eine besondere Gruppe von Verbindungen der Formel I besteht aus denjenigen Verbindungen I, in denen W eine Gruppe a) bedeutet, worin $R^3$ Wasserstoff, Fluor, Chlor, Brom, gegebenenfalls substituiertes $C_{1-6}$-Alkyl (bei dem ein allfällig vorhandener Substituent insbesondere Halogen, Methoxy, Aethoxy, Nitro, Cyano, Methoxycarbonyl, Aethoxycarbonyl, Phenyl oder Methoxyphenyl ist), $C_{2-3}$-Alkenyl, $C_{2-3}$-Alkinyl, gegebenenfalls substituiertes Phenyl (bei dem ein allfällig vorhandener Substituent insbesondere Fluor, Chlor, Methyl, Methoxy oder Trifluormethyl ist), Hydroxy, $C_{1-6}$-Alkoxy, $C_{1-6}$-Alkylthio, Phenoxy, Phenylthio, Cyano oder $C_{2-5}$-Alkoxycarbonyl und $R^4$ Wasserstoff oder $C_{1-6}$-Alkyl, oder $R^3$ Wasserstoff, Fluor, Chlor, Brom, $C_{1-6}$-Alkyl, Hydroxy, $C_{1-6}$-Alkoxy, $C_{1-6}$-Alkylthio, Phenoxy, Phenylthio oder Cyano und $R^4$ Trifluormethyl bedeuten; oder W eine Gruppe b), c) oder d) bedeutet, worin $R^5$-$R^{12}$ die oben angegebenen Bedeutungen besitzen; und X Sauerstoff, $Y^1$ und $Y^2$ jeweils Sauerstoff oder Schwefel, Z $CR^{13}$ oder Stickstoff, $R^1$ Fluor, Chlor, $C_{1-3}$-Alkyl, Fluormethyl, Methoxymethyl, $C_{1-3}$-Alkoxy, Difluormethoxy oder Methylthio, $R^2$ Methyl, $C_{1-2}$-Alkoxy oder $C_{1-2}$-Fluoralkoxy, $R^{13}$ Wasserstoff, Fluor, Chlor oder Methyl und $R^{14}$ Wasserstoff bedeuten.

Unabhängig voneinander bedeuten W vorzugsweise eine Gruppe a) oder b), insbesondere eine Gruppe a); X und/oder $Y^1$, insbesondere aber sowohl X als auch $Y^1$, vorzugsweise Sauerstoff; $Y^2$ vorzugsweise Sauerstoff; Z vorzugsweise CH oder Stickstoff, insbesondere CH; $R^1$ vorzugsweise Wasserstoff, Chlor, Methyl, Methoxy oder Difluormethoxy und $R^2$ vorzugsweise Methoxy, Aethoxy, Methylamino, Dimethylamino oder N-Methoxymethylamino, wobei die Kombination $R^1$ und $R^2$, bei der mindestens eine Methoxygruppe vorhanden ist, besonders bevorzugt ist; $R^3$ der Gruppe a) vorzugsweise Wasserstoff, Vinyl, Aethinyl, Hydroxy, $C_{1-4}$-Alkoxy, mit Halogen, Vinyl, Aethinyl, $C_{1-2}$-Alkoxy, Cyano, Carboxy oder $C_{2-3}$-Alkoxycarbonyl substituiertes $C_{1-2}$-Alkoxy, $C_{1-2}$-Alkylthio, Cyano, Carboxymethyl, $C_{2-3}$-Alkoxycarbonylmethyl oder Carbamoyl und $R^4$ vorzugsweise Wasserstoff oder $C_{1-4}$-Alkyl; $R^5$ der Gruppe b) vorzugsweise Wasserstoff

oder $C_{1-3}$-Alkyl und $R^6$ vorzugsweise Wasserstoff; $R^7$, $R^8$ und $R^9$ der Gruppe c) vorzugsweise jeweils Wasserstoff oder Methyl; $R^{11}$ und $R^{12}$ der Gruppe d) vorzugsweise jeweils Wasserstoff oder Methyl; und $R^{14}$ vorzugsweise Wasserstoff oder Methyl, insbesondere Wasserstoff.

Besonders bevorzugte einzelne Verbindungen der Formel I sind:
7-[(4,6-Dimethoxy-pyrimidin-2-yl)oxy]-3-methyl-phthalid,
7-[(4,6-Dimethoxy-pyrimidin-2-yl)oxy]-phthalid,
3-Aethyl-7-[(4,6-dimethoxy-pyrimidin-2-yl)oxy]-phthalid,
7-[(4,6-Dimethoxy-pyrimidin-2-yl)oxy]-3-isopropyl-phthalid,
7-[(4,6-Dimethoxy-pyrimidin-2-yl)oxy]-3-methoxy-phthalid,
7-[(4-Methoxy-6-methyl-pyrimidin-2-yl)oxy]-3-methyl-phthalid,
7-[(4,6-Dimethoxy-1,3,5-triazin-2-yl)oxy]-3-methyl-phthalid,
3-Aethyliden-7-[(4,6-dimethoxy-pyrimidin-2-yl)oxy]phthalid (insbesondere dessen (Z)-Isomeres),
8-[(4,6-Dimethoxy-pyrimidin-2-yl)oxy]-3-methyl-isochroman -1-on,
7-[(4,6-Dimethoxy-pyrimidin-2-yl)thio]-3-methyl-phthalid,
3-Aethoxy-7-[(4,6-dimethoxy-pyrimidin-2-yl)oxy]-phthalid,
7-[(4,6-Dimethoxy-pyrimidin-2-yl)oxy]-3,6-dimethyl-phthalid,
7-[(4,6-Dimethoxy-pyrimidin-2-yl)oxy]-3-methoxy-3-methyl-phthalid,
3-Carbamoyl-7-[(4,6-dimethoxy-pyrimidin-2-yl)oxy]phthalid,
3-(2-Chloräthoxy)-7-[(4,6-dimethoxy-pyrimidin-2-yl)oxy]phthalid,
7-[(4,6-Dimethoxy-pyrimidin-2-yl)oxy]-3-propargyloxy-phthalid,
7-[(4,6-Dimethoxy-pyrimidin-2-yl)oxy]-3-(n-propoxy)phthalid,
7-[(4,6-Dimethoxy-pyrimidin-2-yl)oxy]-3-(2-methoxyäthoxy)-phthalid,
7-[(4-Chlor-6-methoxy-pyrimidin-2-yl)oxy]-3-methyl-phthalid,
7-[(4-Methoxy-pyrimidin-2-yl)oxy]-3-methyl-phthalid,
7-[(4-Aethoxy-6-methoxy-pyrimidin-2-yl)oxy]-3-methyl-phthalid,
7-[(4-Chlor-6-methoxy-pyrimidin-2-yl)oxy]-3-methoxy-phthalid,
7-[(4,6-Dimethoxy-1,3,5-triazin-2-yl)thio]-3-methyl-phthalid,
7-[(4-Dimethylamino-6-methoxy-1,3,5-triazin-2-yl)oxy]-3-methyl-phthalid,
7-[(4-Methoxy-6-methyl-1,3,5-triazin-2-yl)oxy]-3-methyl-phthalid,
7-[(4-Methoxy-6-methylamino-1,3,5-triazin-2-yl)oxy]-3-methyl-phthalid,
7-[(4-Chlor-6-methylamino-1,3,5-triazin-2-yl)oxy]-3-methyl-phthalid,
3-Aethyl-7-[(4,6-dimethoxy-1,3,5-triazin-2-yl)oxy]phthalid,
8-[(4,6-Dimethoxy-pyrimidin-2-yl)oxy]-3,4-dimethyl-isochroman-2-on,
7-[(4,6-Dimethoxy-pyrimidin-2-yl)oxy]-3-hydroxy-phthalid,
7-[(4,6-Dimethoxy-pyrimidin-2-yl)oxy]-3-methylthio-phthalid,
7-[(4,6-Dimethoxy-pyrimidin-2-yl)oxy]-3-vinyl-phthalid,
3-Cyano-7-[(4,6-dimethoxy-pyrimidin-2-yl)oxy]-phthalid,
3-Cyanomethoxy-7-[(4,6-dimethoxy-pyrimidin-2-yl)oxy]phthalid,
7-[(4,6-Dimethoxy-pyrimidin-2-yl)oxy]-3-(methoxycarbonylmethoxy)-phthalid,
3-Aethoxycarbonylmethyl-7-[(4,6-dimethoxy-pyrimidin-2-yl)oxy]-phthalid,
7-[(4,6-Dimethoxy-pyrimidin-2-yl)oxy]-3-methyl-2-benzothiophen-1(3H)-on,
7-[(4,6-Dimethoxy-pyrimidin-2-yl)oxy]-3-methyl-isobenzofuran-1(3H)-thion,
7-[(4,6-Dimethoxy-1,3,5-triazin-2-yl)oxy]-3-methyl-isobenzofuran-1(3H)-thion,
7-[(4-Difluormethoxy-6-methoxy-pyrimidinyl)oxy]-3-methyl-phthalid,
8-[(4,6-Dimethoxy-pyrimidin-2-yl)oxy]-4-methylisochroman-1-on und
3-Acetoxy-7-[(4,6-dimethoxy-pyrimidin-2-yl)oxy]-phthalid.

Weitere Vertreter von Verbindungen der Formel I sind:
7-[(4,6-Dimethoxy-pyrimidin-2-yl)oxy]-3-isobutyl-phthalid,
7-[(4,6-Dimethoxy-pyrimidin-2-yl)oxy]-3-sek.butyl-phthalid,

diejenigen Verbindungen der Formel I, in denen W eine Gruppe a), X, $Y^1$ und $Y^2$ alle Sauerstoff, Z CH, $R^1$ und $R^2$ beide Methoxy, $R^4$ und $R^{14}$ beide Wasserstoff und $R^3$ Brom, Chlormethyl, Trichlormethyl, Hydroxymethyl, Methoxymethyl, Cyanomethyl, Carboxymethyl, Methoxycarbonylmethyl, Allyl, Aethinyl, Propargyl, n-Butoxy Cyclopropylmethoxy, Difluormethoxy, 2,2,2-Trifluoräthoxy, Methoxymethoxy, Methylthiomethoxy, 2-Methylthio-äthoxy, Carboxymethoxy, 1-Carboxyäthoxy, 1-Methoxycarbonyl-äthoxy, Aethoxycarbonylmethoxy, 1-Aethoxycarbonyl-äthoxy, N-Methylcarbamoylmethoxy, 2-(N,N-Dimethylamino)-äthoxy, Formyl, Carboxy, Aethoxycarbonyl, Formyloxy, Acetyloxy, Methoxycarbonyloxy, Aethoxycarbonyloxy oder N,N-Dimethylcarbamoyloxy bedeuten;

diejenigen Verbindungen der Formel I, in denen W eine Gruppe a), X, $Y^1$ und $Y^2$ alle Sauerstoff, Z CH,

$R^1$ und $R^2$ beide Methoxy, $R^4$ Methyl, $R^{14}$ Wasserstoff und $R^3$ Fluor, Vinyl, Hydroxy, Aethoxy, Methylthio, carboxy oder Methoxycarbonyl bedeuten;

3-Aethyl-7-[(4,6-dimethyl-pyrimidin-2-yl)oxy]-3-methoxy-phthalid,

7-[(4,6-Dimethyl-pyrimidin-2-yl)oxy]-3-methoxy-3-trifluormethyl-phthalid,

3-Aethoxy-7-[(4,6-dimethyl-pyrimidin-2-yl)oxy]-3-trifluormethyl-phthalid,

diejenigen Verbindungen der Formel I, in denen W eine Gruppe a), X, $Y^1$ und $Y^2$ alle Sauerstoff, Z CH, $R^1$ und $R^2$ beide Methoxy, $R^3$ Methyl, $R^4$ Wasserstoff und $R^{14}$ 4-Fluor, 5-Fluor, 6-Fluor, 6-Chlor, 4-Methyl, 5-Methyl, 4-Methoxy, 5-Methoxy oder 6-Methoxy bedeuten;

diejenigen Verbindungen der Formel I, in denen W eine Gruppe a), X, $Y^1$ und $Y^2$ alle Sauerstoff, Z CH, $R^2$ Methoxy, $R^3$ Methyl, $R^4$ und $R^{14}$ beide Wasserstoff und $R^1$ Fluor, Aethyl oder Methylthio bedeuten;

diejenigen Verbindungen der Formel I, in denen W eine Gruppe a), X, $Y^1$ und $Y^2$ alle Sauerstoff, Z CH, $R^1$ Methoxy, $R^3$ Methyl, $R^4$ und $R^{14}$ beide Wasserstoff und $R^2$ Methylamino, Dimethylamino oder Aethylamino bedeuten;

diejenigen Verbindungen der Formel I, in denen W eine Gruppe a), X, $Y^1$ und $Y^2$ alle Sauerstoff, Z Stickstoff, $R^1$ und $R^2$ beide Methoxy, $R^4$ und $R^{14}$ beide Wasserstoff und $R^3$ Wasserstoff, Aethyl, n-Propyl oder n-Butyl bedeuten;

7-[(4-Methoxy-6-{N-methoxymethylamino}-1,3,5-triazin-2-yl)oxy]-3-methyl-phthalid,

7-[(4,6-Dimethoxy-1,3,5-triazin-2-yl)oxy]-3-methoxy-3-methyl-phthalid,

7-[(4,6-Dimethyl-1,3,5-triazin-2-yl)oxy]-3-methyl-phthalid,

diejenigen Verbindungen der Formel I, in denen W eine Gruppe a), X, $Y^1$ und $Y^2$ alle Sauerstoff, Z CH, $R^1$ und $R^2$ beide Difluormethoxy, $R^3$ Wasserstoff, Methoxy oder Aethoxy, $R^4$ Wasserstoff, Methyl oder Aethyl und $R^{14}$ Wasserstoff bedeuten;

diejenigen Verbindungen der Formel I, in denen W eine Gruppe a), X und $Y^1$ beide Sauerstoff, $Y^2$ Schwefel, Z CH, $R^1$ und $R^2$ beide Methoxy, $R^4$ und $R^{14}$ beide Wasserstoff und $R^3$ Wasserstoff, Aethyl, Methoxycarbonylmethyl, Hydroxy, Methoxy, Aethoxy oder Methoxycarbonyloxy bedeuten;

7-[(4,6-Dimethoxy-pyrimidin-2-yl)thio]-3-methyl-isobenzofuran-1(3H)-thion,

7-[(4,6-Dimethoxy-pyrimidin-2-yl)thio]-3-methyl-2-benzothiophen-1(3H)-on,

7-[(4,6-Dimethoxy-pyrimidin-2-yl)oxy]-3-methyl-2-benzothiophen-1(3H)-thion,

3-Aethyl-7-[(4,6-dimethoxy-pyrimidin-2-yl)oxy]isobenzofuran-1(3H)-thion,

7-[(4,6-Dimethoxy-pyrimidin-2-yl)oxy]-3-methoxyisobenzofuran-1(3H)-thion,

3-Aethyl-7-[(4,6-dimethoxy-pyrimidin-2-yl)oxy]-2-benzothiophen-1(3H)-on,

7-[(4,6-dimethoxy-pyrimidin-2-yl)oxy]-3-methoxy-2-benzothiophen-1(3H)-on,

7-[(4,6-Dimethyl-pyrimidin-2-yl)thio]-3-methyl-phthalid,

7-[(4-Methoxy-6-methyl-pyrimidin-2-yl)thio]-3-methyl-phthalid,

7-[(5-Chlor-4,6-dimethoxy-pyrimidin-2-yl)thio]-3-methyl-phthalid,

7-[(4,6-Dimethoxy-5-fluor-pyrimidin-2-yl)oxy]-3-methyl-phthalid,

7-[(4,6-Dimethoxy-5-methyl-pyrimidin-2-yl)oxy]-3-methyl-phthalid,

7-[(5-Chlor-4,6-dimethoxy-pyrimidin-2-yl)oxy]-3-methyl-phthalid,

7-[(4,6-Dimethoxy-pyrimidin-2-yl)oxy]-3-methyliden-phthalid,

7-[(4,6-Dimethoxy-pyrimidin-2-yl)oxy]-3-propyliden-phthalid,

3-Butyliden-7-[(4,6-dimethoxy-pyrimidin-2-yl)oxy]phthalid,

3-Aethyliden-7-[(4,6-dimethoxy-1,3,5-triazin-2-yl)oxy]phthalid,

3-Methyliden-7-[(4,6-dimethoxy-1,3,5-triazin-2-yl)oxy]phthalid,

diejenigen Verbindungen der Formel I, in denen W eine Gruppe c), X, $Y^1$ und $Y^2$ alle Sauerstoff, Z CH, $R^1$ und $R^2$ beide Methoxy, $R^7$, $R^8$ und $R^9$ alle Wasserstoff, $R^{14}$ Wasserstoff und $R^{10}$ Wasserstoff, Methoxy oder Aethoxy bedeuten;

8-[(4-Methoxy-6-methyl-pyrimidin-2-yl)oxy]-4-methyl-isochroman-1-on,

8-[(4,6-Dimethyl-pyrimidin-2-yl)thio]-3-methyl-isochroman-1-on,

8-[(4,6-Dimethoxy-1,3,5-triazin-2-yl)oxy]-isochroman-1-on,

8-[(4,6-Dimethoxy-1,3,5-triazin-2-yl)oxy]-4-methyl-isochroman-1-on,

8-[(4,6-Dimethoxy-pyrimidin-2-yl)oxy]-4-methyl-isochromen-1-on,

4-Aethyl-8-[(4,6-dimethoxy-pyrimidin-2-yl)oxy]-isochromen -1-on,

8-[(4,6-Dimethoxy-pyrimidin-2-yl)oxy]-3,4-dimethyl-isochromen-1-on und

8-[(4,6-Dimethoxy-1,3,5-triazin-2-yl)oxy]-3-methyl-isochromen-1-on.

Das erfindungsgemässe Verfahren zur Herstellung der Verbindungen der Formel I ist dadurch gekennzeichnet, dass man eine Verbindung der allgemeinen Formel

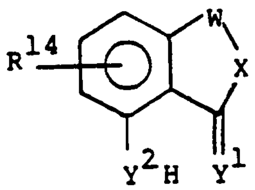

II

worin W, X, $Y^1$, $Y^2$ und $R^{14}$ die oben angegebenen Bedeutungen besitzen. mit einer Verbindung der allgemeinen Formel

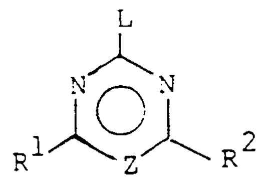

III

worin $R^1$, $R^2$ und Z die oben angegebenen Bedeutungen besitzen und L eine Abgangsgruppe bedeutet, umsetzt.

Vorzugsweise werden nach dem erfindungsgemäßen Verfahren diejenigen Verbindungen der Formel I hergestellt,
worin W eine Gruppe a)
und $R_3$ Wasserstoff, Fluor, Chlor, Brom, $C_{1-6}$-Alkyl, $C_{2-3}$-Alkenyl, $C_{2-3}$-Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, $C_{1-6}$-Alkylthio, Phenoxy, Phenylthio, Cyano oder $C_{2-5}$-Alkoxycarbonyl und $R^4$ Wasserstoff oder $C_{1-6}$-Alkyl bedeuten, oder $R^3$ Wasserstoff, Fluor, Chlor, Brom, $C_{1-6}$-Alkyl, Hydroxy, $C_{1-6}$-Alkoxy, $C_{1-6}$-Alkylthio, Phenoxy, Phenylthio oder Cyano und $R^4$ Trifluormethyl bedeuten; oder W eine Gruppe b), c) oder d) bedeutet, worin $R^5$, $R^6$, $R^7$, $R^8$, $R^9$, $R^{10}$, $R^{11}$ und $R^{12}$ die oben angegebenen Bedeutungen besitzen; und X Sauerstoff bedeutet, $Y^1$, $Y^2$ und Z die in Anspruch 19 angegebenen Bedeutungen besitzen und $R^1$ Fluor, Chlor, $C_{1-3}$-Alkyl, Fluormethyl, Methoxymethyl, $C_{1-3}$-Alkoxy, Difluormethoxy oder Methylthio, $R^2$ Methyl, $C_{1-2}$-Alkoxy oder $C_{1-2}$_Fluoralkoxy, $R^{13}$ Wasserstoff, Fluor, Chlor oder Methyl und $R^{14}$ Wasserstoff bedeuten.

Unter dem Ausdruck "Abgangsgruppe" (L) ist insbesondere ein Halogenatom, vorzugsweise Chlor oder Brom, oder eine jeweils gegebenenfalls substituierte Alkylthio-, Benzylthio-, Phenylthio-, Alkylsulfinyl-, Benzylsulfinyl-, Phenylsulfinyl-, Alkylsulfonyl-, Benzylsulfonyl- Phenylsulfonyl-, Alkylsulfonyloxy-, Benzylsulfonyloxy-, Phenylsulfonyloxy- oder 3-Alkylsulfonyl-1H-1,2,4-triazol -1-ylgruppe (z.B. 3-Methylsulfonyl-1H-1,2,4-triazol-1-yl) zu verstehen. Unter derartigen schwefelhaltigen Abgangsgruppen L sind Methansulfonyl, Aethansulfonyl und Benzylsulfonyl besonders bevorzugt.

Die Umsetzung erfolgt zweckmässigerweise in einem inerten Verdünnungsmittel, in Gegenwart einer Base oder eines reaktionsbeschleunigenden Zusatzes, und bei Temperaturen zwischen 0 °C und 160 °C, vorzugsweise zwischen 20 °C und 100 °C bzw. dem Siedepunkt des Reaktionsgemisches. Als Verdünnungsmittel kommen insbesondere organische Lösungsmittel, vorzugsweise aprotische, wie aliphatische oder cyclische Aether, z.B. Dimethoxyathan und Tetrahydrofuran; aliphatische Ketone, z.B. Aceton und 2-Butanon; aliphatische Nitrile, z.B. Acetonitril und Propionitril; Dimethylformamid; Dimethylacetamid; und heteroaromatische Verbindungen, z.B. Pyridin und Lutidin, und als Basen insbesondere Alkalimetallhydride, z.B. Natriumhydrid und Kaliumhydrid; Erdalkalimetallhydride, z.B. Calciumhydrid; Alkalimetallhydrogencarbonate, z.B. Natriumhydrogencarbonat und Kaliumhydrogencarbonat; Alkalimetallcarbonate, z.B. Natriumcarbonat und Kaliumcarbonat; Erdalkalimetallcarbonate, z.B. Calciumcarbonat und Magnesiumcarbonat; aliphatische tertiäre Amine, z.B. Triäthylamin; vollsubstituierte Amidine, z.B. Diazabicycloundecen; und basische Heteroaromaten, z.B. Pyridin, in Betracht. Es eignen sich als reaktionsbeschleunigende Zusätze insbesondere Kronenäther und Phasentransferkatalysatoren, aber auch Substanzen, welche die Reaktion dadurch beschleunigen, dass sie die Abgangsgruppe L vorübergehend ersetzen, bzw. im Falle von L = Halogen dadurch, dass sie die Abgangsgruppe L aktivieren. Ein Beispiel der erstgenannten Substanzen ist Dimethylaminopyridin. Beispiele der zweitgenannten Substanzen sind Silber- und Kupfersalze, wie Silbernitrat und

Kupfer(I)chlorid.

Weitere Verfahren zur Herstellung derjenigen erfindungsgemässen Verbindungen der Formel I, in denen W eine Gruppe a) oder b) und X und $Y^1$ jeweils Sauerstoff bedeuten, bestehen darin, eine Verbindung der allgemeinen Formel

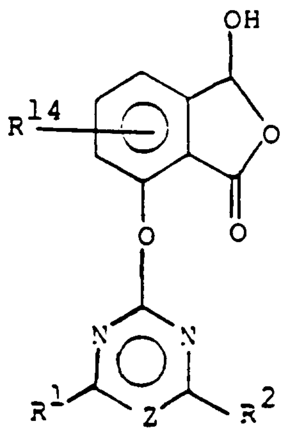

I'

worin $R^1$, $R^2$, $R^{14}$ und Z die oben angegebenen Bedeutungen besitzen,
einer entsprechenden Alkylierungs-, Acylierungs-, Carbonylierungs-, Carbamoylierungs-, Halogenierungs-, Substitution-, Grignard- bzw. Wittig-Reaktion zu unterwerfen, und zwar analog den nachfolgend beschriebenen diesbezüglichen Methoden, die im Zusammenhang mit der Herstellung der Ausgangsmaterialien der Formel II angewendet werden.

Ferner können diejenigen Verbindungen der Formel I, in denen X und/oder $Y^1$ Schwefel bedeutet, durch Schwefelung der entsprechenden Verbindungen I, in denen X und/oder $Y^1$ Sauerstoff bedeutet, hergestellt werden

Die erhaltenen Verbindungen der Formel I können nach an sich bekannten Methoden isoliert und gereinigt werden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II sind teilweise neu, teilweise bekannt. So sind beispielsweise 7-Hydroxyphthalid und 3,7-Dihydroxy-phthalid (Verbindungen der Formel II, worin W Methylen bzw. Hydroxymethylen, X, $Y^1$ und $Y^2$ jeweils Sauerstoff und $R^{14}$ Wasserstoff bedeuten) aus E.L. Eliel et al., J. Org. Chem. 18, 1679 ff. (1953) bekannt geworden. Des weiteren ist die Herstellung von 7-Hydroxy-3-methyl-phthalid und 3-Aethyl-7-hydroxy-phthalid in S. Kushner et al., J.A.C.S. 75, 1097 ff. (1953) bzw. J. Blair und G.T. Newbold. J. Org. Chem. 1955, 2871 ff. beschrieben. Ebenfalls bekannt geworden ist ein Zugang zum 3,7-Dihydroxy-3-methyl-phthalid [siehe Z. Horii et al.,J. Pharm. Soc. Japan 74, 466 ff. (1954)]. Die neuen Ausgangsmaterialien der Formel II können nach an sich bekannten Methoden hergestellt werden.

Diejenigen Ausgangsmaterialien der Formel II, in denen W eine Gruppe a) oder b) und X und $Y^1$ beide Sauerstoff bedeuten, können in an sich bekannter Weise. z.B. gemäss den nachfolgenden Reaktionsschemata 1 und 2, hergestellt werden:

Reaktionsschema 1

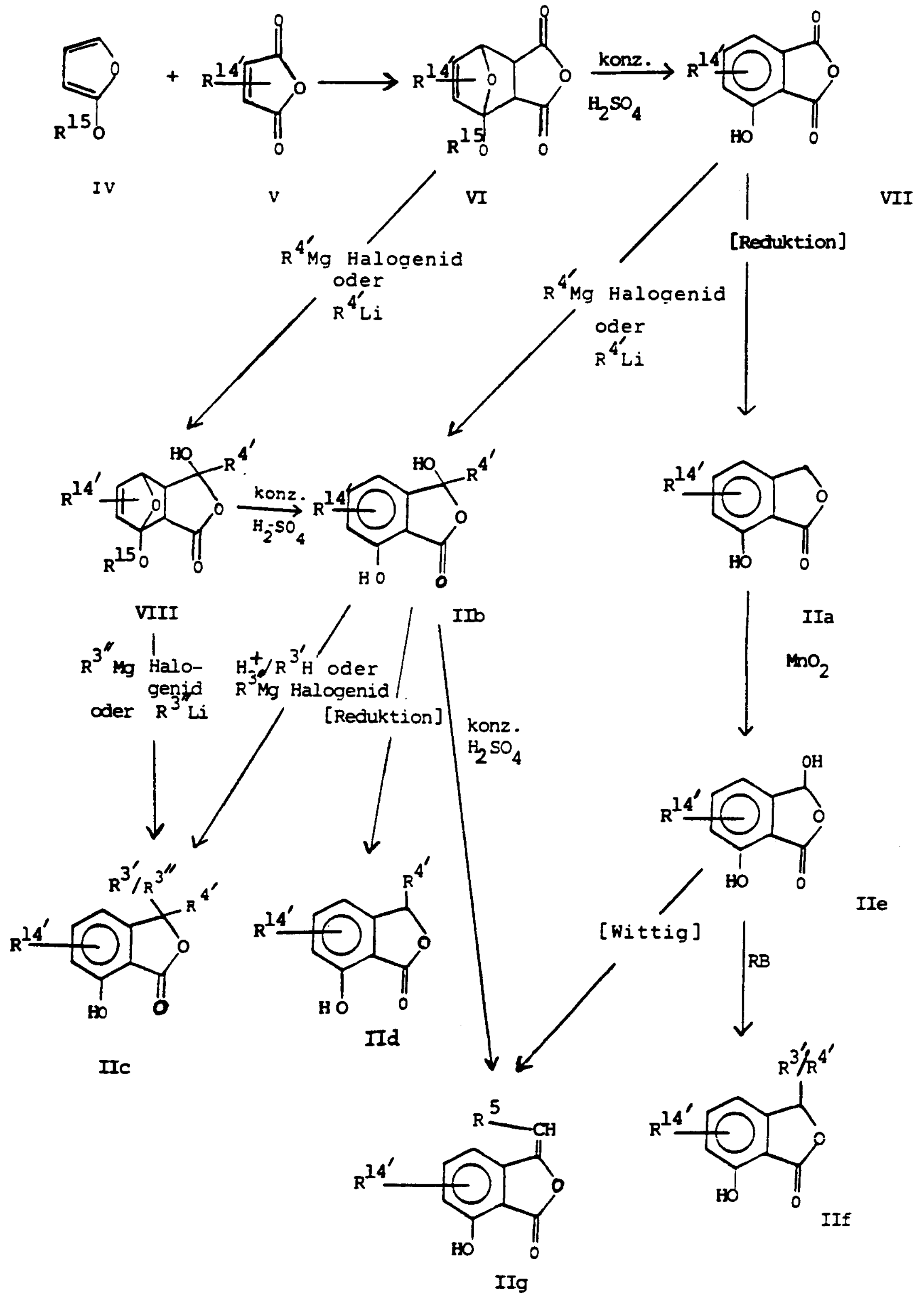

Reaktionsschema 2

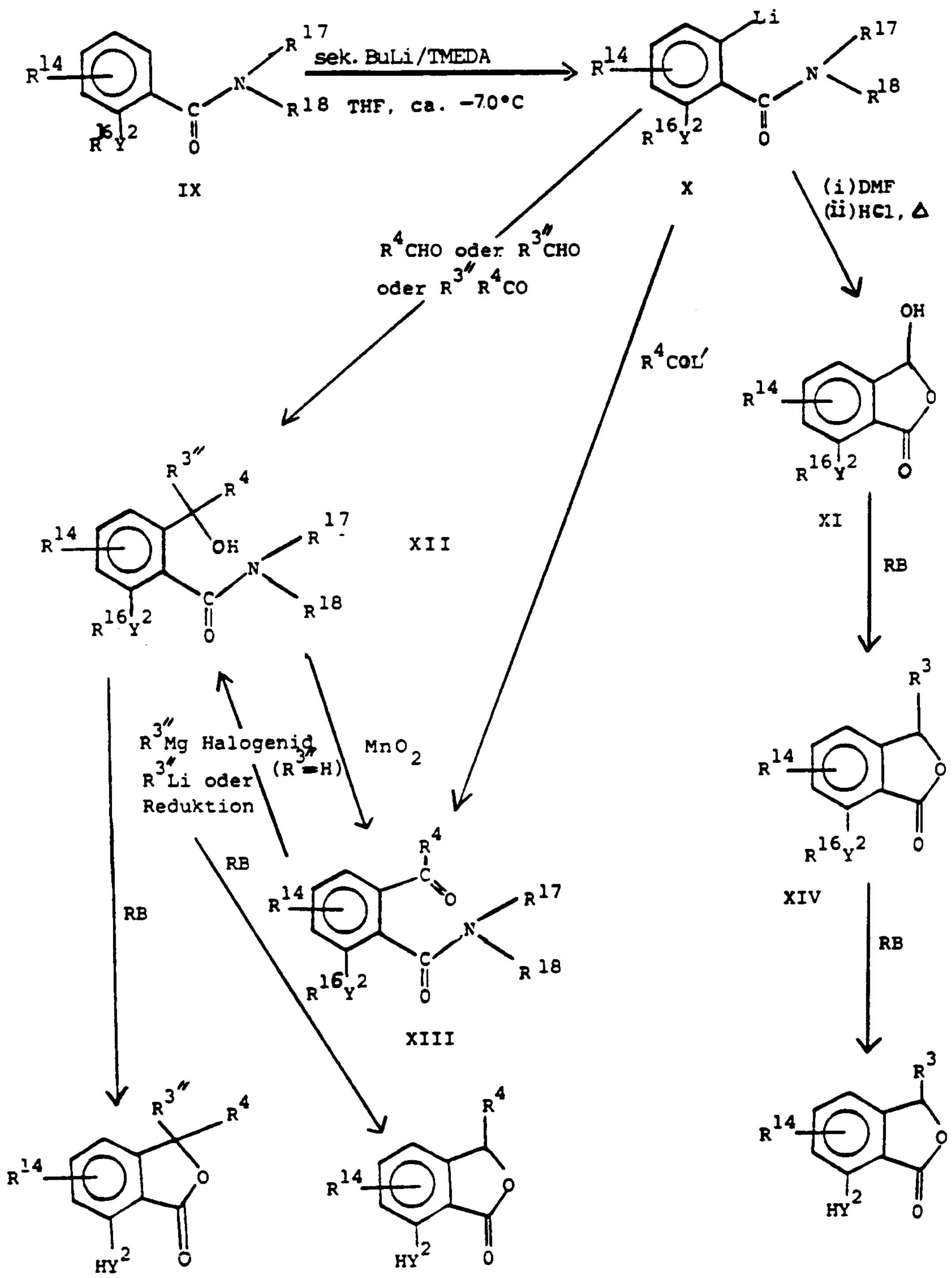


IX X XI XII XIII XIV IIh IIi IIj

In den obigen Reaktionsschemata 1 und 2 besitzen $Y^2$, $R^3$, $R^4$, $R^5$ und $R^{14}$ die oben angegebenen Bedeutungen; $R^{3'}$ bedeutet gegebenenfalls substituiertes $C_{1-6}$-Alkyl, $C_{2-3}$-Alkenyl, $C_{2-3}$-Alkinyl, gegebenenfalls substituiertes Phenyl oder gegebenenfalls substituiertes $C_{1-6}$-Alkoxy; $R^{3''}$ bedeutet gegebenenfalls substituiertes $C_{1-6}$-Alkyl, $C_{2-3}$-Alkenyl, $C_{2-3}$-Alkinyl oder gegebenenfalls substituiertes Phenyl; $R^{4'}$ bedeutet $C_{1-6}$-Alkyl; $R^{14'}$ bedeutet Wasserstoff oder $C_{1-2}$-Alkyl; $R^{15}$ bedeutet Alkanoyl, insbesondere $C_{2-5}$-

Alkanoyl, oder eine Gruppe $-Si(CH_3)_2R^{19}$, worin $R^{19}$ für $C_{1-6}$-Alkyl steht; $R^{16}$ bedeutet Wasserstoff, Methyl, tert.Butyl oder eine in der Chemie übliche Schutzgruppe, wie beispielsweise Benzyl, p-Methoxybenzyl oder Methoxyäthoxymethyl; $R^{17}$ bedeutet Wasserstoff oder $C_{1-2}$-Alkyl und $R^{18}$ $C_{1-4}$-Alkyl, oder $R^{17}$ und $R^{18}$ bedeuten zusammen Tetramethylen, das mit einer Methoxy- oder Hydroxymethylgruppe substituiert sein kann.

Die einzelnen Reaktionen, wie beispielsweise die verschiedenen Grignard-Reaktionen unter Verwendung von $R^{4'}$ MgHalogenid, $R^{4'}$ Li $R^{3'}$MgHalogenid, $R^{3''}$MgHalogenid bzw. $R^{3''}$Li, die Hydrolyse mit konzentrierter Schwefelsäure ("konz. $H_2SO_4$"), die Reduktion einer Ring-Carbonylgruppe (z.B. in der Reaktionsstufe VII→IIa) oder einer Hydroxygruppe (z.B. in der Reaktionsstufe IIb→IId), die Oxidation mittels Mangandioxid ("$MnO_2$") und die Wittig-Reaktion, sind dem Fachmann geläufig. So können beispielsweise Verbindungen der Formel VII aus Verbindungen der Formel VI nach der Vorschrift von J.-A.H. Näsman, Synthesis 1985, 788, hergestellt werden (in diesem Fall bedeutet $R^{15}$ die 2,2-Dimethylpropanoylgruppe). Ferner können beispielsweise Verbindungen der Formel VII aus den entsprechenden o-Nitrophthalsäureanhyriden nach allgemein bekannten Vorschriften hergestellt werden - siehe z.B. E.L. Eliel, J.A.C.S. 77, 5092 ff. (1955). Dabei wird die Nitrogruppe vorerst reduziert und anschliessend mittels Diazotierung in die entsprechende Hydroxyverbindung übergeführt. Weitere relevante Literaturstellen bezüglich geeigneter Reaktionsbedingungen gewisser Reaktionsstufen sind J. Blair und G.T. Newbold, J. Org. Chem. 1955, 2871 ff. sowie B.L. Chenard et al., J. Org. Chem. 49, 318 ff. (1984).

Ferner bedeuten in den Reaktionsschemata 2 TMEDA Tetramethyläthylendiamin, THF Tetrahydrofuran, DMF Dimethylformamid und L' eine Abgangsgruppe, wie Chlor, Acetyl, Imidazolyl, N,O-Dimethylhydroxylamino oder Dimethylamino. Die bevorzugten Gruppen $NR^{17}R^{18}$ für die direkte Metallierung in ortho-Stellung zur Carboxamidfunktion $CONR^{17}R^{18}$ (Reaktionsstufe IX→X) sind Diäthylamino [siehe V. Snieckus, Heterocycles 14, 1649 ff. (1980)], Methylamino [siehe S.N. Yeola und R.S. Mali, Ind. J. Chem. 25B, 804 ff. (1986) sowie N.S. Narasimhan und R.S. Mali, Synthesis 1983, 957 ff.] und tert.Butylmethylamino [siehe D.B. Reitz und S.M. Massey, J. Org. Chem. 55, 1375 ff. (1990)].

In den wenigen Fällen, in denen auf die Reaktionsbedingungen (RB) nicht hingewiesen wird, insbesondere bei den Verfahrensstufen IIe→IIf und XI→XIV, hängen die Reaktionsbedingungen von der Natur der einzuführenden Gruppe $R^3$ bzw. $R^4$ ab. Falls $R^3$ gegebenenfalls substituiertes $C_{1-6}$-Alkyl, $C_{2-3}$-Alkenyl, $C_{2-3}$-Alkinyl oder gegebenenfalls substituiertes Phenyl ($R^{3''}$) bzw. $R^4$ $C_{1-6}$-Alkyl ($R^{4'}$) bedeutet, wird als $R^{3''}$ - oder $R^{4'}$ - enthaltendes Reagens zweckmässigerweise das entsprechende $R^{3''}$ Mg Halogenid bzw. $R^{4'}$ Mg Halogenid oder $R^{3''}$ Li bzw. $R^{4'}$ Li verwendet, während falls $R^3$ gegebenenfalls substituiertes $C_{1-6}$-Alkoxy bedeutet, das Ausgangsmaterial IIb bzw. IIe mit der entsprechenden Hydroxyverbindung $R^3H$ zweckmässigerweise unter Säurekatalyse, z.B. mittels Schwefelsäure. p-Toluolsulfonsäure oder Trifluoressigsäure, behandelt wird. Beispiele des ersteren Reaktionstyps sind u.a. von J. Grandguillot und F. Roussac in Synthesis 1979, 607 ff., und von P. Canonne et al. in Tetrahedron Lett. 26, 4719 (1985) beschrieben.

Bezüglich der Reaktionsstufen XII→IIh, XII→IIi und XIV→IIj ist es ferner allgemein bekannt, dass sich eine phenolische Methyläthergruppe oder die entsprechende Thioäthergruppe ($R^{16}$ bedeutet Methyl) mit Bromwasserstoff in Wasser oder Eisessig, mit Bortribromid oder mit dem Bortribromid-Dimethylsulfid-Komplex in Methylen- oder Aethylendichlorid leicht zur Hydroxy- bzw. Thiolgruppe ($R^{16}$ bedeutet Wasserstoff) spalten lässt. Weitere geeignete Spaltungsreagentien sind Aluminiumtrichlorid, Bortrichlorid, Dimethylborbromid in Methylenchlorid wie auch Alkylmercaptide, z.B. Natriumäthylmercaptid in Toluol oder Xylol.

Diejenigen Ausgangsmaterialien der Formel IIb, in denen $R^{4'}$ Methyl oder Aethyl bedeutet, und die entsprechenden geschützten Verbindungen können auch dadurch hergestellt werden, dass man ein entsprechendes Phthalsäureanhydrid mit Malonsäure oder Methylmalonsäure in Gegenwart einer Base, z.B. Triäthylamin (TEA) oder Pyridin (Py), die zudem als Verdünnungsmittel dienen kann, solange erhitzt, bis die Entwicklung von Kohlenstoffdioxid beendet ist, und zwar gemäss der nachfolgenden Gleichung:

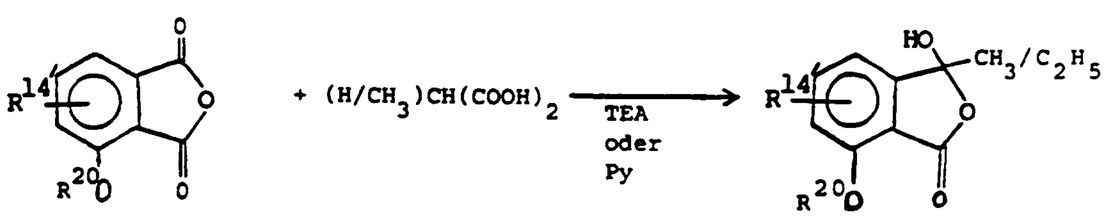


XV IIb'

worin $R^{20}$ Wasserstoff oder Methyl bedeutet.

Aus der Fachliteratur sind noch weitere Methoden zur Herstellung der Ausgangsmaterialien der Formel II bekannt geworden. Beispielsweise können die im Reaktionsschema 2 angegebenen 3-Hydroxyphthalide der Formel XI auch nach der Lehre von B.M. Trost et al.. J. Org. Chem. 45, 1835 ff. (1980), F. Hauser & R. Lee. J. Org. Chem. 45, 3061 ff. (1980), und J.N. Freskos et al., J. Org. Chem. 50, 805 ff. (1985), hergestellt, und diese dann wie oben angedeutet in die entsprechenden Ausgangsmaterialien der Formel IIg oder IIj übergeführt werden.

Um auf obige Weise zu optisch aktiven Verbindungen der Formel IIi zu gelangen, kann man eine Verbindung der Formel IIb, IIb' oder XIII in Gegenwart eines optisch aktiven Reduktionsmittels, wie beispielsweise des mit Binaphthol modifizierten chiralen Lithiumaluminiumhydrid-Reagenses (R)- oder (S)-BINAL-H [siehe R. Noyori et al.. J.A.C.S. 106, 6717 ff. (1984)] oder in Gegenwart eines optisch aktiven, chiralen Hydrierungs-Katalysators, wie beispielsweise des Ruthenium[(R)- oder (S)-BINAP] [siehe R. Noyori et al., J.A.C.S. 109, 5856 ff., (1987)].

Eine weitere Variante zur Herstellung optisch aktiver Verbindungen der Formel IIi besteht darin, ein chirales Carbinol nach der Methode von Trost et al., J. Org. Chem. 45, 1835 ff. (1980) successiv mit n-Butyllithium/Tetramethyläthylendiamin, mit Kohlenstoffoxid oder Aethylchlorformat, und mit Bromwasserstoff zu behandeln, und zwar gemäss der nachfolgenden Gleichung:

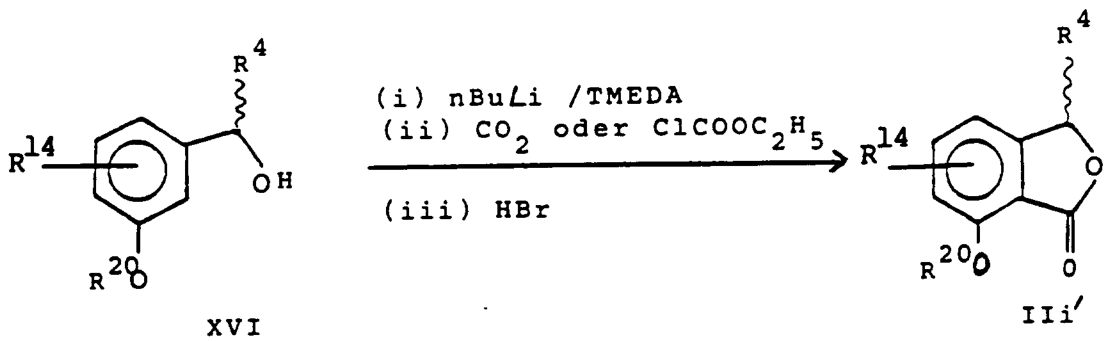


XVI

IIi'

Die Ausgangsmaterialien der Formel IX sind entweder bekannt oder können nach an sich bekannten Methoden hergestellt werden.

Diejenigen Ausgangsmaterialien der Formel II, in denen W eine Gruppe a) und $R^3$ Chlor oder Brom bedeuten, können durch Halogenierung der entsprechenden Ausgangsmaterialien der Formel II, in denen $R^3$ Hydroxy bedeutet, z.B. der Ausgangsmaterialien der oben angegebenen Formeln IIb und IIe, hergestellt werden. Als Halogenierungsmittel (Chlor- bzw. Bromierungsmittel) eignen sich insbesondere das entsprechende Thionylhalogenid $SOHal'_2$, Phosphoroxyhalogenid $POHal'_3$, Phosphortrihalogenid $PHal'_3$ oder Phosphorpentahalogenid $PHal'_5$, worin Hal' jeweils Chlor oder Brom bedeutet. Eine weitere Methode zur Herstellung solcher chlor- oder bromhaltigen Ausgangsmaterialien der Formel II besteht darin, die entsprechenden Ausgangsmaterialien II, in denen $R^3$ Wasserstoff bedeutet, z.B. die Ausgangsmaterialien der oben angegebenen Formel IIi, mit N-Chlor- bzw. N-Bromsuccinimid zu behandeln. Um zu den Ausgangsmaterialien der Formel II zu gelangen, in denen W eine Gruppe a) und $R^3$ Fluor, $C_{1-6}$-Alkylthio, Cyano oder Rhodano bedeuten, kann man die entsprechenden Ausgangsmaterialien der Formel II, in denen $R^3$ Chlor oder Brom bedeutet, einer Halogenaustauschreaktion, z.B. mit einem Alkalimetallfluorid wie Kaliumfluorid, mit einem Natriummercaptid, mit Natriumcyanid bzw. mit Kaliumrhodamid, unterwerfen. All diese Umwandlungen können unter an sich bekannten Reaktionsbedingungen durchgeführt werden.

Diejenigen Ausgangsmaterialien der Formel II, in denen W eine Gruppe a) und $R^3$ Cyano bedeuten, können ebenfalls dadurch hergestellt werden, dass man ein entsprechendes Ausgangsmaterial der Formel II, worin $R^3$ Hydroxy bedeutet, z.B. ein Ausgangsmaterial der oben angegebenen Formel IIb oder IIe, mit Kaliumcyanid oder Blausäure behandelt, und zwar unter an sich bekannten Reaktionsbedingungen [siehe beispielsweise J.N. Frescos et al., J. Org. Chem. 50, 805 ff (1985)].

Diejenigen Verbindungen der Formel II, in denen W eine Gruppe a) und $R^3$ $C_{2-7}$-Carboxyalkyl (Beispiel von "gegebenenfalls substituiertem $C_{1-6}$-Alkyl") Carboxy oder Carbamoyl bedeuten, können durch konventionelle Hydrolyse der entsprechenden Verbindungen II, in denen $R^3$ ($C_{2-5}$-Alkoxycarbonyl)-$C_{1-6}$-alkyl, $C_{2-7}$-Alkoxycarbonyl bzw. Cyano bedeutet, hergestellt werden.

Diejenigen Verbindungen der Formel II, in denen W eine Gruppe a) und $R^3$ Formyloxy, $C_{2-5}$-Alkanoyloxy, $C_{2-5}$-Alkoxycarbonyloxy, $C_{2-3}$-Alkylcarbamoyloxy oder Di($C_{1-2}$-alkyl)carbamoyloxy bedeuten, können durch konventionelle Formylierung, Acylierung, Carbonylierung bzw. Carbamoylierung der entsprechenden Verbindungen II, in denen $R^3$ Hydroxy bedeutet.

Schliesslich können diejenigen Verbindungen der Formel II, in denen W eine Gruppe a) und $R^3$ Di-($C_{1-2}$-alkoxy)phosphonyl bedeuten, hergestellt werden, indem man die entsprechenden Verbindungen II, in denen $R^3$ Hydroxy bedeutet, mit einem $C_{1-2}$-Alkylphosphit umsetzt.

Diejenigen Ausgangsmaterialien der Formel II, in denen W eine Gruppe b), $Y^1$ und $Y^2$ beide Sauerstoff und $R^6$ Wasserstoff bedeuten, können beispielsweise gemäss dem nachfolgenden Reaktionsschema 3 hergestellt werden, in dem $R^5$, $R^{14}$ und $R^{16}$ die oben angegebenen Bedeutungen besitzen und $R^{21}$ und $R^{22}$ jeweils Methoxy oder Aethoxy bedeutet.

Reaktionsschema 3

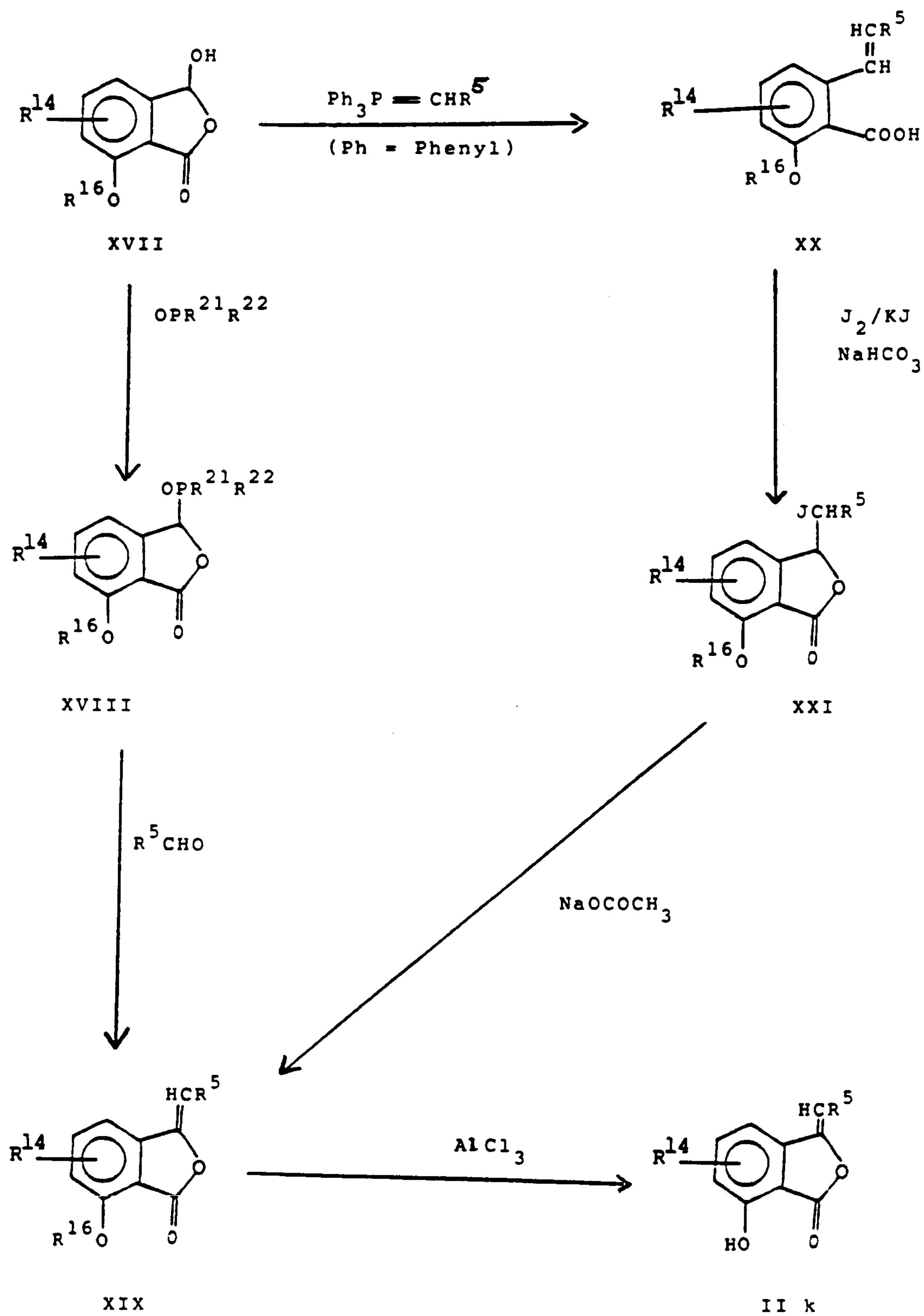


In R.S. Mali und S.L. Patil. Synthetic Comm. 20, 167 ff. (1990) und in E. Napolitano et al., Syntheses 1985, 38-40, ist ein analoges Reaktionsschema beschrieben, in dem die einzelnen Reaktionsstufen erläutert sind.

Diejenigen Ausgangsmaterialien der Formel II, in denen W eine Gruppe c) und X und $Y^1$ Sauerstoff bedeuten, können in an sich bekannter Weise, z.B. gemäss den Methoden von N.S. Narasimhan und B.H.

Bhide, Tetr. 27, 6171 (1971), J. Sinha et al., J. Ind. Chem. Soc. 63, 907 (1986) und H.N. Singh und R.P. Singh, J. Ind. Chem. Soc. 65, 685 (1988) wie auch gemäss dem nachfolgenden Reaktionsschema 4 hergestellt werden:

Reaktionsschema 4

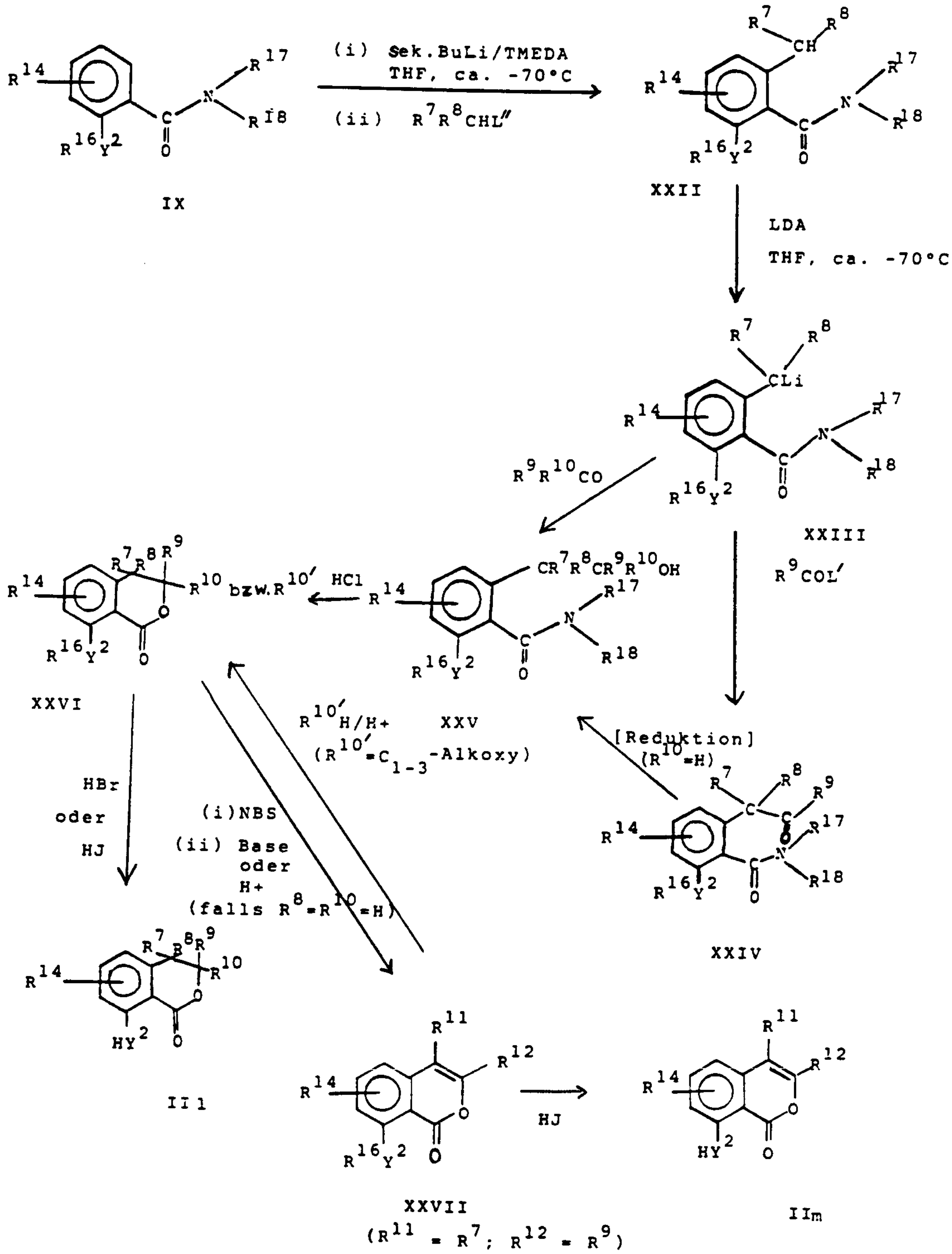


IX

XXII

XXIII

XXV

XXVI

XXIV

II1

XXVII
($R^{11}$ = $R^7$; $R^{12}$ = $R^9$)

IIm

In diesem Reaktionsschema besitzen $Y^2$, $R^7$, $R^8$, $R^9$, $R^{10}$, $R^{11}$, $R^{12}$, $R^{14}$, $R^{16}$, $R^{17}$, $R^{18}$, TMEDA, THF und L' die oben angegebenen Bedeutungen und bedeuten LDA Lithiumdiisopropylamid, NBS N-Bromsuccinimid und L" eine Abgangsgruppe, wie Halogen, insbesondere Chlor oder 2-Imidazolyl.

Auch bei diesem Reaktionsschema sind die involvierten einzelnen Reaktionen an sich bekannt.

Diejenigen Ausgangsmaterialien der Formel II, in denen W eine Gruppe d) und X und $Y^1$ Sauerstoff bedeuten, können beispielsweise auch gemäss den Methoden von N.L. Lewis et al., Synthesis 1986, 944 und F.M. Hauser et al., J. Org. Chem. 53, 4676 (1988), hergestellt werden.

Im allgemeinen können diejenigen Ausgangsmaterialien der Formel II, in denen X und/oder $Y^1$ Sauerstoff bedeutet, durch an sich bekannte Schwefelungsmethoden [siehe z.B. N. Lozach, Sulfur Reports 9, 153 ff. (1980)] in die entsprechenden Ausgangsmaterialien der Formel II, in denen X und/oder $Y^1$ Schwefel bedeutet, übergeführt werden. Zur Schwefelung wird zweckmässigerweise Phosphorpentasulfid, gegebenenfalls in Gegenwart von Pyridin, z.B. als Phosphorpentasulfid-Pyridin (1:2)-Komplex, das Lawesson-Reagens 2,4-Bis-(4-methoxyphenyl)-1,2-dithioxo -1,3,2,4-dithiaphosphetan [siehe z.B. S.-O. Lawesson et al., Bull. Soc. Chim. Belg. 87, 229-238 (1978)] oder das Davy-Reagens 2,4-Bis-(methylthio)-1,3,2,4-dithiadiphosphetan (siehe z.B. Sulfur Lett. 1983, 1, 167) verwendet, wobei dieses vorzugsweise in stöchiometrischer Menge oder in geringem Ueberschuss (z.B. bis zu 20%) eingesetzt wird. Zweckmässigerweise wird in einem inerten organischen Verdünnungsmittel, wie einem gegebenenfalls halogenierten Aromaten, z.B. Toluol oder Dichlorbenzol, oder einem aliphatischen oder cyclischen Aether, z.B. Dimethoxyäthan, und bei erhöhter Temperatur, insbesondere bei Temperaturen zwischen 80 °C und der Rückflusstemperatur des Reaktionsgemisches, gearbeitet. Zudem wird vorteilhaft eine katalytische Menge, also ca. 0,1 bis 10 Gewichtsprozent, bezogen auf die Menge der Verbindung II, Hexamethylphosphortriamid zugesetzt. Dieses Verfahren eignet sich insbesondere zur Herstellung von denjenigen Verbindungen der Formel II, in denen X Sauerstoff und $Y^1$ Schwefel bedeuten.

Diejenigen Ausgangsmaterialien der Formel II, in denen X Schwefel und $Y^1$ Sauerstoff bedeuten, können beispielsweise auch dadurch hergestellt werden, dass man eine Hydroxyverbindung der Formel XII (siehe Reaktionsschema 2) oder XXV (siehe Reaktionsschema 4) bzw. eine Verbindung der Formel XXII (siehe Reaktionsschema 4) gemäss den dem Fachmann bekannten Umwandlungsreaktionen, wie Halogenierung und Schwefelung, in die entsprechende Thiolverbindung überführt und anschliessend zur Verbindung der Formel II laktonisiert:

Reaktionsschema 5

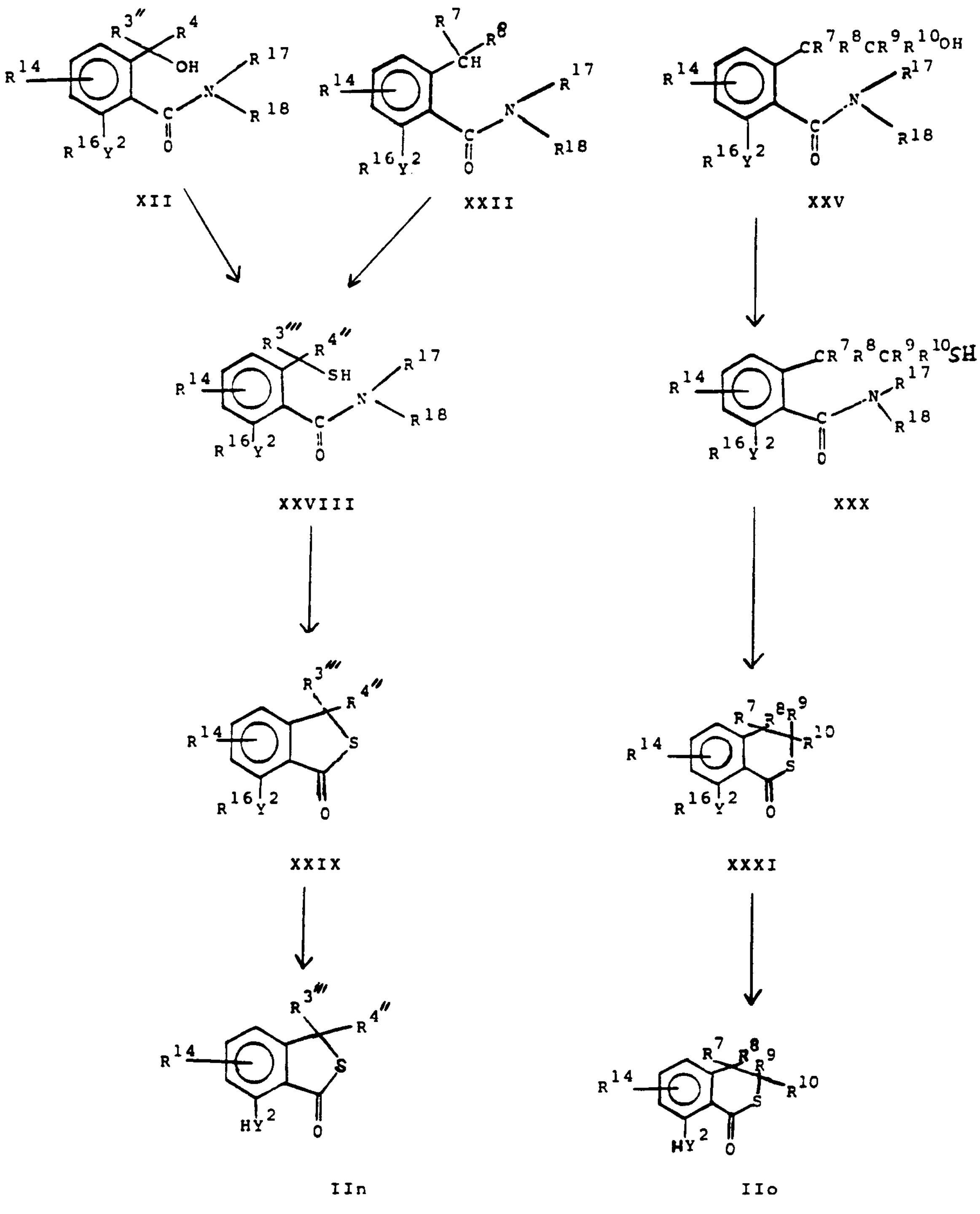

XII XXII XXV

XXVIII XXX

XXIX XXXI

IIn IIo

In diesem Reaktionsschema bedeuten $R^{3'''}$ und $R^{4''}$ der Verbindung XXVIII $R^{3''}$ und $R^4$ bzw. $R^7$ und $R^8$, je nachdem, ob man aus einer Verbindung der Formel XII bzw. aus einer Verbindung der Formel XXII ausgeht.

Diejenigen Ausgangsmaterialien der Formel II, in denen $Y^2$ Schwefel bedeutet, können - falls nicht bereits gemäss den bisher beschriebenen Methoden (siehe beispielsweise Reaktionsschemata 2, 4 und 5) erhältlich - in an sich bekannter Weise, z.B. gemäss dem nachfolgenden Reaktionsschema 6, hergestellt werden, in dem W, X, $Y^1$ und $R^{14}$ die oben angegebenen Bedeutungen besitzen:

Reaktionsschema 6

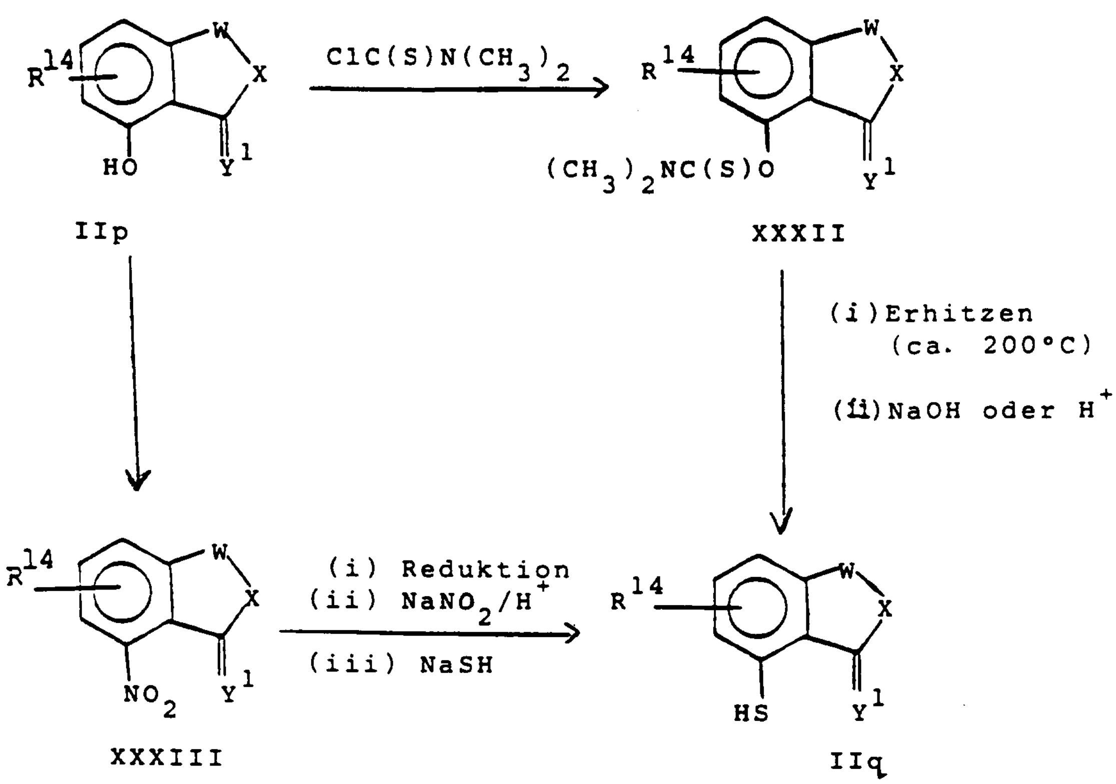


IIp XXXII XXXIII IIq

In den obigen Reaktionsschemata 1-6 und Gleichungen (XV→IIb; XVI→IIi') stellen die Produkte der Formeln IIa-IIq Untergruppen von Ausgangsmaterialien der Formel II dar. Die Ausgangsmaterialien der Formeln IV, V, IX, XV, XVI und XVII sind entweder bekannt oder können nach an sich bekannten Methoden hergestellt werden.

Die Ausgangsmaterialien der Formel III sind grösstenteils bekannt, und die neuen Ausgangsmaterialien III können analog den bekannten Ausgangsmaterialien III hergestellt werden.

Die Verbindungen der Formel I (im folgenden als erfindungsgemässe Verbindungen oder Wirkstoffe bezeichnet) besitzen herbizide Eigenschaften und eignen sich zur Bekämpfung von Unkräutern, einschliesslich Ungräsern, u.a. Agropyron repens, Alopecurus myosuroides, Avena fatua, Bromus inermis, Echinochloa crus-galli, Poa annua, Sorghum halepense, Abutilon theophrasti, Amaranthus retroflexus, Cassia obtusifolia, Chenopodium album, Galium aparine, Matricaria chamomilla, Sinapis arvensis und Stellaria media, in diversen Nutzpflanzenkulturen, u.a. Reis- (insbesondere Wasserreis-), Weizen-, Mais-, Soja-, Raps- und Baumwollekulturen. Zudem sind die Verbindungen sowohl Vorauflauf- als auch Nachauflauf-Herbizide. Bei einigen Vertretern der Verbindungen I hat sich eine gute Selektivität gezeigt, z.B. bei der Bekämpfung von Unkräutern, Ungräsern in Soja- und Baumwollekulturen.

Des weiteren besitzen die erfindungsgemässen Verbindungen pflanzenwachstumsregulierende Eigenschaften und eignen sich als Wirkstoffe zur positiven Beeinflussung des Wachstums von Nutzpflanzen. Dieser Effekt kann sowohl in Kulturpflanzen eine gewünschte Wachstumshemmung herbeiführen als auch Unkräuter nach ihrer Keimung genügend hemmen, um sie als Konkurrenten der Kulturpflanzen auszuschalten. Im Hinblick auf ökologische Zusammenhänge ist dies von Vorteil und demzufolge äusserst wünschenswert. Insbesondere sind dabei der Schutz der Erdoberfläche vor Austrocknung und/oder Erosion sowie die

Verminderung des Unkraut-Samenvorrates im Boden (bei gleichzeitiger Verhinderung der Blüte und der erneuerten versamung) zu nennen. Daher ist dieser Wirkung unter Umständen der Vorzug zu geben vor der völligen aber eventuell zeitlich begrenzten Verhinderung der Unkrautkeimung.

In der Praxis genügt üblicherweise eine Konzentration von 1 g bis 3 kg erfindungsgemässe Verbindung/ha, vorzugsweise 10 g bis 1 kg erfindungsgemässe Verbindung/ha, um den gewünschten herbiziden Effekt zu erzielen. Um den gewünschten herbiziden Effekt bei optimaler Nutzpflanzen-Verträglichkeit zu erzielen, ist der Bereich von 10 bis 100 g/ha in der Voraufkaufbehandlung und von 100 bis 1000 g/ha in der Nachauflaufbehandelung besonders günstig.

Die vorliegende Erfindung betrifft auch Verfahren zur Bekämpfung von Unkräutern und zur Regulation des Pflanzenwachstums durch Behandlung der Unkräuter bzw. der Pflanzen und/oder des gegen Unkräuter zu schützenden Guts mit einer wirksamen Menge gewisser Verbindungen der Formel I, sowie die Verwendung dieser Verbindungen zur Bekämpfung von Unkräutern oder zur Regulation des Pflanzenwachstums.

Das erfindungsgemässe Unkrautbekämpfungsmittel und Pflanzenwachstumsregulierende Mittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden Formulierungshilfsstoffe aus der Gruppe: feste Trägerstoffe; Lösungs- bzw. Dispersionsmittel; Tenside (Netzmittel und Emulgatoren); Dispergiermittel (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netzmitteln oder Emulgatoren und/oder von Dispergiermitteln, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netzmittel und Emulgatoren) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatrlumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre

Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, Z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 95 Gewichtsprozent, vorzugsweise zwischen 0,5 und 75 Gewichtsprozent einer bzw. mehrerer erfindungsgemässer Verbindungen als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 5 und 30 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,001 bis 10 Gewichtsprozent, insbesondere ca. 0,005 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. mit einem erfindungsgemässen Unkrautbekämpfungsmittel behandelt.

Die nachfolgenden Beispiele dienen zur näheren Erläuterung der Erfindung.

I. Herstellung der Verbindungen der Formel I:

Beispiel 1

7-[(4,6-Dimethoxy-pyrimidin-2-yl)oxy]-3-methyl-phthalid

Ein Gemisch von 0,4 g 7-Hydroxy-3-methyl-phthalid, 0,52 g 4,6-Dimethoxy-pyrimidin-2-methylsulfon und 1,05 g Kaliumcarbonat wird in 5 ml Dimethylformamid 2 Stunden auf Rückflusstemperatur erhitzt. Anschliessend wird das Gemisch mit Aethylacetat verdünnt und je einmal mit Wasser und Natriumchloridlösung gewaschen. Das nach Abdampfen des Lösungsmittels verbleibende Rohprodukt wird an Kieselgel mit Aethylacetat/n-Hexan (1:2) chromatographisch gereinigt. Auf diese Weise erhält man das 7-[(4,6-Dimethoxy-pyrimidin-2-yl)oxy]-3-methyl-phthalid, Smp. 190-191 °C; IR-Spektrum ($CHCl_3$): C=O 1765 $cm^{-1}$; $^1H$-NMR ($CDCl_3$, 200 MHz): 7,84 ppm (doppel-d,$J_1 = J_2$ =8Hz,1H), 7,57 ppm (d,J=8Hz,1H), 7,37 ppm (d,J=8Hz,1H), 6,01 ppm (s,1H), 5,72 ppm (q,J=7Hz,1H), 3,73 ppm (s,6H der beiden $OCH_3$), 1,56 ppm (d,J=7Hz,3H).

Beispiele 2-81

Analog dem in Beispiel 1 beschriebenen Verfahren (wobei in einigen Fällen Acetonitril oder Tetrahydrofuran als Lösungsmittel und Natriumhydrid als Base verwendet werden) werden die entsprechenden Verbindungen der Formeln II und III miteinander umgesetzt, um die in den nachstehenden Tabellen 1-5 aufgeführten Verbindungen der Formel I herzustellen:

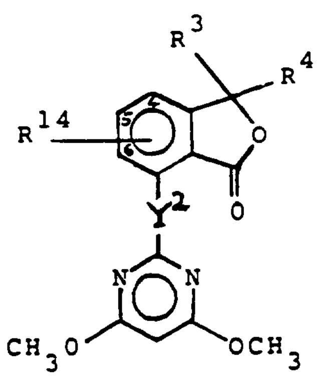

Tabelle 1

| Beispiel | $R^3$ | $R^4$ | $Y^2$ | $R^{14}$ | Physikalische Daten |
|---|---|---|---|---|---|
| 2 | H | H | O | H | Smp. 238°C; IR($CHCl_3$): C=O 1760 $cm^{-1}$ |
| 3 | Aethyl | H | O | H | Smp. 159-161°C; IR($CHCl_3$): C=O 1765$cm^{-1}$ |
| 4 | Isopropyl | H | O | H | Smp. 124-126°C; IR($CHCl_3$): C=O 1764 $cm^{-1}$ |
| 5 | Methyl | Methyl | O | H | Smp. 154-156°C; |
| 6 | Aethyl | Methyl | O | H | Smp. 115°C; IR($CHCl_3$): C=O 1760 $cm^{-1}$ |
| 7 | Phenyl | H | O | H | Smp. 170-173°C |
| 8 | Methoxy | H | O | H | Smp. 129-130°C; IR($CHCl_3$): C=O 1760 $cm^{-1}$ |
| 9 | Isopropoxy | H | O | H | Smp. 120-122°C; IR($CHCl_3$): C=O 1770 $cm^{-1}$ |
| 10 | Benzyloxy | H | O | H | Smp. 130-132°C |
| 11 | tert.Butoxy | H | O | H | Smp. 125-127°C |
| 12 | Methyl | H | S | H | Smp. 159-160°C |
| 13 | Aethoxy | H | O | H | Smp. 97-98°C |
| 14 | Methyl | H | O | 6-Methyl | Smp. 145-147°C |
| 15 | Methoxy | Methyl | O | H | Smp. 114-115°C |
| 16 | H | Methyl | O | H | (R-Isomeres) $[a]^D_{20}$ + 10,19° |

| | | | | | |
|---|---|---|---|---|---|
| 17 | Carbamoyl | H | O | H | Smp. 240°C (unter Zersetzung) |
| 18 | Trifluormethyl | H | O | H | Smp. 162-164°C |
| 19 | 2-Chloräthoxy | H | O | H | Smp. 93-95°C |
| 20 | Propargyloxy | H | O | H | Smp. 125-128°C |
| 21 | n-Propoxy | H | O | H | Smp. 99-100°C |
| 22 | 2-Methoxy-äthoxy | H | O | H | Smp. 95-96°C |
| 23 | Methyl | H | O | 4-Chlor | Smp. 145-148°C |
| 24 | Methyl | H | O | 4-Brom | Smp. 147-149°C |
| 25 | n-Propyl | H | O | H | Smp. 94-95°C |
| 26 | n-Butyl | H | O | H | Smp. 94-95°C |
| 27 | Aethinyl | H | O | H | |
| 28 | Nitromethyl | H | O | H | Smp. 150-152°C |
| 29 | Methoxy-carbonyl | H | O | H | |
| 30 | Fluor | H | O | H | |
| 31 | 2-Dimethyl-amino-äthoxy | H | O | H | |
| 32 | 2-Methylthio-äthoxy | H | O | H | |
| 33 | n-Butoxy | H | O | H | |
| 34 | (N-Methylcar-bamoyl)methoxy | H | O | H | |
| 35 | Acetyloxy | H | O | H | |
| 36 | Aethoxycar-bonyloxy | H | O | H | |
| 37 | Hydroxy | Trifluor-methyl | O | H | Smp. 234-235°C |

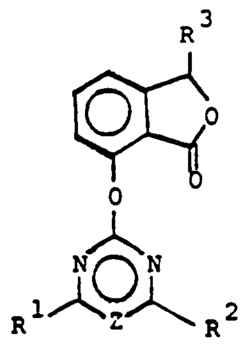

Tabelle 2

| Beispiel | Z | $R^1$ | $R^2$ | $R^3$ | Physikalische Daten |
|---|---|---|---|---|---|
| 38 | CH | Methyl | Methyl | Methyl | Smp. 163-164°C; IR($CHCl_3$): C=O 1770 $cm^{-1}$ |
| 39 | CH | Aethoxy | Aethoxy | Methyl | Smp. 99-100°C; IR($CHCl_3$): C=O 1765 $cm^{-1}$ |
| 40 | CH | Methoxy | Methyl | Methyl | Smp. 148-149°C; IR($CHCl_3$): C=O 1765 $cm^{-1}$ |
| 41 | C-Cl | Methoxy | Methoxy | Methyl | Smp. 195-196°C |
| 42 | CH | Trifluor-methyl | Methoxy | Methyl | Smp. 114-117°C |
| 43 | CH | Chlor | Methoxy | Methyl | Smp. 149-150°C |
| 44 | CH | Isopro-poxy | Methoxy | Methyl | Smp. 79-82°C |
| 45 | CH | Methoxy | Methyl | Methoxy | Smp. 107°C |
| 46 | CH | n-Pro-poxy | Methoxy | Methyl | Smp. 108-109°C |
| 47 | CH | Chlor | Difluor-methoxy | Methyl | Smp. 114-117°C |
| 48 | CH | Chlor | Dimethyl-amino | Methyl | Smp. 180-183°C |

| | | | | | |
|---|---|---|---|---|---|
| 49 | CH | Methyl | 2,2,2-Trifluoräthoxy | Methyl | Smp. 79-81°C |
| 50 | CH | Chlor | Methylamino | Methyl | Smp. 185-188°C |
| 51 | CH | H | Methoxy | Methyl | Smp. 110-112°C |
| 52 | CH | Methoxy | Aethoxy | Methyl | Smp. 113-116°C |
| 53 | CH | Chlor | Methoxy | Methoxy | Smp. 114-115°C |
| 54 | CH | Methoxymethyl | Methoxy | Methyl | Smp. 76-78°C |
| 55 | CH | Difluormethoxy | Methoxy | Methyl | Smp. 108-109°C |
| 56 | CH | Methoxy | n-Methoxy-methylamino | Methyl | Smp. 106-108°C |
| 57 | CH | Methoxy | Aethyl amino | Methyl | Smp. 143-146°C |

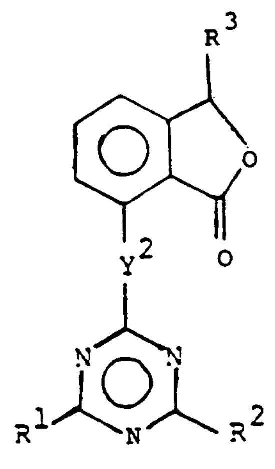

Tabelle 3

| Beispiel | $Y^2$ | $R^1$ | $R^2$ | $R^3$ | Physikalische Daten |
|---|---|---|---|---|---|
| 58 | O | Methoxy | Methoxy | Methyl | Smp. 133-134 °C |
| 59 | O | Methoxy | Methoxy | Methoxy | Smp. 88-90 °C |
| 60 | S | Methoxy | Methoxy | Methyl | Smp. 150-151 °C |
| 61 | O | Chlor | Methyl | Methyl | Smp. 144-147 °C |
| 62 | O | Methoxy | Dimethyl-amino | Methyl | Smp. 148-151 °C |
| 63 | O | Methyl | Methoxy | Methyl | Smp. 148-151 °C |
| 64 | O | Methoxy | Methyl-amino | Methyl | Smp. 179-182 °C |
| 65 | O | Chlor | Methyl-amino | Methyl | Smp. 181-183 °C |
| 66 | O | Methoxy | Methoxy | Aethyl | Smp. 137-140 °C |
| 67 | O | Chlor | Methoxy | Methyl | Smp. 139-142 °C |

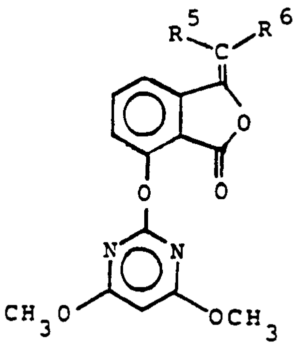

Tabelle 4

| Beispiel | $R^5$ | $R^6$ | Physikalische Daten |
|---|---|---|---|
| 68 | Methyl | H | Smp. 165-167 °C; (Z)<br>IR ($CHCl_3$): C = O 1765 $cm^{-1}$<br>$^1$H-NMR ($CDCl_3$): 5,69 ppm (q, J = 7Hz, C<u>H</u> = ) |
| 69 | Methyl | Methyl | Smp. 193-196 °C; (Z)<br>IR ($CHCl_3$): C = O 1768 $cm^{-1}$ |
| 70 | 4-Methoxy-phenyl | H | Smp. 231-233 °C; (E)<br>$^1$H-NMR ($CDCl_3$): 6,99 ppm (s, C<u>H</u> = ) |
| 71 | Phenyl | H | Smp. 191-192 °C; (Z)<br>$^1$H-NMR ($CDCl_3$): 6,46 ppm (s, C<u>H</u> = ) (Z) |
| 72 | 3-Methoxy-phenyl | H | Smp. 144-147 °C; (Z)<br>$^1$H-NMR ($CDCl_3$): 6,43 ppm (s, C<u>H</u> = ) |
| 73 | Aethyl | H | Smp. 122-125 °C; (Z)<br>$^1$H-NMR ($CDCl_3$): 5,64 ppm (t, J = 7Hz, C<u>H</u> = ) |
| 74 | n-Propyl | H | Smp. 115-118 °C; (Z)<br>$^1$H-NMR ($CDCl_3$): 5,66 ppm (t, J = 8Hz, C<u>H</u> = ) |
| 75 | H | H | |

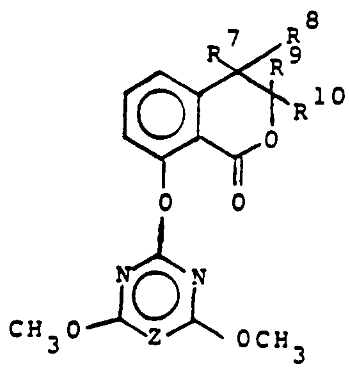

Tabelle 5

| Beispiel | Z | $-CR^7R^8-CR^9R^{10}-$ | Physikalische Daten |
|---|---|---|---|
| 76 | CH | $-CH_2CH(CH_3)-$ | Smp. 180-182 °C;<br>IR($CHCl_3$): C = O 1728 $cm^{-1}$ |
| 77<br>78 | N<br>CH | $-CH_2CH(CH_3)-$<br>$-CH_2CH(C_2H_5)-$ | Smp. 140-143 °C<br>Smp. 78-80 °C |
| 79 | CH | $-CH(CH_3)CH(CH_3)-$ | (trans-Form)<br>$^1$H-NMR ($CDCl_3$): 4,38 ppm (m, 1H) |
| 80 | CH | $CH(CH_3)CH(CH_3)-$ | (cis-Form)<br>$^1$H-NMR ($CDCl_3$): 4,68 ppm (m, 1H) |
| 81 | CH | $CH(CH_3)CH_2-$ | Smp. 116-119 °C |

Beispiel 82

8-[(4,6-Dimethoxy-pyrimidin-2-yl)oxy]-3-methyl-isochromen-1-on

Analog dem in Beispiel 1 beschriebenen Verfahren wird 8-Hydroxy-3-methyl-isochromen-1-on mit 4,6-Dimethoxy-pyrimidin-2-methylsulfon umgesetzt, um 8-[(4,6-Dimethoxy-pyrimidin-2-yl)oxy]-3-methyl-isochromen-1-on, Smp. 188-190 °C, herzustellen.

Beispiel 83

3-[(Z)-Aethyliden]-7-[(4,6-dimethoxy-pyrimidin-2-yl)oxy]-phthalid

350 mg (2 mMol) 7-Hydroxy-3-vinyl-phthalid, 460 mg (2,2 mMol) 4,6-Dimethoxy-2-methylsulfonyl-pyrimidin und 414 mg (3 mMol) Kaliumcarbonat werden in 10 ml Dimethylformamid eine Stunde bei 100 °C erhitzt. Dann wird das Reaktionsgemisch auf 100 ml halbkonzentrierte Kochsalzlösung gegossen und das wässrige Gemisch zweimal mit 50 ml Aethylacetat extrahiert. Man trocknet die organische Phase über wasserfreiem Magnesiumsulfat, engt sie unter vermindertem Druck ein und unterwirft das Rohprodukt einer chromatographischen Reinigung an Kieselgel mit Aethylacetat/n-Hexan (2:3).

Auf diese Weise erhält man 250 mg 3-[(Z)-Aethyliden)-7-[(4,6-dimethoxy-pyrimidin-2-yl)oxy]-phthalid in Form farbloser Kristalle, Smp. 163-165 °C; IR-Spektrum($CHCl_3$): C=O 1765 $cm^{-1}$. Dieses Produkt ist identisch mit der Verbindung des Beispiels 68.

Beispiel 84

7-[(4,6-Dimethoxy-pyrimidin-2-yl)oxy]-3-hydroxy-phthalid

a) 15,2 g 7-[(4,6-Dimethoxy-pyrimidin-2-yl)oxy]-3-methoxyphthalid (siehe Beispiel 8) wird in einem 1:1-Gemisch von Tetrahydrofuran und Salzsäure 3 Stunden auf Rückflusstemperatur erhitzt. Unter vermindertem Druck wird dann eingeengt, und die ausgefallenen Kristalle werden abfiltriert und mit Wasser gewaschen. Man erhält reines 7-[(4,6-Dimethoxy-pyrimidin-2-yl)oxy]-3-hydroxy-phthalid, Smp. 143-145 °C; IR (KBr): 1770 $cm^{-1}$; $^1$H-NMR ($CDCl_3$): 6,60 ppm (s, 1H).

b) Man kann das obige Produkt auch dadurch herstellen, dass man 6 g 3,7-Dihydroxy-phthalid in einer methanolischen Lösung von 2,6 g Kaliumhydroxid löst und eine resultierende Lösung azeotrop mit Toluol zur Trockene eindampft. Das Mono-Kaliumsalz wird dann in 100 ml trockenem Dimethylsulfoxid aufgenommen und portionenweise mit 1,9 g Natriumhydrid behandelt. Man rührt noch 10 Minuten bei 40 °C nach, versetzt dann bei Raumtemperatur mit 9,9 g 4,6-Dimethoxy-pyrimidinyl-2-methylsulfon und hält das Reaktionsgemisch noch 1 Stunde bei 30-35 °C. Man versetzt mit Wasser und extrahiert zur Entfernung von Verunreinigungen mit Aethylacetat. Die wässrige Phase wird mit Salzsäure angesäuert und dann mit frishem Aethylacetat extrahiert. Man erhält so nach Behandlung mit Aktivkohle und/oder Filtration an Kieselgel als leicht gelbliche Kristalle das 7-[(4,6-Dimethoxy-pyrimidin-2-yl)oxy]-3-hydroxy-phthalid, Smp. 144-146 °C.

Beispiel 85

3-Chlor-7-[(4,6-dimethoxy-pyrimidin-2-yl)oxy]-phthalid

2,3 g 7-[(4,6-Dimethoxy-pyrimidin-2-yl)oxy]-3-hydroxy-phthalid (siehe Beispiel 84) werden in ca. 40 ml Phosphoroxychlorid suspendiert, und die Suspension wird 2 Stunden auf 90 °C erhitzt. Das Reaktionsgemisch wird dann in 250 ml Wasser bei 35-45 °C eingetragen. Man verdünnt dann mit weiteren 500 ml Wasser und filtriert das ausgefallene Kristallisat ab und wäscht es mit Wasser neutral. Man erhält als leicht gelbliche Kristalle reines 3-Chlor-7-[(4,6-dimethoxy-pyrimidin-2-yl)oxy]-phthalid, Smp. 143-144 °C.

Beispiel 86

7-[(4,6-Dimethoxy-pyrimidin-2-yl)oxy]-3-fluor-phthalid

Ein Gemisch von 1,0 g 3-Chlor-7-[(4,6-dimethoxy-pyrimidin-2-yl)oxy]-phthalid (siehe Beispiel 85) und 0,19 g sprühgetrocknetem Kaliumfluorid sowie einer Spatelspitze 18-Crown-6 wird 100 Minuten auf

Rückflusstemperatur erhitzt. Man filtriert dann über Celite® ab, dampft ein und kristallisiert das rohe Produkt aus Aethylacetat/n-Hexan um. Man er- hält so das 7-[(4,6-Dimethoxy-pyrimidin-2-yl)oxy]-3-fluor-phthalid, Smp. 172-174 °C.

Beispiel 87

7-[(4,6-Dimethoxy-pyrimidin-2-yl)oxy]-3-methylthio-phthalid

1,0 g 3-Chlor-7-[(4,6-dimethoxy-pyrimidin-2-yl)oxy]-phthalid (siehe Beispiel 85) und 0,24 g Natriummethylmercaptid werden ca. 16 Stunden in 20 ml Tetrahydrofuran verrührt. Anschliessend filtriert man durch Celite® ab und dampft unter vermindertem Druck ein. Durch Umkristallisation aus Diäthyläther/n-Hexan erhält man das 7-[(4,6-Dimethoxy-pyrimidin-2-yl)oxy]-3-methylthio-phthalid, Smp. 138-140 °C.

Beispiel 88

3-Aethylthio-7-[(4,6-dimethoxy-pyrimidin-2-yl)oxy]-phthalid

Analog der oben beschriebenen Methode (Beispiel 87) erhält man aus 3-Chlor-7-[(4,6-dimethoxy-pyrimidin-2-yl)oxy]-phthalid (siehe Beispiel 85) und Natriumäthylmercaptid das 3-Aethylthio-7-[(4,6-dimethoxy-pyrimidin-2-yl)oxy]-phthalid, Smp. 103-106 °C.

Beispiel 89

7-[(4,6-Dimethoxy-pyrimidin-2-yl)oxy]-3-rhodano-phthalid

Ein Gemisch von 1,4 g 3-Chlor-7-[(4,6-dimethoxy-pyrimidin-2-yl)oxy]-phthalid (siehe Beispiel 85) und 0,46 g Kaliumrhodanid wird in Gegenwart einer Spatelspitze 18-Crown-6 in 15 ml Acetonitril 4 Stunden erhitzt. Man filtriert dann durch Celite® ab, dampft ein und chromatographiert mit 30% Aethylacetat/n-Hexan. Man erhält als hellgelbes Kristallisat das 7-[(4,6-Dimethoxy-pyrimidin-2-yl)oxy]-3-rhodano-phthalid, Smp. 161-163 °C.

Beispiel 90

7-[(4,6-Dimethoxy-pyrimidin-2-yl)oxy]-3-phenoxy-phthalid

Ein Gemisch von 1,4 g 3-Chlor-7-[(4,6-dimethoxy-pyrimidin-2-yl)oxy]-phthalid (siehe Beispiel 85) und 0,63 g Kaliumphenolat wird in Gegenwart einer Spatelspitze 18-Crown-6 in 15 ml Acetonitril auf Rückflusstemperatur erhitzt. Nach 19 Stunden filtriert man durch Celite®, dampft ein und reinigt das Rohprodukt mittels Säulenchromatographie (Laufmittel 25% Aethylacetat/n-Hexan). Man erhält als weisses Kristallisat das 7-[(4,6-Dimethoxy-pyrimidin-2-yl)oxy]-3-phenoxy-phthalid, Smp. 155-157 °C.

Beispiel 91

7-[(4,6-Dimethoxy-pyrimidin-2-yl)oxy]-3-vinyl-phthalid

3,6 g 7-[(4,6-Dimethoxy-pyrimidin-2-yl)oxy]-3-hydroxy-phthalid (siehe Beispiel 84) werden bei -45 °C in 60 ml absolutem Tetrahydrofuran vorgelegt, und die Lösung wird innert 10 Minuten mit 18 ml einer 2M Lösung von Vinylmagnesiumchlorid in Tetrahydrofuran behandelt. Man lässt ca. 16 Stunden bei Raumtemperatur nachrühren, setzt weitere 6 ml Vinylmagnesiumchloridlösung zu und erhitzt noch eine Stunde auf Rückflusstemperatur. Sodann wird die erkaltete Reaktionslösung mit 1N Salzsäure angesäuert und am Rotationsverdampfer vom Tetrahydrofuran befreit. Die wässrige Phase wird dann mit tert.Butylmethyläther extrahiert, und die organische Phase gewaschen und unter vermindertem Druck eingedampft. Nach Kieselgelchromatographie mit Aethylacetat/n-Hexan (2:3) als Laufmittel erhält man reines 7-[-(4,6--Dimethoxy-pyrimidin-2-yl)oxy]-3-vinyl-phthalid, Smp. 94-97 °C.

Beispiel 92

3-Cyano-7-[(4,6-dimethoxy-pyrimidin-2-yl)oxy]-phthalid

1,0 g 7-[(4,6-Dimethoxy-pyrimidin-2-yl)oxy]-3-hydroxy-phthalid (siehe Beispiel 84) werden in eine Lösung von 2,1 g Kaliumcyanid in 15 ml Wasser eingetragen und das Ganze bei -7 bis -3 °C unter Kühlung tropfenweise über 10 Minuten mit 10 ml konzentrierter Salzsäure behandelt. Man lässt ca. 16 Stunden bei Raumtemperatur nachrühren und filtriert dann die ausgefallenen Kristalle ab. Man wäscht sie mit Wasser nach und kristallisiert sie aus Aceton/n-Hexan um. Man erhält 0,2 g 3-Carbamoyl-7-[(4,6-dimethoxy-pyrimidin-2-yl)oxy]-phthalid (siehe auch Beispiel 17).

Die Mutterlauge wird mit Aethylacetat/n-Hexan (1:1) als Laufmittel chromatographiert. Man erhält reines 3-Cyano-7-[(4,6-dimethoxy-pyrimidin-2-yl)oxy]-phthalid, Smp. 157-159 °C.

Beispiel 93

3-Cyanomethoxy-7-[(4,6-dimethoxy-pyrimidin-2-yl)oxy]-phthalid

Ein Gemisch von 1,5 g 7-[(4,6-Dimethoxy-pyrimidin-2-yl)oxy]-3-hydroxy-phthalid (siehe Beispiel 84) und 0,8 ml Chloracetonitril wird in Gegenwart von 2,1 g trockenem Kaliumcarbonat und je einer Spatelspitze Natriumjodid und 18-Crown-6 1 Stunde in Aethylmethylketon bei 60 °C gehalten. Das erkaltete Reaktionsgut wird in tert.Butylmethyläther aufgenommen und die Lösung mit verdünnter Salzsäure und Wasser gewaschen, eingedampft und chromatographisch an Kieselgel (Laufmittel: Aethylacetat/n-Hexan 1:1) gereinigt. Man erhält so das 3-Cyanomethoxy-7-[(4,6-dimethoxy-pyrimidin-2-yl)oxy]-phthalid, Smp. 99-102 °C.

Beispiel 94

7-[(4,6-Dimethoxy-pyrimidin-2-yl)oxy]-3-(methoxycarbonylmethoxy)-phthalid

Analog der oben beschriebenen Methode (Beispiel 93) erhält man aus 7-[(4,6-Dimethoxy-pyrimidin-2-yl)oxy]-3-hydroxy-phthalid (siehe Beispiel 84) und Chloressigsäure-methylester das 7-[(4,6-Dimethoxy-pyrimidin-2-yl)oxy] -3-(methoxycarbonylmethoxy)-phthalid, Smp. 102-104 °C.

Beispiel 95

3-Acetoxy-7-[(4,6-dimethoxy-pyrimidin-2-yl)oxy]-phthalid

1,3 g 7-[(4,6-Dimethoxy-pyrimidin-2-yl)oxy]-3-hydroxy-phthalid (siehe Beispiel 84) werden in 30 ml Tetrahydrofuran gelöst, und die Lösung wird mit 0,6 ml Triäthylamin versetzt und dann tropfenweise bei 25 °C (Innentemperatur) mit 0,5 ml Acetylchlorid behandelt. Nach 2-stündigem Nachrühren bei Raumtemperatur extrahiert man mit Aethylacetat gegen Eiswasser und verdünnter Salzsäure, wäscht die organische Phase mit Natriumchloridlösung, trocknet sie über wasserfreiem Magnesiumsulfat und dampft sie unter vermindertem Druck ein. Mittels Säulenchromatographie an Kieselgel [Laufmittel: Aethylacetat/n-Hexan (2:3)] erhält man als weisses Kristallisat das 3-Acetoxy-7-[(4,6-dimethoxy-pyrimidin-2-yl)oxy]-phthalid, Smp. 119-120 °C.

Beispiel 96

3-(Aethoxycarbonylmethyl)-7-[(4,6-dimethoxy-pyrimidin-2-yl)oxy]-phthalid

Ein Gemisch von 1,5 g 7-[(4,6-Dimethoxy-pyrimidin-2-yl)oxy]-3-hydroxy-phthalid (siehe Beispiel 84) und 2,5 g (Aethoxycarbonylmethylen)-triphenyl-phosphoran wird in ca. 40 ml Tetrahydrofuran 10 Stunden bei Rückflusstemperatur gehalten. Das Lösungsmittel wird dann abgedampft und das zurückbleibende Reaktionsgut an einer Kieselgelsäule (Laufmittel: Aethylacetat/n-Hexan 1:1) gereinigt. Man erhält so das 3-(Aethoxycarbonylmethyl)-7-[(4,6-dimethoxy-pyrimidin -2-yl)oxy]-phthalid, Smp. 150-152 °C.

Beispiel 97

3-(Dimethoxyphosphonyl)-7-[(4,6-dimethoxy-pyrimidin-2-yl)oxy]-phthalid

1,0 g 7-[(4,6-Dimethoxy-pyrimidin-2-yl)oxy]-3-hydroxy-phthalid (siehe Beispiel 84) wird in 30 ml Methanol gelöst, 0,88 ml 5,4M Natriummethylat-Lösung zugesetzt und dann das Ganze tropfenweise mit 0,43 ml Dimethylphosphit behandelt. Man belässt eine Stunde bei Raumtemperatur und fügt dann 0,33 ml Methansulfonsäure zu und dampft das Reaktionsgemisch unter vermindertem Druck ein. Der Rückstand wird in Aethylacetat aufgenommen, und die Lösung je einmal mit 2N Salzsäure und Natriumchloridlösung gewaschen. Man behandelt die organische Lösung mit Aktivkohle und kristallisiert das Produkt aus Aethylacetat/n-Hexan um. Man erhält das 3-(Dimethoxyphosphonyl)-7-[(4,6-dimethoxy-pyrimidin -2-yl)oxy]-phthalid, $^1$H-NMR ($CDCl_3$): 5,70 ppm (d, 11Hz, 1H), 3,93 und 3,64 ppm (2d, 11Hz, P(O) $(OCH_3)_2$); Smp. 135-137 °C.

Beispiel 98

7-[(4,6-Dimethoxy-pyrimidin-2-yl)oxy]-3-methyl-2-benzothiophen-1(3H)-on

1,21 g 7-Hydroxy-3-methyl-isobenzofuran-1(3H)-thion werden in 10 ml Acetonitril zusammen mit 0,83 g 4,6-Dimethoxy-pyrimidinyl-2-methylsulfon und 1,05 g Kaliumcarbonat in Gegenwart einer Spatelspitze 18-Crown-6 2 Stunden auf Rückfluzstemperatur erhitzt. Das Reaktionsgut wird in Aethylacetat aufgenommen, und die organische Lösung mit Wasser und Natriumchloridlösung gewaschen, unter vermindertem Druck eingedampft und dann chromatographisch gereinigt (Laufmittel 30% Diäthyläther in n-Hexan). Man erhält als gelbliche Kristalle das 7-[(4,6-Dimethoxy-pyrimidin-2-yl)oxy]-3-methyl -2-benzothiophen-1(3H)-on, Smp. 157-159 °C; IR-Spektrum ($CHCl_3$): 1682, 1600, 1555, 1356, 1240, 1190 $cm^{-1}$; $^1$H-NMR ($CDCl_3$): 4,86 ppm (q, 1H), 1,78 ppm (d, 3H).

Beispiel 99

7-[(4,6-Dimethoxy-pyrimidin-2-yl)oxy]-3-methyl-isobenzofuran-1(3H)-thion

Ein Gemisch von 6,35 g 7-[(4,6-Dimethoxy-pyrimidin-2-yl)oxy]-3-methyl-phthalid (siehe Beispiel 1) und 8,92 g 2,4-Bis-(4-methoxyphenyl)-2,4-dithioxo -1,3,2,4-dithiadiphosphetan (Lawesson Reagens) wird in 40 ml Xylol ca. 16 Stunden bei Rückflusstemperatur gehalten. Das Reaktionsgut wird dann über Kieselgel (Laufmittel: 30% Diäthyläther/n-Hexan) filtriert und aus Aethylacetat/n-Hexan umkristallisiert. Man erhält so als gelbes Kristallisat das 7-[(4,6-Dimethoxy-pyrimidin-2-yl)oxy]-3-methyl-isobenzofuran -1(3H)-thion, Smp. 163-164 °C; IR-Spektrum ($CHCl_3$): 1602, 1570, 1358, 1304, 1195 $cm^{-1}$; $^1$H-NMR ($CDCl_3$): 5,80 ppm (q, 1H), 1,71 ppm (d, 3H).

Beispiel 100

7-[(4,6-Dimethoxy-1,3,5-triazin-2-yl)oxy]-3-methyl-isobenzofuran-1(3H)-thion

1,0 g 7-Hydroxy-3-methyl-isobenzofuran-1(3H)-thion wird zu einer Suspension von 0,15 g Natriumhydrid in absolutem Dimethylformamid eingetragen und nach beendeter Wasserstoffentwicklung mit 1,0 g 2-Chloro-4,6-dimethoxy-1,3,5-triazin versetzt. Man lässt 5 Stunden bei Raumtemperatur nachrühren, gibt dann Eiswasser zu, extrahiert mit Aethylacetat und wäscht mit Natriumchloridlösung. Das eingedampfte Rohprodukt wird chromatographisch gereinigt (Laufmittel: 45% Aethylacetat in n-Hexan) und aus Aceton/n-Hexan umkristallisiert. Man erhält so als gelbes Kristallisat das 7-[(4,6-Dimethoxy-1,3,5-triazin-2-yl)oxy] -3-methyl-isobenzofuran-1(3H)-thion, Smp. 177-178 °C; IR-Spektrum ($CHCl_3$): 1590, 1560, 1366, 1304, 1140 $cm^{-1}$; $^1$H-NMR ($CDCl_3$): 5,81 ppm (q, 1H), 1,73 ppm (d, 3H).

II. Herstellung der Ausgangsmaterialien der Formel II

Beispiel 101

7-Hydroxy-3-methyl-phthalid

(i) Zu einer Lösung von 5,32 g 1-(2,2-Dimethylpropanoyloxy)-3,5-dioxo-exo -10-oxatricyclo[5.2.1.0(2,6)]-dec-8-en (Verbindung der Formel VI, in der $R^{14'}$ Wasserstoff und $R^{15}$ 2,2-Dimethylpropanoyloxy bedeuten) in 50 ml absolutem Tetrahydrofuran werden unter Argon bei -78°C 12,5 ml Methyllithium (1,6M in Diäthyläther) innert 30 Minuten zugetropft. Nach Beendigung der Zugabe lässt man das Gemisch auf Raumtemperatur kommen und giesst es dann auf 100 ml eiskalte gesättigte Monokaliumphosphatlösung. Die wässrige Lösung wird dreimal mit Diäthyläther extrahiert, und die vereinigten organischen Phasen werden mit Monokaliumphosphatlösung gewaschen, über wasserfreiem Magnesiumsulfat getrocknet und nach Entfärben mit Aktivkohle filtriert und eingeengt. Das gelbfarbene Rohprodukt wird aus Diäthyläther/n-Hexan umkristallisiert. Auf diese Weise erhält man 1,8 g (32% der theoretischen Ausbeute) (3aα,4β,7β,7aα)-Hexahydro-1-hydroxy-1-methyl -3-oxo-4,7-epoxyisobenzofuran-4-yl-pivalat als gelbliche Kristalle, Smp. 153-154°C (= Verbindung der Formel VIII, in der $R^{4'}$ Methyl, $R^{14'}$ Wasserstoff und $R^{15}$ 2,2-Dimethylpropanoyloxy bedeuten).

(ii) 1,7 g des Produktes der bisherigen Reaktionsstufe werden bei 2-4°C unter Argon zu einer Suspension von 0,31 g Natriumborhydrid in 40 ml absolutem Aethanol gegeben. Nach erfolgter Zugabe lässt man das Gemisch auf Raumtemperatur kommen und giesst es auf 60 ml 1N Salzsäure. Man dampft das Aethanol unter vermindertem Druck ab, filtriert die resultierenden weissen Kristalle ab und wäscht sie mit Wasser nach und trocknet sie. Auf diese Weise erhält man 0,87 g (86% der theoretischen Ausbeute) (3aα,4β,7β,7aα)-Hexahydro-1-methyl-3-oxo -4,7-epoxyisobenzofuran-4-yl-pivalat als farblose Kristalle, Smp. 114-115°C.

(iii) 11,4 g des Produktes der bisherigen Reaktionsstufe werden portionenweise zu 30 ml mit Aethanol/Eis gekühlter konzentrierter Schwefelsäure gegeben. Nach erfolgter Zugabe wird das Gemisch auf Eis gegossen, und man filtriert dann die ausgefallenen Kristalle und wäscht sie bis zur Neutralität mit Wasser nach. Auf diese Weise erhält man 6,4 g (91% der theoretischen Ausbeute) 7-Hydroxy-3-methyl-phthalid als beige Kristalle, Smp. 107-108°C.

Beispiel 102

3,3-Dimethyl-7-hydroxy-phthalid

(i) Eine aus 1,43 g Magnesium und 8,37 g Methyljodid in 65 ml Diäthyläther hergestellte Grignard-Lösung wird zu einer Lösung von 7,85 g 1-(2,2-Dimethylpropanoyloxy)-3,5-dioxo-exo -10-oxatricyclo-[5.2.1.0(2,6)]dec-8-en in 35 ml Tetrahydrofuran so zugetropft, dass die Temperatur -25°C nicht übersteigt. Nach Beendigung der Zugabe rührt man das Reaktionsgemlsch noch einige Stunden bei Raumtemperatur nach, säuert es dann mit 2N Salzsäure auf pH 2 an und extrahiert es mit Diäthyläther. Nach Trocknen der Aetherlösung und Abdampfen des Lösungsmittels verbleiben 7,5 g Rohprodukt, das man anschliessend an Kieselgel mit Diäthyläther/n-Hexan (3:1) chromatographisch reinigt. Auf diese Weise erhält man das (3aα,4β,7β,7aα)-Hexahydro-1,1-dimethyl-3-oxo -4,7-epoxyisobenzofuran-4-yl-pivalat als Oel.

(ii) Das ölige Produkt der bisherigen Reaktionsstufe wird in 2,8 ml konzentrierte Schwefelsäure aufgenommen, und die Lösung wird 20 Minuten bei 5°C gerührt. Dann giesst man die Lösung auf Eis und extrahiert mit Diäthyläther. Die organische Phase wird mit Wasser gewaschen, über wasserfreiem Magnesiumsulfat getrocknet und eingedampft. Auf diese Weise erhält man das 3,3-Dimethyl-7-hydroxy-phthalid als farblose Kristalle, Smp. 128-134°C.

Beispiel 103

3-Aethyl-7-hydroxy-phthalid

(i) Zu einer Lösung von 5,2 g 2-Methoxy-N,N-diäthyl-benzamid und 2,91 g Tetramethylenäthylendiamin in 50 ml absolutem Tetrahydrofuran werden 13,7 ml sek.Butyllithium (1,4M in Cyclohexan/Isopentan) so zugetropft, dass die Temperatur -68°C nicht übersteigt. Nach erfolgter Zugabe rührt man das Reaktionsgemisch noch eine Stunde bei -78°C und gibt anschliessend 2,5 ml Propionaldehyd zu. Man lässt das

Reaktionsgemisch langsam auf Raumtemperatur kommen, rührt es eine Stunde nach und verdünnt es mit 300 ml Diäthyläther. Die organische Phase wird mit 2N Salzsäure und danach mit gesättigter Natriumchloridlösung gewaschen, über wasserfreiem Magnesiumsulfat getrocknet und eingedampft. Auf diese Weise erhält man das 6-(1-Hydroxyäthyl)-2-methoxy-N,N-diäthyl-benzamid als Rohprodukt.

(ii) Das Rohprodukt der bisherigen Reaktionsstufe wird in 48%igen wässrigen Bromwasserstoff aufgenommen, und die Lösung ca. 16 Stunden auf Rückflusstemperatur erhitzt. Dann wird das Gemisch auf Raumtemperatur abgekühlt und zweimal mit Diäthyläther extrahiert. Nach Trocknen über wasserfreiem Magnesiumsulfat und Eindampfen verbleibt ein Rohprodukt, das man anschliessend an Kieselgel mit Diäthyläther/n-Hexan (1:4) chromatographisch reinigt. Auf diese Weise erhält man das 3-Aethyl-7-hydroxy-phthalid, IR-Spektrum ($CHCl_3$): C=O 1738 $cm^{-1}$.

Beispiel 104

7-Hydroxy-3-isopropyl-phthalid

(i) Eine Lösung von 8.0 g 1-(2,2-Dimethylpropanoyloxy)-3,5-dioxo-exo -10-oxatricyclo[5.2.1.0(2,6)]dec-8-en in 25 ml Tetrahydrofuran wird zu einer Grignard-Lösung von 6,8 g Isopropylmagnesiumchlorid in 33 ml Tetrahydrofuran so zugetropft, dass die Temperatur -20°C nicht übersteigt. Nach erfolgter Zugabe lässt man das Reaktionsgemisch auf Raumtemperatur kommen und rührt es ca. 16 Stunden nach. Unter Eiskühlung wird dann mit 45 ml 2N Salzsäure der pH-Wert des Gemisches auf 2 gebracht, und man extrahiert zweimal mit 300 ml Diäthyläther. Die organische Phase wird über wasserfreiem Magnesiumsulfat getrocknet, unter vermindertem Druck eingedampft und das resultierende Rohprodukt an Kieselgel mit Diäthyläther/n-Hexan (3:7) chromatographisch gereinigt. Auf diese Weise erhält man das (3aα,4β,7β,7aα)-Hexahydro-1-isopropyl-3-oxo -4,7-epoxyisobenzofuran-4-yl-pivalat.

(ii) Das Produkt der bisherigen Reaktionsstufe wird in konzentrierte Schwefelsäure eingetragen, und die Lösung wird 20 Minuten bei 5°C gerührt. Dann giesst man das Gemisch auf Eis und extrahiert mit Diäthyläther. Nach Trocknen und Eindampfen der Aether-Lösung erhält man das 7-Hydroxy-3-isopropyl-phthalid als farblose Kristalle.

Beispiel 105

3,7-Dihydroxyphthalid

4,4 g 3-Hydroxy-7-methoxy-phthalid [siehe B.L. Chenard et al., J. Org. Chem. 49, 318 (1984) für dessen Herstellung] werden in 100 ml 48%iger Bromwasserstoffsäure 75 Minuten auf Rückflusstemperatur erhitzt. Anschliessend giesst man das Reaktionsgemisch auf Eiswasser und extrahiert das wässrige Gemisch viermal mit Aethylacetat. Die vereinigten organischen Phasen werden mit Kochsalzlösung gewaschen, über wasserfreiem Magnesiumsulfat getrocknet und unter vermindertem Druck eingeengt. Man filtriert das Rohprodukt durch eine kurze Kieselgelsäule mit Aethylacetat als Laufmittel und entfärbt das so erhaltene braune Oel durch Erhitzen in Gegenwart von Aktivkohle. Auf diese Weise erhält man 2,5 g eines gelblichen, amorphen Festkörpers, also das rohe 3,7-Dihydroxyphthalid, das eventuell ungereinigt zur Herstellung eines weiteren Ausgangsmaterials der Formel II, z.B. 7-Hydroxy-3-methoxy-phthalid (siehe Beispiel 118) verwendet werden kann.

Beispiel 106

3,7-Dihydroxyphthalid

Die Titelverbindung kann auch wie folgt hergestellt werden:

3,0 g 3-Hydroxy-7-methoxy-phthalid [siehe B.L. Chenard et al., J. Org. Chem. 49, 318 (1984)] werden portionenweise zu einer Suspension von 8,2 g Aluminiumtrichlorid in 50 ml Methylenchlorid eingetragen, wobei die Innentemperatur bei 27°C gehalten wird. Man rührt noch weitere 5 Stunden bei Raumtemperatur nach, giesst das Gemisch dann in 200 ml eiskalte 1N Salzsäure und extrahiert dreimal mit je 200 ml Aethylacetat. Man wäscht die organische Lösung mit Natriumchlorid-Lösung und trocknet sie über wasserfreiem Magnesiumsulfat. Nach Abdampfen der Lösungsmittel und Umkristallisieren aus Aethylacetat und n-Hexan erhält man reines 3,7-Dihydroxyphthalid, Smp. 124-126°C.

Beispiel 107

3,3-Diisopropyl-7-hydroxy-phthalid

Analog dem in Beispiel 102 beschriebenen Verfahren erhält man ausgehend von 1-(2,2-Dimethylpropanoyloxy)-3,5-dioxo-exo -10-oxatricyclo[5.2.1.0(2,6)]dec-8-en und Isopropylmagnesiumjodid über das (3aα,4β,7β,7aα)-Hexahydro-1,1-diisopropyl-3-oxo -4,7-epoxyisobenzofuran-4-yl-pivalat das 3,3-Diisopropyl-7-hydroxy-phthalid.

Beispiele 108-117

Analog dem in Beispiel 103 beschriebenen Verfahren erhält man aus 2-Methoxy-N,N-diäthyl-benzamid, Butyllithium und dem diesbezüglichen Aldehyd bzw. Keton $R^{3''}R^4CO$ (siehe Reaktionsschema 2) die in der nachfolgenden Tabelle 6 angegebenen Ausgangsmaterialien der Formel IIh' oder IIi":

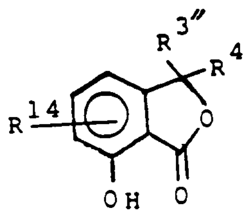

IIh'

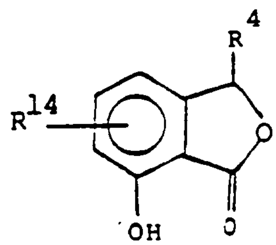

IIi"

Tabelle 6

| Beispiel | Formel IIh' oder IIi" | $R^{3''}$ | $R^4$ | $R^{14}$ | Physikalische Daten |
|---|---|---|---|---|---|
| 108 | IIh' | tert.Butyl | H | H | |
| 109 | IIh' | Aethyl | Methyl | H | Flüssigkeit |
| 110 | IIh' | Phenyl | H | H | Smp. 166-167 °C |
| 111 | IIi" | - | Methyl | H | Smp. 107-108 °C |
| 112 | IIi" | - | H | H | Festkörper |
| 113 | IIi" | - | Methyl | 6-Methyl | Smp. 44-45 °C |
| 114 | IIi" | - | Methyl | 4-Chlor | Smp. 114-115 °C |
| 115 | IIi" | - | Methyl | 4-Brom | Smp. 105-107 °C |
| 116 | IIi" | - | n-Propyl | H | $^1$H-NMR ($CDCl_3$): 5,50 ppm (doppel-d, 8x4 Hz, 1H) |
| 117 | IIi" | - | n-Butyl | H | $^1$H-NMR ($CDCl_3$): 5,50 ppm (doppel-d, 8x4 Hz, 1H) |

Beispiel 118

7-Hydroxy-3-methoxy-phthalid

Eine Lösung von 2,5 g 3,7-Dihydroxyphthalid (als Rohprodukt - siehe Beispiele 105 und 106) in 70 ml Methanol wird mit 4 Tropfen konzentrierter Schwefelsäure versetzt und nach anschliessender Zugabe von 3A-Molekularsieb ca. 16 Stunden stehen gelassen. Dann filtriert man das Gemisch und dampft das Filtrat ein. Das Rohprodukt, 3 g gelbes Oel, wird an Kieselgel unter Verwendung von Aethylacetat/n-Hexan (3:7) chromatographisch gereinigt (Flashchromatographie). Auf diese Weise erhält man 1,9 g (70% der theoretischen Ausbeute) 7-Hydroxy-3-methoxy-phthalid als rötliche Kristalle, Smp. 77-79 °C (nach Kristallisation aus Diäthyläther/n-Hexan); IR-Spektrum ($CHCl_3$): C = O 1745 $cm^{-1}$.

Beispiele 119-129

Analog dem in Beispiel 118 beschriebenen Verfahren erhält man aus 3,7-Dihydroxyphthalid bzw. 3,7-Dihydroxy-3-methyl-phthalid und der diesbezüglichen Hydroxyverbindung in Gegenwart einer katalytischen Menge Schwefelsäure die in der nachfolgenden Tabelle 7 angegebenen Ausgangsmaterialien der Formel IIc' oder IIf':

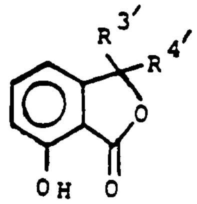

IIc'

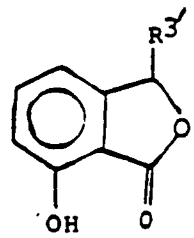

IIf'

| Beispiel | Formel IIc'oder IIf' | $R^{3'}$ | $R^{4'}$ | Physikalische Daten |
|---|---|---|---|---|
| 119 | IIf' | Isopropoxy | - | Smp. 98-100 °C |
| 120 | IIf' | Benzyloxy | - | Smp. 94-95 °C |
| 121 | IIf' | tert.Butoxy | - | Smp. 144-146 °C |
| 122 | IIf' | Aethoxy | - | $^1$H-NMR ($CDCl_3$): 6,39 ppm (s, 1H) |
| 123 | IIc' | Methoxy | Methyl | IR-Spektrum ($CHCl_3$): C=O 1738 $cm^{-1}$ |
| 124 | IIc' | 2-Chloräthoxy | H | Smp. 117-119 °C |
| 125 | IIc' | Propargyloxy | H | Smp. 94-96 °C |
| 126 | IIc' | n-Propoxy | H | Smp. 63-64 °C |
| 127 | IIc' | 2-Methoxy-äthoxy | H | Smp. 79-80 °C |
| 128 | IIc' | 2-Methylthio-äthoxy | H | |
| 129 | IIc' | n-Butoxy | H | |

Beispiel 130

7-Hydroxy-3-methoxy-3-methyl-phthalid

Die Titelverbindung (siehe auch Beispiel 123) kann auch wie folgt hergestellt werden:

1,5 g 3-Hydroxy-7-methoxy-3-methyl-phthalid (siehe folgendes Beispiel 131) werden in 50 ml mit Chlorwasserstoff gesättigtem Methanol eine Stunde bei Raumtemperatur stehen gelassen, und das Gemisch wird danach in Aethylacetat aufgenommen und mit eiskalter Natriumchloridlösung gewaschen. Nach Entfernen der Lösungsmittel erhält man das 3,7-Dimethoxy-3-methyl-phthalid, Smp. 101-104 °C.

1,45 g des obigen Produktes werden in 20 ml Methylenchlorid mit 3,4 g Aluminiumtrichlorid 45 Minuten rühren gelassen, und das Gemisch wird dann mit Eiswasser versetzt und zweimal mit Methylenchlorid extrahiert. Die organische Phase wird über wasserfreiem Magnesiumsulfat getrocknet und unter vermindertem Druck eingeengt. Man erhält so das 7-Hydroxy-3-methoxy-3-methyl-phthalid, Smp. 106-109 °C; IR-Spektrum ($CHCl_3$), C=O 1740 $cm^{-1}$.

Beispiel 131

3,7-Dihydroxy-7-methoxy-3-methyl-phthalid

5,4 g 3-Methoxy-phthalsäureanhydrid [siehe A.V.R. Rao et al., Indian J. Chem. 20B, 248 ff. (1981)], 4,7 g Malonsäure und 9 ml Triäthylamin werden langsam erhitzt. Dabei wird die Suspension bei 55 °C rührbar, und ab 68 °C erfolgt eine mässig starke $CO_2$-Entwicklung. Man hält die Innentemperatur bis zur Beendigung der Gasentwicklung bei 73 °C (ca. 1 Stunde) und erhitzt dann noch weitere 4 Stunden auf Rückflusstemperatur (Innentemperatur 85 °C). Das erkaltete Reaktionsgemisch wird mit Aethylacetat gegen Wasser extrahiert. Die wässrige Phase wird dann auf pH 1,7 gestellt und mit frischem Aethylacetat dreimal

extrahiert, über wasserfreiem Natriumsulfat getrocknet und unter vermindertem Druck eingedampft. Durch fraktionierte Kristallisation (Aethylacetat/n-Hexan) erhält man das 3-Hydroxy-7-methoxy-3-methyl-phthalid, Smp. 156-158 °C; IR-Spektrum ($CHCl_3$): C=O 1772 $cm^{-1}$.

1,9 g des obigen Produktes wird in 20 ml Methylenchlorid gelöst, und die Lösung wird bei -70 °C mit 3,5 g Bortribromid behandelt. Man nimmt das Gemisch dann in eiskalte verdünnte Salzsäure auf und extrahiert die organische Phase mit Aethylacetat, trocknet sie über wasserfreiem Natriumsulfat und dampft sie unter vermindertem Druck ein. Auf diese Weise erhält man als amorphen Feststoff rohes 3,7-Dihydroxy-3-methyl-phthalid, das eventuell ungereinigt zur Herstellung eines weiteren Ausgangsmaterials der Formel II (z.B. 7-Hydroxy-3-methoxy-3-methyl-phthalid) verwendet werden kann.

Beispiel 132

4-Hydroxy-1,3-dihydro-3-oxo-1-isobenzofurancarboxamid

2,8 g 3,7-Dihydroxy-phthalid (siehe Beispiele 105 und 106) wird zu einer Lösung von 7,5 g Kaliumcyanid in 35 ml Wasser eingetragen, und das Gemisch wird unter Eiskühlung langsam mit 25 ml konzentrierter Salzsäure versetzt, so dass die Innentemperatur nicht über 10 °C steigt. Man hält das Reaktionsgemisch weitere 5 Stunden bei Raumtemperatur und filtriert dann die Kristalle ab. Das mit Wasser gewaschene und gut getrocknete Produkt (Smp. 213-215 °C) ist reines 4-Hydroxy-1,3-dihydro-3-oxo-1-isobenzofurancarboxamid.

Beispiel 133

7-Hydroxy-3-trifluormethyl-phthalid

Ein Gemisch von 26,4 g N,N-Dimethyl-2-methoxy-benzamid und 19 ml Tetramethyläthylendiamin in 120 ml absolutem Tetrahydrofuran wird bei -70 °C mit 100 ml einer 1,4-molaren Lösung von sek.Butyllithium in Cyclohexan/Isooctan behandelt und nach 45 Minuten innert 20 Minuten tropfenweise mit 27,3 ml Trifluoracetessigester versetzt. Man lässt ca. 16 Stunden auf Raumtemperatur kommen, versetzt mit 200 ml Wasser und säuert mit Salzsäure auf pH 2 an. Das Gemisch wird mit insgesamt 600 ml Diäthyläther extrahiert, und die organische Phase über wasserfreiem Magnesiumsulfat getrocknet und unter vermindertem Druck eingedampft. Der Rückstand wird an Kieselgel chromatographiert (Laufmittel:Aethylacetat/n-Hexan 1:1). Man erhält auf diese Weise das 3-Hydroxy-7-methoxy-3-trifluormethyl-phthalid, IR-Spektrum ($CHCl_3$): C=O 1785 $cm^{-1}$.

11,1 g des obigen Produktes wird in 140 ml Aethanol portionenweise mit 1,5 g Natriumborhydrid bei Raumtemperatur versetzt. Man nimmt in salzsaures Eiswasser auf und extrahiert mit frischem Diäthyläther. Nach Entfernen der Lösungsmittel und anschliessender Chromatographie erhält man reines 7-Methoxy-3-trifluormethyl-phthalid, IR-Spektrum ($CHCl_3$): C=O 1785 $cm^{-1}$.

4,6 g des obigen Produktes werden in 30 ml Tetrahydrofuran bei -70 °C tropfenweise mit 7,0 g Bortribromid behandelt. Man lässt langsam auf Raumtemperatur kommen und schüttelt dann mit Aethylacetat gegen Eiswasser aus. Die Lösungsmittel werden entfernt und das Rohprodukt über Kieselgel filtriert (Laufmittel:Aethylacetat/n-Hexan 1:1). Man erhält kristallines 7-Hydroxy-3-trifluormethyl-phthalid, IR-Spektrum ($CHCl_3$): C=O 1766 $cm^{-1}$; $^1$H-NMR ($CDCl_3$): 5,68 ppm (q, 6 Hz, 1H).

Beispiel 134

3,7-Dihydroxy-3-trifluormethyl-phthalid

Die Titelverbindung kann analog dem obigen Beispiel (133) aus 3-Hydroxy-7-methoxy-3-trifluormethyl-phthalid (siehe Beispiel 133) und Bortribromid hergestellt werden; Smp. 128-129 °C.

Beispiel 135

7-Mercapto-3-methyl-phthalid

24 g 7-Hydroxy-3-methylphthalid werden in eine wässrige Lösung von 8,2 g Kaliumhydroxid in 130 ml Wasser eingetragen, und das Gemisch wird dann tropfenweise unter gutem Rühren und Kühlen mit 23,5 g Dimethylcarbamoylchlorid behandelt. Nach 90 Minuten wird mit Natriumhydroxid-Lösung auf pH 11 gestellt,

Eis zugegeben und zweimal mit Aethylacetat extrahiert. Nach Trocknen und Abdampfen des Lösungsmittels sowie ümkristallisieren aus Aethylacetat und n-Hexan erhält man das O-(1,3-Dihydro-3-methyl-1-oxo -7-isobenzofuranyl)-dimethylthiocarbamat, Smp. 163-164 ° C.

26 g des obigen Produktes werden in 180 ml Resorcin-dimethyläther aufgenommen, und das Gemisch wird 24 Stunden auf 212 ° C erhitzt. Das erkaltete Reaktionsgemisch wird mit Aethylacetat/n-Hexan (2:1) als Laufmittel durch Kieselgel filtriert und nach Entfernen der Lösungsmittel umkristallisiert. Man erhält das S-(1,3-Dihydro-3-methyl -1-oxo-7-isobenzofuranyl)-dimethylthiocarbamat, Smp. 144-145 ° C.

18,4 g des obigen Produktes in einem Gemisch von 120 ml Methanol und 60 ml Chloroform wird mit einer Lösung von 4,2 g Natrium in 180 ml Methanol behandelt. Man rührt ca. 3 Stunden bei Raumtemperatur und fügt dann 390 ml Aethylacetat zu. Die Lösungsmittel werden nun grösstenteils unter vermindertem Druck entfernt, und der Rückstand wird mit Aethylacetat gegen gesättigte Natriumchlorid-Lösung ausgeschüttelt. Durch Filtration an Kieselgel (Laufmittel: Aethylacetat/n-Hexan 1:2) erhält man reines 7-Mercapto-3-methyl-phthalid, Smp. 40 ° C.

Beispiel 136

7-Hydroxy-3-methyl-isobenzofuran-1(3H)-thion

3,9 g 7-Hydroxy-3-methyl-phthalid (siehe Beispiele 101 und 111) und 5,1 g Lawesson-Reagens werden in 20 ml Xylol 4 Stunden auf 140 ° C erhitzt. Durch chromatographische Filtration des Gemisches an Kieselgel [Laufmittel: Aethylacetat/n-Hexan (1:9)] erhält man das 7-Hydroxy-3-methyl-isobenzofuran-1(3H)-thion, Smp. 39-41 ° C; IR-Spektrum ($CHCl_3$): C = O 1624, 1604, 1368, 1330, 1304, 1165 $cm^{-1}$; Massenspektrum: 180 ($M^+$ = 100), 165(58), 137(24).

Beispiel 137

7-Hydroxy-3-vinyl-phthalid

600 mg 3,7-Dihydroxyphthalid (siehe Beispiele 105 und 106) werden in 20 ml absolutem Tetrahydrofuran bei -78 ° C gerührt. Unter einer Argon-Atmosphäre werden dann 6 ml einer 2m Vinylmagnesiumchloridlösung in Tetrahydrofuran mittels Spritze zugegeben. Man lässt das Reaktionsgemisch auf Raumtemperatur kommen und giesst es auf 100 ml 1N Salzsäure. Man extrahiert anschliessend zweimal mit je 75 ml Aethylacetat und wäscht die vereinigten organischen Phasen mit Kochsalzlösung, trocknet mit wasserfreiem Magnesiumsulfat und engt die organische Lösung unter vermindertem Druck ein. Es verbleiben 600 mg Rohprodukt, das man an Kieselgel mit Aethylacetat/n-Hexan (1:1) einer Flashchromatographie unterwirft.

Auf diese Weise erhält man 380 mg (60% der theoretischen Ausbeute) 7-Hydroxy-3-vinyl-phthalid.

Beispiel 138

7-Hydroxy-3-isopropyliden-phthalid

(i) Analog dem in Beispiel 104 beschriebenen Verfahren werden 10 g 1-(2,2-Dimethylpropanoyloxy)-3,5-dioxo-exo -10-oxatricyclo[5.2.1.0(2,6)]dec-8-en in 25 ml Tetrahydrofuran bei -35 ° C vorgelegt, und die Lösung wird dann mit 3,9 g Isopropylmagnesiumchlorid in 30 ml Tetrahydrofuran versetzt. Man lässt das Gemisch auf Raumtemperatur kommen, gibt dann 27 ml 2N Salzsäure zu, extrahiert zweimal mit Diäthyläther, trocknet die organische Phase über wasserfreiem Magnesiumsulfat und dampft sie ein. Auf diese Weise erhält man das (3a$\alpha$,4$\beta$,7$\beta$,7a$\alpha$)-Hexahydro-1-hydroxy-1-isopropyl -3-oxo-4,7-epoxyisobenzofuran-4-yl-pivalat als Rohprodukt.

(ii) Das so erhaltene Rohprodukt wird bei 0 ° C in konzentrierte Schwefelsäure aufgenommen und die Lösung 20 Minuten gerührt. Man giesst die Lösung anschliessend auf Eis, extrahiert das wässrige Gemisch mit Diäthyläther und trocknet die organische Phase über wasserfreiem Magnesiumsulfat. Das ölige Rohprodukt wird anschliessend mit einer 20%igen Lösung von Diäthyläther in n-Hexan chromatographisch aufgetrennt. Man erhält auf diese Weise das 7-Hydroxy-3-isopropyliden-phthalid als nicht weiter charakterisierten Feststoff.

Beispiel 139

(Z)-3-Aethyliden-7-hydroxy-phthalid

Zu einer Lösung von 3,6 g Natrium in 130 ml Methanol werden unter Stickstoff bei 0-10 °C 14,5 ml Dimethylphosphit zugetropft und gleichzeitig portionenweise 20 g 3-Hydroxy-7-methoxy-phthalid (siehe Beispiel 118) eingetragen. Man lässt dann 30 Minuten bei Raumtemperatur nachrühren und gibt dann innert 10 Minuten 11,3 ml Methansulfonsäure zu. Nach einer weiteren Stunde wird das Methanol unter vermindertem Druck grösstenteils abdestilliert, der Rückstand auf 400 ml verdünnte Salzsäure und Eis gegossen und das wässrige Gemisch dreimal mit 900 ml Aethylacetat extrahiert. Man wäscht die organische Phase mit Natriumchloridlösung, trocknet sie über wasserfreiem Magnesiumsulfat, dampft unter vermindertem Druck ein und kristallisiert den Rückstand aus Aethylacetat/n-Hexan um. Man erhält auf diese Weise reines 3-Dimethoxyphosphonyl -7-methoxy-phthalid, Smp. 129-131 °C.

12,3 g des obigen Produktes werden in 750 ml trockenem Tetrahydrofuran gelöst, und die Lösung wird bei 3 °C mit 5,2 g Kalium-tert.butylat behandelt. Nach einstündigem Nachrühren bei dieser Temperatur werden 2,8 ml Acetaldehyd zugetropft, und das Reaktionsgemisch wird eine weitere Stunde bei 12 °C nachgerührt. Es wird wie oben auf verdünnte Salzsäure und Eis gegossen und mit Aethylacetat extrahiert und die organische Phase mit Wasser und mit Natriumchloridlösung gewaschen, über wasserfreiem Magnesiumsulfat getrocknet und unter vermindertem Druck eingedamapft. Man filtriert durch Kieselgel [Laufmittel: Aethylaetat/n-Hexan (7:3)] und erhält als (E/Z)-Gemisch rohes 7-Methoxy-3-äthyliden-phthalid, $^1$H-NMR ($CDCl_3$): 5,88 ppm und 5,64 ppm (2q, J = 7,5 Hz, (E) und (Z) C$\underline{H}$ = ). Eine Vorfraktion enthält reines (Z)-7-Methoxy-3-äthyliden-phthalid, Smp. 106-109 °C.

1,1 g des obigen Produktes werden zusammen mit 2,8 g Aluminiumtrichlorid in 50 ml Methylenchlorid 2 Stunden bei Raumtemperatur gerührt, und das Gemisch wird dann auf eiskalte, verdünnte Salzsäure gegossen und mit Methylenchlorid extrahiert. Die organische Phase wird mit halbgesättigter Natriumchloridlösung gewaschen, über wasserfreiem Magnesiumsulfat getrocknet und unter vermindertem Druck eingeengt. Man erhält auf diese Weise reines (Z)-3-Aethyliden-7-hydroxy-phthalid, $^1$H-NMR ($CDCl_3$): 5,70 ppm (q, J = 7, 5Hz, C$\underline{H}$ = ).

Beispiele 140-144

Analog dem in Beispiel 139 beschriebenen Verfahren erhält man aus 3-Hydroxy-7-methoxy-phthalid über 3-Dimethoxyphosphonyl-7-methoxy-phthalid, das mit dem diesbezüglichen Aldehyd zur entsprechenden Verbindung XIX umgesetzt wird, die ihrerseits mit Aluminiumtrichlorid behandelt wird (siehe Reaktionsschema 3: XVII→XVIII→XIX→IIk), die in der nachfolgenen Tabelle 8 angegebenen Ausgangsmaterialien der Formel

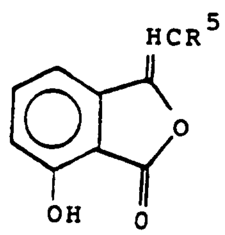

IIk'

| Beispiel | $R^5$ | Physikalische Daten |
|---|---|---|
| 140 | Aethyl | $^1$H-NMR ($CDCl_3$): 5,89 und 5,66 ppm [2q, J = 8Hz, (E) und (Z) CH = ] |
| 141 | 4-Methoxy-phenyl | $^1$H-NMR ($CDCl_3$): 6,40 ppm (s, CH = ) |
| 142 | Phenyl | $^1$H-NMR ($CDCl_3$): 6,45 ppm (s, CH = ) |
| 143 | 3-Methoxy-phenyl | $^1$H-NMR ($CDCl_3$): 6,43 ppm (s, CH = ) |
| 144 | n-Propyl | $^1$H-NMR ($CDCl_3$): 5,90 und 5,67 ppm [2q, J = 8Hz, (E) und (Z) CH = ] |

Beispiel 145

3,4-Dihydro-8-hydroxy-4-methyl-1H-2-benzopyran-1-on

39,5 g N,N-Dimethyl-2-methoxy-benzamid und 28,5 ml Tetramethyläthylendiamin werden in 190 ml absolutem Tetrahydrofuran vorgelegt, und das Gemisch wird bei -70 °C mit 150 ml einer 1,4-molaren sek.-Butyllithium-Lösung in Cyclohexan/Isopentan lithiiert. Nach 45-minütigem Nachrühren bei -70 °C werden innert 25 Minuten 25,6 ml Aethylbromid zugetropft, und das Reaktionsgemisch wird dann langsam auf Raumtemperatur kommen gelassen. Man versetzt dann mit Wasser, stellt mit Salzsäure auf pH 2 und extrahiert zweimal mit Diäthyläther. Der Rückstand wird mit Aethylacetat/n-Hexan (4:6) an Kieselgel gereinigt. Man erhält als farbloses Oel das 6-Aethyl-N,N-dimethyl -2-methoxy-benzamid, $^1$H-NMR ($CDCl_3$): 3,82 ppm (m), 3,40 ppm (m) und 3,12 ppm (2q, $N(\underline{CH_2}CH_3)_2$), 2,56 ppm (m, $\underline{CH_2}CH_3$).

5,8 g des obigen Produktes und 3,5 g Tetramethyläthylendiamin werden bei -70 °C in 150 ml absolutem Tetrahydrofuran mit 24,8 ml 1,4-molarer sek.-Butyllithium-Lösung in Cyclohexan/Isooctan lithiiert und nach 1 Stunde mit 3 g frisch sublimiertem Formaldehyd versetzt. Man lässt langsam auf Raumtemperatur kommen, gibt 30 ml konzentrierte Salzsäure zu (pH 1,5) und extrahiert zweimal mit Aethylacetat. Die Lösungsmittel werden dann unter vermindertem Druck entfernt. Das zurückbleibende ölige Produkt wird in 150 ml 48%-ige Bromwasserstoffsäure aufgenommen und das Gemisch 6 Stunden auf Rückflusstemperatur erhitzt. Man extrahiert mit frischem Aethylacetat, wäscht mit verdünnter Natriumbicarbonatlösung, trocknet über wasserfreiem Magnesiumsulfat und dampft unter vermindertem Druck ein. Das zurückbleibende Produkt wird chromatographiert (Laufmittel: Aethylacetat/n-Hexan 1:3). Man erhält als gelbes Oel das 3,4-Dihydro-8-hydroxy-4-methyl -1H-2-benzopyran-1-on, $^1$H-NMR ($CDCl_3$): 4,56 und 4,27 ppm (2g, 7x11 Hz, 2H), 3,14 ppm (m, 1H), 1,36 ppm (d, 7 Hz, $CH_3$).

Beispiel 146

cis- und trans-3,4-Dihydro-8-hydroxy-3,4-dimethyl-1H-2-benzopyran-1-on

Analog dem in Beispiel 143 beschriebenen Verfahren kann aus 6-Aethyl-N,N-dimethyl-2-methoxy-benzamid und Acetaldehyd das zur Herstellung der Verbindungen der Beispiele 79 und 80 verwendete 3,4-trans- und cis-3,4-Dihydro-8-hydroxy-3,4-dimethyl-1H-2-benzopyran-1-on hergestellt werden, IR-Spektrum ($CHCl_3$): 1675 $cm^{-1}$.

Beispiel 147

7-Hydroxy-3-methyl-isobenzofuran-1(3H)-thion

1,67 g 7-Hydroxy-3-methyl-phthalid (siehe Beispiel 101) und 2,17 g 2,4-Bis-(4-methoxyphenyl)-2,4-dithioxo -1,3,2,4-dithiadiphosphetan (Lawesson Reagens) werden in 8 ml Xylol 3 Stunden auf 138 °C erhitzt. Das erkaltete Reaktionsgut wird direkt über eine Kieselgelsäure [Laufmittel: Aethylacetat/n-Hexan (1:4)] filtriert, um das 7-Hydroxy-3-methyl-isobenzofuran-1(3H)-thion zu isolieren; Smp. 38-40 °C; IR-Spektrum: 1624, 1604, 1368, 1330, 1304, 1165 $cm^{-1}$; Massenspektrum: 180 ($M^+$ = 100), 165 (58), 137 (24).

III. Formulierungsbeispiele

Beispiel 148

Zur Herstellung eines 25% Spritzpulvers werden die nachstehend aufgeführten Bestandteile miteinander vermischt:

| | Gewichtsprozent |
|---|---|
| Erfindungsgemässe Verbindung (Wirkstoff) | 25 |
| Kieselsäure, hydratisiert (Trägerstoff, Mahlhilfsmittel) | 20 |
| Natrium-laurylsulfat (Netzmittel) | 2 |
| Natrium-lignosulfonat (Dispergiermittel) | 4 |
| Kaolin (Trägerstoff) | 49 |
| | 100 |

Es wird vorerst der flüssige bzw. geschmolzene Wirkstoff in einer Mahleinrichtung auf die vorgelegte Kieselsäure aufgetragen. Anschliessend werden die weiteren Bestandteile zugemischt, und das Gemisch wird unter Verwendung einer Stiftmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

Insbesondere erfindungsgemässe Verbindungen, die flüssig sind oder einen niederen Schmelzpunkt, also bis etwa 100 ° C, aufweisen, eignen sich als Wirkstoff in dieser Formulierung.

Beispiel 149

Erfindungsgemässe Verbindungen mit hohen Schmelzpunkten, also ab etwa 100 ° C, können vorzugsweise als Wirkstoffe in konzentrierteren Spritzpulvern verwendet werden, z.B. wie folgt:

| | Gewichtsprozent |
|---|---|
| Erfindungsgemässe Verbindung (Wirkstoff) | 75 |
| Kieselsäure, hydratisiert (Trägerstoff, Mahlhilfsmittel) | 1 |
| Alkylnaphthalinsulfonat und Alkylcarboxylatsulfat als Natriumsalze, z.B. Morwett® EFW (De Soto) [Netzmittel] | 2 |
| Sulfoniertes Naphthalin-Formaldehyd-Kondensat, als Natriumsalz, z.B. Morwett® D-425 (De Soto) [Dispergiermittel] | 10 |
| Polyvinylpyrrolidon, z.B. PVP-K-30 (GAF Corp.) [Bindemittel] | 1 |
| Kaolin (Trägerstoff) | 11 |

Die Bestandteile werden miteinander gemischt und unter Verwendung einer Stiftmühle oder vergleichbaren Mahleinrichtung, insbesondere einer Strahlmühle, fein gemahlen. Beim Einrühren in Wasser ergibt das resultierende Spritzpulver eine feine Suspension beliebiger Konzentration, die sich als gebrauchsfertige Spritzbrühe eignet.

Beispiel 150

Ein Spritzpulver, das auf dem obigen Formulierungsbeispiel (149) basiert, kann auch in ein dispergierbares Granulat übergeführt werden. Dazu wird das gemahlene Pulver in einer geeigneten Granuliervorrichtung (z.B. Granulierteller, Mischtrommel, Intensivmischer oder Wirbelschichtgranulator) mit einer wässrigen Lösung des Bindemittels besprüht, bis sich Agglomerate gebildet haben. Danach wird das zugefügte Wasser in einem Trocknungsprozess wieder entfernt, und die Granulate der gewünschten Grösse werden ausgesiebt. Das resultierende Granulat hat gegenüber dem Spritzpulver verschiedene Vorteile (keine Staubbildung bei der Applikation, leichtere Dosierbarkeit dank besseren Fliesseigenschaften). Die Applikation erfolgt nach Einrühren des Präparates in Wasser und nach vollständigem Zerfall der Granulate in die Primärpartikel genau gleich wie beim Spritzpulver.

Beispiel 151

Die erfindungsgemässen Verbindungen sind in den gängigen organischen Lösungsmitteln beschränkt löslich. Demgemäss sind nur emulgierbare Konzentrate von verhältnismässig tiefer Konzentration möglich; beispielsweise:

| | |
|---|---|
| Erfindungsgemässe Verbindung (Wirkstoff) | 125 g/l |
| Soprophor®BSU (Emulgator, Rhône-Poulenc) | 300 g/l |
| N-Methyl-2-pyrrolidon (Lösungsmittel) | ad 1000 ml |

Der Wirkstoff und der Emulgator werden unter Rühren ins Lösungsmittel eingetragen, und das Gemisch wird gerührt, bis eine homogene Lösung entsteht.

Das resultierende emulgierbare Konzentrat lässt sich in Wasser emulgieren und ergibt so eine gebrauchsfertige Spritzbrühe mit der gewünschten Konzentration.

Beispiel 152

Erfindungsgemässe Verbindungen mit einem Schmelzpunkt ab etwa 60 °C können auch als sogenannte Suspensionskonzentrate ("Flowables") formuliert werden, beispielsweise:

| | |
|---|---|
| Erfindungsgemässe Verbindung (Wirkstoff) | 250 g/l |
| Aethylenglykol (Frostschutz) | 80 g/l |
| Kieselsäure (Antiabsetzmittel) | 5 g/l |
| Xanthan gum, z.B. Kelzan® (Kelco) [Verdickungsmittel] | 2 g/l |
| Silicon-Entschäumer, z.B. Rhodorsil® 426 (Rhône-Poulenc) | 5 g/l |
| Nonylphenol-polyäthoxylat (Netzmittel) | 20 g/l |
| Sulfoniertes Naphthalin-Formaldehyd-Kondensat als Natriumsalz, z.B. Morwett® D-425 (De Soto) (Dispergiermittel) | 40 g/l |
| Wasser | ad 1000 ml |

Die Formulierungshilfsstoffe werden in Wasser gelöst. In der Lösung wird der vorgemahlene Wirkstoff unter Rühren dispergiert. Die resultierende grobe Suspension wird nun einer Nassmahlung unterzogen (z.B. in einer Kolloid-Mühle, Rührwerkkugelmühle). Gegebenenfalls sind nun noch weitere Zusätze in kleinen Mengen möglich, wie Antischaummittel, Antiabsetzmittel und Biozide.

Das resultierende "Flowable" lässt sich zur Anwendung mit Wasser beliebig verdünnen und ergibt so eine gebrauchsfertige Spritzbrühe der gewünschten Konzentration.

**Patentansprüche**

**1.** Verbindungen der allgemeinen Formel

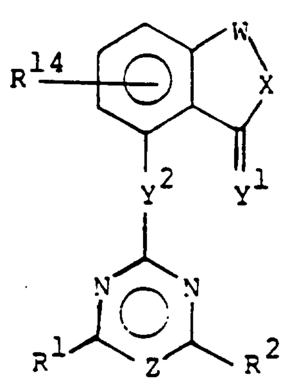

I

worin

W eine der zweiwertigen Gruppen a) - d)

a) 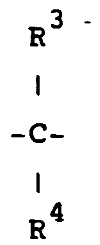 b) 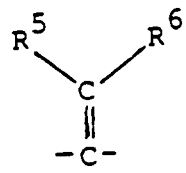 c) 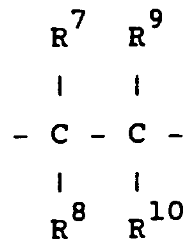

d) 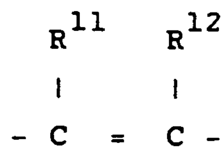

| | |
|---|---|
| X, $Y^1$ und $Y^2$ | jeweils Sauerstoff oder Schwefel, |
| Z | $CR^{13}$ oder Stickstoff, |
| $R^1$ | Wasserstoff, Fluor, Chlor, $C_{1-3}$-Alkyl, Halogenmethyl, Methoxymethyl, $C_{1-3}$-Alkoxy, Difluormethoxy oder Methylthio, |
| $R^2$ | Methyl, $C_{1-2}$-Alkoxy, $C_{1-2}$-Fluoralkoxy, $C_{1-2}$-Alkylamino, Di($C_{1-2}$-alkyl)amino oder N-Methoxymethylamino, |
| $R_3$ | Wasserstoff, Fluor, Chlor, Brom, $C_{1-6}$-Alkenyl, $C_{2-3}$-Alkenyl; $C_{2-3}$-Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, oder durch Halogen, Vinyl, Ethinyl, Cyclopropyl, Phenyl, $C_{1-2}$-Alkoxy, $C_{1-2}$-Alkylthio, Cyano, Carboxy, $C_{2-5}$-Alkoxycarbonyl, Carbamoyl, N-($C_{1-2}$-Alkyl)-carbamoyl, N,N-Di($C_{1-2}$-Alkyl)carbamoyl oder $C_{3-5}$-Alkylideniminooxy substituiertes $C_{1-6}$-Alkoxy, $C_{1-6}$-Alkylthio, Phenoxy, Phenylthio, Cyano, Rhodano, Formyl, Carboxy, $C_{2-5}$-Alkoxycarbonyl, Carbamoyl, Formyloxy, $C_{2-5}$-Alkanoyloxy, $C_{2-5}$-Alkoxycarbonyloxy, $C_{2-3}$-Alkylcarbamoyloxy, Di($C_{1-2}$-alkyl)carbamoyloxy oder Di-($C_{1-2}$-alkoxy)phosphonyl, |
| $R^4$ | Wasserstoff, $C_{1-6}$-Alkyl oder Trifluormethyl, |
| $R^5$ | Wassserstoff, $C_{1-6}$-Alkyl, Phenyl, oder durch Fluor, Chlor, Methyl, Methoxy oder Trifluormethyl substituiertes Phenyl; |
| $R^6$ | Wasserstoff oder Methyl, |
| $R^7$, $R^8$ und $R^9$ | unabhängig voneinander Wasserstoff oder $C_{1-3}$-Alkyl, |
| $R^{10}$ | Wasserstoff oder $C_{1-3}$-Alkoxy, |
| $R^{11}$ und $R^{12}$ | unabhängig voneinander Wasserstoff oder $C_{1-3}$-Alkyl, $R^{13}$ Wasserstoff, Fluor, Chlor oder Methyl |
| | und |
| $R^{14}$ | Wasserstoff, Halogen, $C_{1-2}$-Alkyl oder $C_{1-2}$-Alkoxy bedeuten. |

**2.** Verbindungen nach Anspruch 1, worin W eine Gruppe a) und $R_3$ Wasserstoff, Fluor, Chlor, Brom, $C_{1-6}$-Alkyl, $C_{2-3}$-Alkenyl, $C_{2-3}$-Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, $C_{1-6}$-Alkylthio, Phenoxy, Phenylthio, Cyano oder $C_{2-5}$-Alkoxycarbonyl und $R^4$ Wasserstoff oder $C_{1-6}$-Alkyl bedeuten, oder $R^3$ Wasserstoff, Fluor, Chlor, Brom, $C_{1-6}$-Alkyl, Hydroxy, $C_{1-6}$-Alkoxy, $C_{1-6}$-Alkylthio, Phenoxy, Phenylthio oder Cyano und $R^4$ Trifluormethyl bedeuten; oder W eine Gruppe b), c) oder d) bedeutet, worin $R^5$, $R^6$, $R^7$, $R^8$, $R^9$, $R^{10}$, $R^{11}$ und $R^{12}$ die in Anspruch 1 angegebenen Bedeutungen besitzen; und X Sauerstoff bedeutet,
$R^{13}$ Wasserstoff, Fluor, Chlor oder Methyl, $Y^1$, $Y^2$ und Z die in Anspruch 1 angegebenen Bedeutungen

besitzen und $R^1$ Fluor, Chlor, $C_{1-3}$-Alkyl, Fluormethyl, Methoxymethyl, $C_{1-3}$-Alkoxy, Difluormethoxy oder Methylthio, $R^2$ Methyl, $C_{1-2}$-Alkoxy oder $C_{1-2}$-Fluoralkoxy und $R^{14}$ Wasserstoff bedeuten.

**3.** Verbindungen nach Anspruch 1, worin W eine Gruppe a) bedeutet, in der $R^3$ Wasserstoff, Vinyl, Aethinyl, Hydroxy, $C_{1-4}$-Alkoxy, mit Halogen, Vinyl, Aethinyl, $C_{1-2}$-Alkoxy, Cyano, Carboxymethyl oder $C_{2-3}$-Alkoxycarbonylmethyl substituiertes $C_{1-2}$-Alkoxy, $C_{1-2}$-Alkylthio, Cyano, Carboxy, $C_{2-3}$-Alkoxycarbonyl oder Carbamoyl und $R^4$ Wasserstoff oder $C_{1-4}$-Alkyl bedeuten.

**4.** Verbindungen nach Anspruch 1, worin W eine Gruppe b) bedeutet, in der $R^5$ Wasserstoff oder $C_{1-3}$-Alkyl und $R^6$ Wasserstoff bedeuten.

**5.** Verbindungen nach Anspruch 1, worin W eine Gruppe c) bedeutet, in der $R^7$, $R^8$ und $R^9$ jeweils Wasserstoff oder Methyl bedeuten.

**6.** Verbindungen nach Anspruch 1, worin W eine Gruppe d) bedeutet, in der $R^{11}$ und $R^{12}$ jeweils Wasserstoff oder Methyl bedeuten.

**7.** Verbindungen nach einem der Ansprüche 1 bis 6, worin X und $Y^1$ jeweils Sauerstoff bedeuten.

**8.** Verbindungen nach einem der Ansprüche 1 bis 7, worin $Y^2$ Sauerstoff bedeutet.

**9.** Verbindungen nach einem der Ansprüche 1 bis 8, worin Z CH oder Stickstoff bedeutet.

**10.** Verbindungen nach einem der Ansprüche 1 bis 9, worin $R^1$ Wasserstoff, Chlor, Methyl, Methoxy oder Difluormethoxy und $R^2$ Methoxy, Aethoxy, Methylamino, Dimethylamino oder N-Methoxymethylamino bedeuten.

**11.** Verbindungen nach einem der Ansprüche 1 bis 10, worin mindestens eines von $R^1$ und $R^2$ Methoxy bedeutet.

**12.** Verbindungen nach einem der Ansprüche 1 bis 11, worin $R^{14}$ Wasserstoff bedeutet.

**13.** Verbindungen nach Anspruch 1, ausgewählt aus der Gruppe
7-[(4,6-Dimethoxy-pyrimidin-2-yl)oxy]-3-methyl-phthalid,
7-[(4,6-Dimethoxy-pyrimidin-2-yl)oxy]-phthalid,
3-Aethyl-7-[(4,6-dimethoxy-pyrimidin-2-yl)oxy]-phthalid,
7-[(4,6-Dimethoxy-pyrimidin-2-yl)oxy]-3-isopropyl-phthalid,
7-[(4,6-Dimethoxy-pyrimidin-2-yl)oxy]-3-methoxy-phthalid,
7-[(4-Methoxy-6-methyl-pyrimidin-2-yl)oxy]-3-methyl-phthalid,
7-[(4,6-Dimethoxy-1,3,5-triazin-2-yl)oxy]-3-methyl-phthalid,
(Z)-3-Aethyliden-7-[(4,6-dimethoxy-pyrimidin-2-yl)oxy]-phthalid,
8-[(4,6-Dimethoxy-pyrimidin-2-yl)oxy]-3-methyl-isochroman -1-on,
7-[(4,6-Dimethoxy-pyrimidin-2-yl)thio]-3-methyl-phthalid,
3-Aethoxy-7-[(4,6-dimethoxy-pyrimidin-2-yl)oxy]-phthalid,
7-[(4,6-Dimethoxy-pyrmidin-2-yl)oxy]-3-methoxy-3-methyl-phthalid,
7-[(4-Chlor-6-methoxy-pyrimidin-2-yl)oxy]-3-methyl-phthalid,
7-[(4-Aethoxy-6-methoxy-pyrimidin-2-yl)oxy]-3-methyl-phthalid,
7-[(4,6-Dimethoxy-1,3,5-triazin-2-yl)thio]-3-methyl-phthalid,
7-[(4-Methoxy-6-methyl-1,3,5-triazin-2-yl)oxy]-3-methyl-phthalid,
3-Aethyl-7-[(4,6-dimethoxy-1,3,5-triazin-2-yl)oxy]-phthalid,
8-[(4,6-Dimethoxy-pyrimidin-2-yl)oxy]-3,4-dimethyl-isochroman-2-on,
7-[(4,6-Dimethoxy-pyrimidin-2-yl)oxy]-3-methylthio-phthalid,
7-[(4,6-Dimethoxy-pyrimidin-2-yl)oxy]-3-vinyl-phthalid, und 3-Cyano-7-[(4,6-dimethoxy-pyrimidin-2-yl)oxy]-phthalid.

**14.** Verbindungen nach Anspruch 1, ausgewählt aus der Gruppe
7-[(4,6-Dimethoxy-pyrmidin-2-yl)oxy]-3,6-dimethyl-phthalid,
3-Carbamoyl-7-[(4,6-dimethoxy-pyrimidin-2-yl)oxy]-phthalid,

3-(2-Chloräthoxy)-7-[(4,6-dimethoxy-pyrimidin-2-yl)oxy]-phthalid,
7-[(4,6-Dimethoxy-pyrimidin-2-yl)oxy]-3-propargyloxy-phthalid,
7-[(4,6-Dimethoxy-pyrimidin-2-yl)oxy]-3-(n-propoxy)-phthalid,
7-[(4,6-Dimethoxy-pyrimidin-2-yl)oxy]-3-(2-methoxy-äthoxy)-phthalid,
7-[(4-Methoxy-pyrimidin-2-yl)oxy]-3-methyl-phthalid,
7-[(4-Chlor-6-methoxy-pyrimidin-2-yl)oxy]-3-methoxy-phthalid,
7-[(4-Dimethylamino-6-methoxy-1,3,5-triazin-2-yl)oxy]-3-methyl-phthalid,
7-[(4-Methoxy-6-methylamino-1,3,5-triazin-2-yl)oxy]-3-methyl-phthalid,
7-[(4-Chlor-6-methylamino-1,3,5-triazin-2-yl)oxy]-3-methyl-phthalid,
7-[(4,6-Dimethoxy-pyrimidin-2-yl)oxy]-3-hydroxy-phthalid,
3-Cyanomethoxy-7-[(4,6-dimethoxy-pyrimidin-2-yl)oxy]-phthalid,
7-[(4,6-Dimethoxy-pyrimidin-2-yl)oxy]-3-(methoxycarbonylmethoxy)-phthalid,
3-Aethoxycarbonylmethyl-7-[(4,6-dimethoxy-pyrimidin-2-yl)oxy]-phthalid,
7-[(4,6-Dimethoxy-pyrimidin-2-yl)oxy]-3-methyl-2-benzothiophen-1(3H)-on,
7-[(4,6-Dimethoxy-pyrimidin-2-yl)oxy]-3-methyl-isobenzofuran-1(3H)-thion und
7-[(4,6-Dimethoxy-1,3,5-triazin-2-yl)oxy]-3-methyl-isobenzofuran-1(3H)-thion.

**15.** Unkrautbekämpfungsmittel und pflanzenwachstumsregulierendes Mittel, dadurch gekennzeichnet, dass es eine wirksame Menge mindestens einer Verbindung der allgemeinen Formel

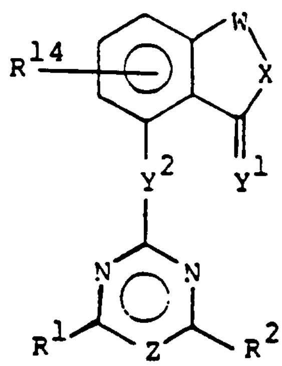

I

worin

W eine der zweiwertigen Gruppen a) - d)

a) 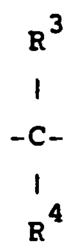 b) 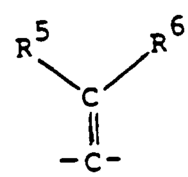 c) 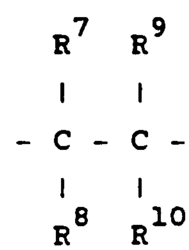

d) 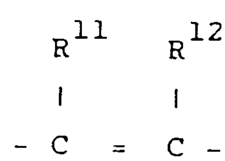

X, $Y^1$ und $Y^2$ jeweils Sauerstoff oder Schwefel,
Z $CR^{13}$ oder Stickstoff,

| | |
|---|---|
| $R^1$ | Wasserstoff, Fluor, Chlor, $C_{1-3}$-Alkyl, Halogenmethyl, Methoxymethyl, $C_{1-3}$-Alkoxy, Difluormethoxy oder Methylthio, |
| $R^2$ | Methyl, $C_{1-2}$-Alkoxy, $C_{1-2}$-Fluoralkoxy, $C_{1-2}$-Alkylamino, Di($C_{1-2}$-alkyl)amino oder N-Methoxymethylamino, |
| $R_3$ | Wasserstoff, Fluor, Chlor, Brom, $C_{1-6}$-Alkyl, $C_{2-3}$-Alkenyl, $C_{2-3}$Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, oder durch Halogen, Vinyl, Ethinyl, Cyclopropyl, Phenyl, $C_{1-2}$-Alkoxy, $C_{1-2}$-Alkylthio, Cyano, Carboxy, $C_{2-5}$-Alkoxycarbonyl, Carbamoyl, N-($C_{1-2}$-Alkyl)-carbamoyl, N,N-Di($C_{1-2}$-Alkyl)carbamoyl oder $C_{3-5}$-Alkylideniminooxy substituiertes $C_{1-6}$-Alkoxy, $C_{1-6}$-Alkylthio, Phenoxy, Phenylthio, Cyano, Rhodano, Formyl, Carboxy, $C_{2-5}$-Alkoxycarbonyl, Carbamoyl, Formyloxy, $C_{2-5}$-Alkanoyloxy, $C_{2-5}$-Alkoxycarbonyloxy, $C_{2-3}$-Alkylcarbamoyloxy, Di($C_{1-2}$-alkyl)carbamoyloxy oder Di-($C_{1-2}$-alkoxy)phosphonyl, |
| $R^4$ | Wasserstoff, $C_{1-6}$-Alkyl oder Trifluormethyl, |
| $R^5$ | Wassserstoff, $C_{1-6}$-Alkyl, Phenyl, oder durch Fluor, Chlor, Methyl, Methoxy oder Trifluormethyl substituiertes Phenyl; |
| $R^6$ | Wasserstoff oder Methyl, |
| $R^7$, $R^8$ und $R^9$ | unabhängig voneinander Wasserstoff oder $C_{1-3}$-Alkyl, |
| $R^{10}$ | Wasserstoff oder $C_{1-3}$-Alkoxy, |
| $R^{11}$ und $R^{12}$ | unabhängig voneinander Wasserstoff oder $C_{1-3}$-Alkyl, |
| $R^{13}$ | Wasserstoff, Fluor, Chlor oder Methyl |
| | und |
| $R^{14}$ | Wasserstoff, Halogen, $C_{1-2}$-Alkyl oder $C_{1-2}$-Alkoxy bedeuten, sowie Formulierungshilfsstoffe enthält. |

**16.** Unkrautbekämpfungsmittel nach Anspruch 15, dadurch gekennzeichnet, dass es eine wirksame Menge mindestens einer aus der Gruppe
7-[(4,6-Dimethoxy-pyrimidin-2-yl)oxy]-3-methyl-phthalid,
7-[(4,6-Dimethoxy-pyrimidin-2-yl)oxy]-phthalid,
3-Aethyl-7-[(4,6-dimethoxy-pyrimidin-2-yl)oxy]-phthalid,
7-[(4,6-Dimethoxy-pyrimldin-2-yl)oxy]-3-isopropyl-phthalid,
7-[(4,6-Dimethoxy-pyrimidin-2-yl)oxy]-3-methoxy-phthalid,
7-[(4-Methoxy-6-methyl-pyrimidin-2-yl)oxy]-3-methyl-phthalid,
7-[(4,6-Dimethoxy-1,3,5-triazin-2-yl)oxy]-3-methyl-phthalid,
(Z)-3-Aethyliden-7-[(4,6-dimethoxy-pyrimidin-2-yl)oxy]-phthalid,
8-[(4,6-Dimethoxy-pyrimidin-2-yl)oxy]-3-methyl-isochroman -1-on,
7-[(4,6-Dimethoxy-pyrimidin-2-yl)thio]-3-methyl-phthalid,
3-Aethoxy-7-[(4,6-dimethoxy-pyrimidin-2-yl)oxy]-phthalid,
7-[(4,6-Dimethoxy-pyrmidin-2-yl)oxy]-3-methoxy-3-methyl-phthalid,
7-[(4-Chlor-6-methoxy-pyrimidin-2-yl)oxy]-3-methyl-phthalid,
7-[(4-Aethoxy-6-methoxy-pyrimidin-2-yl)oxy]-3-methyl-phthalid,
7-[(4,6-Dimethoxy-1,3,5-triazin-2-yl)thio]-3-methyl-phthalid,
7-[(4-Methoxy-6-methyl-1,3,5-triazin-2-yl)oxy]-3-methyl-phthalid,
3-Aethyl-7-[(4,6-dimethoxy-1,3,5-triazin-2-yl)oxy]-phthalid,
8-[(4,6-Dimethoxy-pyrimidin-2-yl)oxy]-3,4-dimethyl-isochroman-2-on,
7-[(4,6-Dimethoxy-pyrimidin-2-yl)oxy]-3-methylthio-phthalid,
7-[(4,6-Dimechoxy-pyrimidin-2-yl)oxy]-3-vinyl-phthalid, und 3-Cyano-7-[(4,6-dimethoxy-pyrimidin-2-yl)oxy]-phthalid, ausgewählten Verbindung sowie Formulierungshilfsstoffe enthält.

**17.** Unkrautbekämpfungsmittel und pflanzenwachstumsregulierendes Mittel nach Anspruch 15, dadurch gekennzeichnet, dass es eine wirksame Menge mindestens einer aus der Gruppe
7-[(4,6-Dimethoxy-pyrimidin-2-yl)oxy]-3,6-dimethyl-phthalid,
3-Carbamoyl-7-[(4,6-dimethoxy-pyrimidin-2-yl)oxy]-phthalid,
3-(2-Chloräthoxy)-7-[(4,6-dimethoxy-pyrimidin-2-yl)oxy]-phthalid,
7-[(4,6-Dimethoxy-pyrimidin-2-yl)oxy]-3-propargyloxy-phthalid,

7-[(4,6-Dimethoxy-pyrimidin-2-yl)oxy]-3-(n-propoxy)-phthalid,
7-[(4,6-Dimethoxy-pyrimidin-2-yl)oxy]-3-(2-methoxy-äthoxy)-phthalid,
7-[(4-Methoxy-pyrimidin-2-yl)oxy]-3-methyl-phthalid,
7-[(4-Chlor-6-methoxy-pyrimidin-2-yl)oxy]-3-methoxy-phthalid,
7-[(4-Dimethylamino-6-methoxy-1,3,5-triazin-2-yl)oxy]-3-methyl-phthalid,
7-[(4-Methoxy-6-methylamino-1,3,5-triazin-2-yl)oxy]-3-methyl-phthalid,
7-[(4-Chlor-6-methylamino-1,3,5-triazin-2-yl)oxy]-3-methyl-phthalid,
7-[(4,6-Dimethoxy-pyrimidin-2-yl)oxy]-3-hydroxy-phthalid,
3-Cyanomethoxy-7-[(4,6-dimechoxy-pyrimidin-2-yl)oxy]-phthalid,
7-[(4,6-Dimethoxy-pyrimidin-2-yl)oxy]-3-(methoxycarbonyl-methoxy)-phthalid,
3-Aethoxycarbonylmethyl-7-[(4,6-dimethoxy-pyrimidin-2-yl)oxy]-phthalid,
7-[(4,6-Dimethoxy-pyrimidin-2-yl)oxy]-3-methyl-2-benzothiophen-1(3H)-on,
7-[(4,6-Dimethoxy-pyrimidin-2-yl)oxy]-3-methyl-isobenzofuran-1(3H)-thion und
7-[(4,6-Dimethoxy-1,3,5-triazin-2-yl)oxy]-3-methyl-isobenzofuran-1(3H)-thion ausgewählten Verbindung sowie Formulierungshilfsstoffe enthält.

**18.** Verfahren zur Herstellung von Verbindungen der allgemeinen Formel

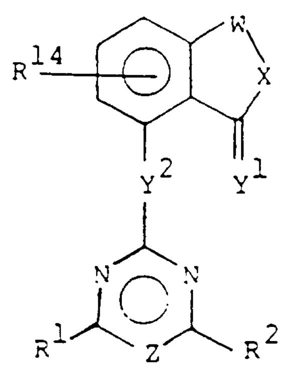

I

worin

W eine der zweiwertigen Gruppen a) - d)

a) 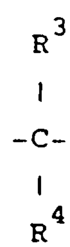

b) 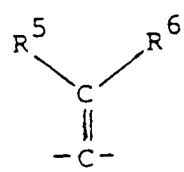

c) 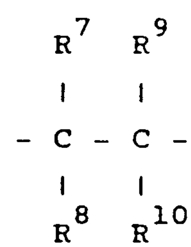

d) 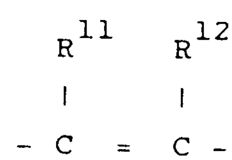

X, $Y^1$ und $Y^2$ jeweils Sauerstoff oder Schwefel,
Z $CR^{13}$ oder Stickstoff,
$R^1$ Wasserstoff, Fluor, Chlor, $C_{1-3}$-Alkyl, Halogenmethyl, Methoxymethyl, $C_{1-3}$-Al-

koxy, Difluormethoxy oder Methylthio,

$R^2$ Methyl, $C_{1-2}$-Alkoxy, $C_{1-2}$-Fluoralkoxy, $C_{1-2}$-Alkylamino, Di($C_{1-2}$-alkyl)amino oder N-Methoxymethylamino,

$R_3$ Wasserstoff, Fluor, Chlor, Brom, $C_{1-6}$-Alkyl, $C_{2-3}$-Alkenyl, $C_{2-3}$-Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, oder durch Halogen, Vinyl, Ethinyl, Cyclopropyl, Phenyl, $C_{1-2}$-Alkoxy, $C_{1-2}$-Alkylthio, Cyano, Carboxy, $C_{2-5}$-Alkoxycarbonyl, Carbamoyl, N-($C_{1-2}$-Alkyl)-carbamoyl, N,N-Di($C_{1-2}$-Alkyl)carbamoyl oder $C_{3-5}$-Alkylideniminooxy substituiertes $C_{1-6}$-Alkoxy, $C_{1-6}$-Alkylthio, Phenoxy, Phenylthio, Cyano, Rhodano, Formyl, Carboxy, $C_{2-5}$-Alkoxycarbonyl, Carbamoyl, Formyloxy, $C_{2-5}$-Alkanoyloxy, $C_{2-5}$-Alkoxycarbonyloxy, $C_{2-3}$-Alkylcarbamoyloxy, Di($C_{1-2}$-alkyl)carbamoyloxy oder Di-($C_{1-2}$-alkoxy)phosphonyl,

$R^4$ Wasserstoff $C_{1-6}$-Alkyl oder Trifluormethyl,

$R^5$ Wassserstoff, $C_{1-6}$-Alkyl, Phenyl, oder durch Fluor, Chlor, Methyl, Methoxy oder Trifluormethyl substituiertes Phenyl;

$R^6$ Wasserstoff oder Methyl,

$R^7$, $R^8$ und $R^9$ unabhängig voneinander Wasserstoff oder $C_{1-3}$-Alkyl,

$R^{10}$ Wasserstoff oder $C_{1-3}$-Alkoxy,

$R^{11}$ und $R^{12}$ unabhängig voneinander Wasserstoff oder $C_{1-3}$-Alkyl,

$R^{13}$ Wasserstoff, Fluor, Chlor oder Methyl und

$R^{14}$ Wasserstoff, Halogen, $C_{1-2}$-Alkyl oder $C_{1-2}$-Alkoxy bedeuten,

dadurch gekennzeichnet, dass man eine Verbindung der allgemeinen Formel

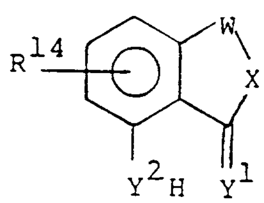

II

worin W, X, $Y^1$, $Y^2$ und $R^{14}$ die oben angegebenen Bedeutungen besitzen,
mit einer Verbindung der allgemeinen Formel

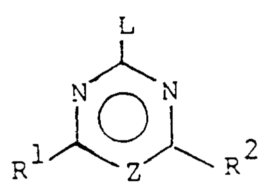

III

worin $R^1$, $R^2$ und Z die oben angegebenen Bedeutungen besitzen und L eine Abgangsgruppe bedeutet,
umsetzt.

**19.** Verfahren nach Anspruch 18, dadurch gekennzeichnet, dass es zur Herstellung einer Verbindung der Formel I dient, worin W eine Gruppe a)
und $R_3$ Wasserstoff, Fluor, Chlor, Brom, $C_{1-6}$-Alkyl, $C_{2-3}$-Alkenyl, $C_{2-3}$-Alkinyl; durch Halogen, Hydroxy, Methoxy, Ethoxy, Nitro, Cyano, Vinyl, Ethinyl, Carboxy, $C_{2-5}$-Alkoxycarbonyl, Phenyl, welches seinerseits durch Methoxy substituiert sein kann, substituiertes $C_{1-6}$-Alkyl; $C_{2-3}$-Alkenyl, $C_{2-3}$-Alkinyl, Phenyl, oder durch Fluor, Chlor, Methyl, Methoxy oder Trifluormethyl substituiertes Phenyl, Hydroxy, $C_{1-6}$-Alkoxy, $C_{1-6}$-Alkylthio, Phenoxy, Phenylthio, Cyano oder $C_{2-5}$-Alkoxycarbonyl und $R^4$ Wasserstoff oder $C_{1-6}$-Alkyl bedeuten, oder $R^3$ Wasserstoff, Fluor, Chlor, Brom, $C_{1-6}$-Alkyl, Hydroxy, $C_{1-6}$-

Alkoxy, $C_{1-6}$-Alkylthio, Phenoxy, Phenylthio oder Cyano und $R^4$ Trifluormethyl bedeuten; oder W eine Gruppe b), c) oder d) bedeutet, worin $R^5$, $R^6$, $R^7$, $R^8$, $R^9$, $R^{10}$, $R^{11}$ und $R^{12}$ die in Anspruch 19 angegebenen Bedeutungen besitzen; und X Sauerstoff bedeutet, $Y^1$, $Y^2$ und Z die in Anspruch 19 angegebenen Bedeutungen besitzen und $R^1$ Fluor, Chlor, $C_{1-3}$-Alkyl, Fluormethyl, Methoxymethyl, $C_{1-3}$-Alkoxy, Difluormethoxy oder Methylthio, $R^2$ Methyl, $C_{1-2}$-Alkoxy oder $C_{1-2}$_Fluoralkoxy, $R^{13}$ Wasserstoff, Fluor, Chlor oder Methyl und $R^{14}$ Wasserstoff bedeuten.

**20.** Verfahren zur Bekämpfung von Unkräutern und zur Regulation des Pflanzenwachstums, dadurch gekennzeichnet, dass man das gegen Unkräuter zu schützende Gut und/oder die Unkräuter bzw. die Pflanzen mit einer wirksamen Menge einer Verbindung gemäss einem der Ansprüche 1, 3 bis 6, 10, 11 und 14 bzw. eines Mittels gemäss Anspruch 15 oder 17 behandelt.

**21.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 2, 7 bis 9, 12 und 13 bzw. eines Mittels gemäss Anspruch 16 behandelt.

**22.** Verwendung einer Verbindung gemäss einem der Ansprüche 1, 3 bis 6, 10, 11 und 14 bzw. eines Mittels gemäss Anspruch 15 oder 17 zur Bekämpfung von Unkräutern oder zur Regulation des Pflanzenwachstums.

**23.** Verwendung einer Verbindung gemäss einem der Ansprüche 2, 7 bis 9, 12 und 13 bzw. eines Mittels gemäss Anspruch 16 zur Bekämpfung von Unkräutern.

## Claims

**1.** A compound of the general formula

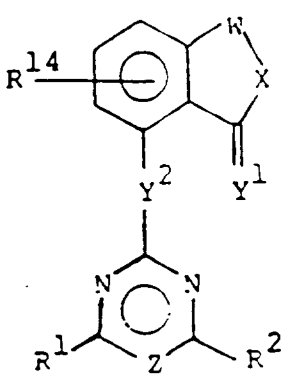

I

wherein

W is one of the divalent groups a) - d)

a) 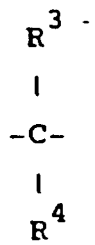

b) 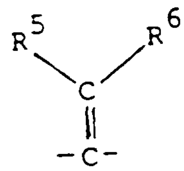

c) 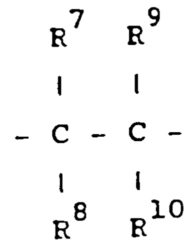

d) 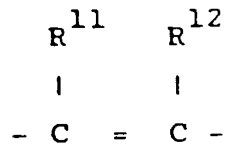

| | |
|---|---|
| X, $Y^1$ and $Y^2$ | are each oxygen or sulfur, |
| Z | is $CR^{13}$ or nitrogen, |
| $R^1$ | is hydrogen, fluorine, chlorine, $C_{1-3}$alkyl, halomethyl, methoxymethyl, $C_{1-3}$alkoxy, difluoromethoxy or methylthio, |
| $R^2$ | is methyl, $C_{1-2}$alkoxy, $C_{1-2}$fluoroalkoxy, $C_{1-2}$alkylamino, di($C_{1-2}$alkyl)amino or N-methoxymethylamino, |
| $R^3$ | is hydrogen, fluorine, chlorine, bromine, $C_{1-6}$alkyl, $C_{2-3}$alkenyl, $C_{2-3}$alkynyl; $C_{1-6}$alkyl substituted by halogen, hydroxy, methoxy, ethoxy, nitro, cyano, vinyl, ethynyl, carboxy, $C_{2-5}$alkoxycarbonyl, or by phenyl that may in turn be substituted by methoxy; $C_{2-3}$alkenyl, $C_{2-3}$alkynyl, phenyl, or phenyl substituted by fluorine, chlorine, methyl, methoxy or by trifluoromethyl; hydroxy, $C_{1-6}$alkoxy, or $C_{1-6}$-alkoxy substituted by halogen, vinyl, ethynyl, cyclopropyl, phenyl, $C_{1-2}$alkoxy, $C_{1-2}$alkylthio, cyano, carboxy, $C_{2-5}$alkoxycarbonyl, carbamoyl, N-($C_{1-2}$alkyl)carbamoyl, N,N-di($C_{1-2}$alkyl)carbamoyl or by $C_{3-5}$alkylideneiminooxy; $C_{1-6}$alkylthio, phenoxy, phenylthio, cyano, thiocyano, formyl, carboxy, $C_{2-5}$alkoxycarbonyl, carbamoyl, formyloxy, $C_{2-5}$-alkanoyloxy, $C_{2-5}$alkoxycarbonyloxy, $C_{2-3}$alkylcarbamoyloxy, di($C_{1-2}$alkyl)carbamoyloxy or di($C_{1-2}$-alkoxy)phosphonyl, |
| $R^4$ | is hydrogen, $C_{1-6}$alkyl or trifluoromethyl, |
| $R^5$ | is hydrogen, $C_{1-6}$alkyl, phenyl, or phenyl substituted by fluorine, chlorine, methyl, methoxy or by trifluoromethyl; |
| $R^6$ | is hydrogen or methyl, |
| $R^7$, $R^8$ and $R^9$ | each independently of the others is hydrogen or $C_{1-3}$alkyl, |
| $R^{10}$ | is hydrogen or $C_{1-3}$alkoxy, |
| $R^{11}$ and $R^{12}$ | each independently of the other is hydrogen or $C_{1-3}$alkyl, |
| $R^{13}$ | is hydrogen, fluorine, chlorine or methyl |
| | and |
| $R^{14}$ | is hydrogen, halogen, $C_{1-2}$alkyl or $C_{1-2}$alkoxy. |

**2.** A compound according to claim 1, wherein W is a group a) and $R^3$ is hydrogen, fluorine, chlorine, bromine, $C_{1-6}$alkyl, $C_{2-3}$alkenyl, $C_{2-3}$alkynyl; $C_{1-6}$alkyl substituted by halogen, hydroxy, methoxy, ethoxy, nitro, cyano, vinyl, ethynyl, carboxy, $C_{2-5}$alkoxycarbonyl or by phenyl that may in turn be substituted by methoxy; $C_{2-3}$alkenyl, $C_{2-3}$alkynyl, phenyl, or phenyl substituted by fluorine, chlorine, methyl, methoxy or by trifluoromethyl; hydroxy, $C_{1-6}$alkoxy, $C_{1-6}$alkylthio, phenoxy, phenylthio, cyano or $C_{2-5}$alkoxycarbonyl and $R^4$ is hydrogen or $C_{1-6}$alkyl, or $R^3$ is hydrogen, fluorine, chlorine, bromine $C_{1-6}$alkyl, hydroxy, $C_{1-6}$alkoxy, $C_{1-6}$alkylthio, phenoxy, phenylthio or cyano and $R^4$ is trifluoromethyl; or W is a group b), c) or d) wherein $R^5$, $R^6$, $R^7$, $R^8$, $R^9$, $R^{10}$, $R^{11}$ and $R^{12}$ are as defined in claim 1; and X is oxygen, $R^{13}$ is hydrogen, fluorine, chlorine or methyl, $Y^1$, $Y^2$ and Z are as defined in claim 1, and $R^1$ is fluorine, chlorine, $C_{1-3}$alkyl, fluoromethyl, methoxymethyl, $C_{1-3}$alkoxy, difluoromethoxy or methylthio, $R^2$ is methyl, $C_{1-2}$alkoxy or $C_{1-2}$fluoroalkoxy, and $R^{14}$ is hydrogen.

3. A compound according to claim 1, wherein W is a group a) wherein $R^3$ is hydrogen, vinyl, ethynyl, hydroxy, $C_{1-4}$alkoxy, $C_{1-2}$alkoxy substituted by halogen, vinyl, ethynyl, $C_{1-2}$alkoxy, cyano, carboxymethyl or by $C_{2-3}$-alkoxycarbonylmethyl, $C_{1-2}$alkylthio, cyano, carboxy, $C_{2-3}$alkoxycarbonyl or carbamoyl, and $R^4$ is hydrogen or $C_{1-4}$alkyl.

4. A compound according to claim 1, wherein W is a group b) wherein $R^5$ is hydrogen or $C_{1-3}$alkyl and $R^6$ is hydrogen.

5. A compound according to claim 1, wherein W is a group c) wherein $R^7$, $R^8$ and $R^9$ are each hydrogen or methyl.

6. A compound according to claim 1, wherein W is a group d) wherein $R^{11}$ and $R^{12}$ are each hydrogen or methyl.

7. A compound according to any one of claims 1 to 6, wherein X and $Y^1$ are each oxygen.

8. A compound according to any one of claims 1 to 7, wherein $Y^2$ is oxygen.

9. A compound according to any one of claims 1 to 8, wherein Z is CH or nitrogen.

10. A compound according to any one of claims 1 to 9, wherein $R^1$ is hydrogen, chlorine, methyl, methoxy or difluoromethoxy, and $R^2$ is methoxy, ethoxy, methylamino, dimethylamino or N-methoxymethylamino.

11. A compound according to any one of claims 1 to 10, wherein at least one of $R^1$ and $R^2$ is methoxy.

12. A compound according to any one of claims 1 to 11, wherein $R^{14}$ is hydrogen.

13. A compound according to claim 1, selected from the group
7-[(4,6-dimethoxy-pyrimidin-2-yl)oxy]-3-methyl-phthalide,
7-[(4,6-dimethoxy-pyrimidin-2-yl)oxy]-phthalide,
3-ethyl-7-[(4,6-dimethoxy-pyrimidin-2-yl)oxy]-phthalide,
7-[(4,6-dimethoxy-pyrimidin-2-yl)oxy]-3-isopropyl-phthalide,
7-[(4,6-dimethoxy-pyrimidin-2-yl)oxy]-3-methoxy-phthalide,
7-[(4-methoxy-6-methyl-pyrimidin-2-yl)oxy]-3-methyl-phthalide,
7-[(4,6-dimethoxy-1,3,5-triazin-2-yl)oxy]-3-methyl-phthalide,
(Z)-3-ethylidene-7-[(4,6-dimethoxy-pyrimidin-2-yl)oxy]-phthalide,
8-[(4,6-dimethoxy-pyrimidin-2-yl)oxy]-3-methyl-isochroman-1-one,
7-[(4,6-dimethoxy-pyrimidin-2-yl)thio]-3-methyl-phthalide,
3-ethoxy-7-[(4,6-dimethoxy-pyrimidin-2-yl)oxy]-phthalide,
7-[(4,6-dimethoxy-pyrimidin-2-yl)oxy]-3-methoxy-3-methyl-phthalide,
7-[(4-chloro-6-methoxy-pyrimidin-2-yl)oxy]-3-methyl-phthalide,
7-[(4-ethoxy-6-methoxy-pyrimidin-2-yl)oxy]-3-methyl-phthalide,
7-[(4,6-dimethoxy-1,3,5-triazin-2-yl)thio]-3-methyl-phthalide,
7-[(4-methoxy-6-methyl-1,3,5-triazin-2-yl)oxy]-3-methyl-phthalide,
3-ethyl-7-[(4,6-dimethoxy-1,3,5-triazin-2-yl)oxy]-phthalide,
8-[(4,6-dimethoxy-pyrimidin-2-yl)oxy]-3,4-dimethyl-isochroman-2-one,
7-[(4,6-dimethoxy-pyrimidin-2-yl)oxy]-3-methylthio-phthalide,
7-[(4,6-dimethoxy-pyrimidin-2-yl)oxy]-3-vinyl-phthalide and
3-cyano-7-[(4,6-dimetpoxy-pyrimidin-2-yl)oxy]-phthalide.

14. A compound according to claim 1, selected from the group
7-[(4,6-dimethoxy-pyrimidin-2-yl)oxy]-3,6-dimethylphthalide,
3-carbamoyl-7-[(4,6-dimethoxy-pyrimidin-2-yl)oxy]-phthalide,
3-(2-chloroethoxy)-7-[(4,6-dimethoxy-pyrimidin-2-yl)oxy]-phthalide,
7-[(4,6-dimethoxy-pyrimidin-2-yl)oxy]-3-propargyloxy-phthalide,
7-[(4,6-dimethoxy-pyrimidin-2-yl)oxy]-3-(n-propoxy)-phthalide,
7-[(4,6-dimethoxy-pyrimidin-2-yl)oxy]-3-(2-methoxy-ethoxy)-phthalide,

7-[(4-methoxy-pyrimidin-2-yl)oxy]-3-methyl-phthalide,
7-[(4-chloro-6-methoxy-pyrimidin-2-yl)oxy]-3-methoxy-phthalide,
7-[(4-dimethylamino-6-methoxy-1,3,5-triazin-2-yl)oxy]-3-methyl-phthalide,
7-[(4-methoxy-6-methylamino-1,3,5-triazin-2-yl)oxy]-3-methyl-phthalide,
7-[(4-chloro-6-methylamino-1,3,5-triazin-2-yl)oxy]-3-methyl-phthalide,
7-[(4,6-dimethoxy-pyrimidin-2-yl)oxy]-3-hydroxy-phthalide,
3-cyanomethoxy-7-[(4,6-dimethoxy-pyrimidin-2-yl)oxy]-phthalide,
7-[(4,6-dimethoxy-pyrimidin-2-yl)oxy]-3-(methoxycarbonyl-methoxy)-phthalide,
3-ethoxycarbonylmethyl-7-[(4,6-dimethoxy-pyrimidin-2-yl)oxy]-phthalide,
7-[(4,6-dimethoxy-pyrimidin-2-yl)oxy]-3-methyl-2-benzothiophen-1(3H)-one,
7-[(4,6-dimethoxy-pyrimidin-2-yl)oxy]-3-methyl-isobenzofuran-1(3H)-thione and
7-[(4,6-dimethoxy-1,3,5-triazin-2-yl)oxy]-3-methyl-isobenzofuran-1(3H)-thione.

**15.** A weed control composition and plant-growth-regulating composition which comprises an effective amount of at least one compound of the general formula

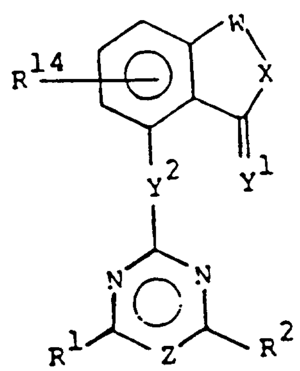

I

wherein

W is one of the divalent groups a) - d)

a) 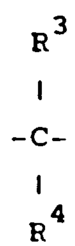

b) 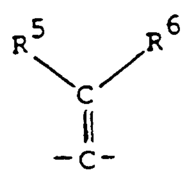

c) 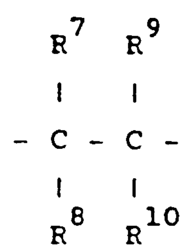

d) 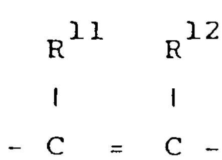

| | |
|---|---|
| X, $Y^1$ and $Y^2$ | are each oxygen or sulfur, |
| Z | is $CR^{13}$ or nitrogen, |
| $R^1$ | is hydrogen, fluorine, chlorine, $C_{1-3}$alkyl, halomethyl, methoxymethyl, $C_{1-3}$alkoxy, difluoromethoxy or methylthio, |
| $R^2$ | is methyl, $C_{1-2}$alkoxy, $C_{1-2}$fluoroalkoxy, $C_{1-2}$alkylamino, di($C_{1-2}$alkyl)amino or N-methoxymethylamino, |
| $R^3$ | is hydrogen, fluorine, chlorine, bromine, $C_{1-6}$alkyl, $C_{2-3}$alkenyl, $C_{2-3}$alkynyl; |

$C_{1-6}$alkyl substituted by halogen, hydroxy, methoxy, ethoxy, nitro, cyano, vinyl, ethynyl, carboxy, $C_{2-5}$alkoxycarbonyl, or by phenyl that may in turn be substituted by methoxy; $C_{2-3}$alkenyl, $C_{2-3}$alkynyl, phenyl, or phenyl substituted by fluorine, chlorine, methyl, methoxy or by trifluoromethyl; hydroxy, $C_{1-6}$alkoxy, or $C_{1-6}$-alkoxy substituted by halogen, vinyl, ethynyl, cyclopropyl, phenyl, $C_{1-2}$alkoxy, $C_{1-2}$alkylthio, cyano, carboxy, $C_{2-5}$alkoxycarbonyl, carbamoyl, N-($C_{1-2}$alkyl)carbamoyl, N,N-di($C_{1-2}$alkyl)carbamoyl or by $C_{3-5}$alkylideneiminooxy; $C_{1-6}$alkylthio, phenoxy, phenylthio, cyano, thiocyano, formyl, carboxy, $C_{2-5}$alkoxycarbonyl, carbamoyl, formyloxy, $C_{2-5}$-alkanoyloxy, $C_{2-5}$alkoxycarbonyloxy, $C_{2-3}$alkylcarbamoyloxy, di($C_{1-2}$alkyl)carbamoyloxy or di($C_{1-2}$-alkoxy)phosphonyl,

$R^4$ is hydrogen, $C_{1-6}$alkyl or trifluoromethyl,

$R^5$ is hydrogen, $C_{1-6}$alkyl, phenyl, or phenyl substituted by fluorine, chlorine, methyl, methoxy or by trifluoromethyl;

$R^6$ is hydrogen or methyl,

$R^7$, $R^8$ and $R^9$ each independently of the others is hydrogen or $C_{1-3}$alkyl,

$R^{10}$ is hydrogen or $C_{1-3}$alkoxy,

$R^{11}$ and $R^{12}$ each independently of the other is hydrogen or $C_{1-3}$alkyl,

$R^{13}$ is hydrogen, fluorine, chlorine or methyl

and

$R^{14}$ is hydrogen, halogen, $C_{1-2}$alkyl or $C_{1-3}$alkoxy, and formulation adjuvants.

**16.** A weed control composition according to claim 15, which comprises an effective amount of at least one compound selected from the group
7-[(4,6-dimethoxy-pyrimidin-2-yl)oxy]-3-methyl-phthalide,
7-[(4,6-dimethoxy-pyrimidin-2-yl)oxy]-phthalide,
3-ethyl-7-[(4,6-dimethoxy-pyrimidin-2-yl)oxy]-phthalide,
7-[(4,6-dimethoxy-pyrimidin-2-yl)oxy]-3-isopropyl-phthalide,
7-[(4,6-dimethoxy-pyrimidin-2-yl)oxy]-3-methoxy-phthalide,
7-[(4-methoxy-6-methyl-pyrimidin-2-yl)oxy]-3-methyl-phthalide,
7-[(4,6-dimethoxy-1,3,5-triazin-2-yl)oxy]-3-methyl-phthalide,
(Z)-3-ethylidene-7-[(4,6-dimethoxy-pyrimidin-2-yl)oxy]-phthalide,
8-[(4,6-dimethoxy-pyrimidin-2-yl)oxy]-3-methyl-isochroman-1-one,
7-[(4,6-dimethoxy-pyrimidin-2-yl)thio]-3-methyl-phthalide,
3-ethoxy-7-[(4,6-dimethoxy-pyrimidin-2-yl)oxy]-phthalide,
7-[(4,6-dimethoxy-pyrimidin-2-yl)oxy]-3-methoxy-3-methyl-phthalide,
7-[(4-chloro-6-methoxy-pyrimidin-2-yl)oxy]-3-methyl-phthalide,
7-[(4-ethoxy-6-methoxy-pyrimidin-2-yl)oxy]-3-methyl-phthalide,
7-[(4,6-dimethoxy-1,3,5-triazin-2-yl)thio]-3-methyl-phthalide,
7-[(4-methoxy-6-methyl-1,3,5-triazin-2-yl)oxy]-3-methyl-phthalide,
3-ethyl-7-[(4,6-dimethoxy-1,3,5-triazin-2-yl)oxy]-phthalide,
8-[(4,6-dimethoxy-pyrimidin-2-yl)oxy]-3,4-dimethyl-isochroman-2-one,
7-[(4,6-dimethoxy-pyrimidin-2-yl)oxy]-3-methylthio-phthalide,
7-[(4,6-dimethoxy-pyrimidin-2-yl)oxy]-3-vinyl-phthalide, and
3-cyano-7-[(4,6-dimethoxy-pyrimidin-2-yl)oxy]-phthalide, and formulation adjuvants.

**17.** A weed control composition and plant-growth-regulating composition according to claim 15, which comprises an effective amount of at least one compound selected from the group
7-[(4,6-dimethoxy-pyrimidin-2-yl)oxy]-3,6-dimethylphthalide,
3-carbamoyl-7-[(4,6-dimethoxy-pyrimidin-2-yl)oxy]-phthalide,
3-(2-chloroethoxy)-7-[(4,6-dimethoxy-pyrimidin-2-yl)oxy]phthalide,
7-[(4,6-dimethoxy-pyrimidin-2-yl)oxy]-3-propargyloxy-phthalide,
7-[(4,6-dimethoxy-pyrimidin-2-yl)oxy]-3-(n-propoxy)-phthalide,
7-[(4,6-dimethoxy-pyrimidin-2-yl)oxy]-3-(2-methoxy-ethoxy)-phthalide,
7-[(4-methoxy-pyrimidin-2-yl)oxy]-3-methyl-phthalide,
7-[(4-chloro-6-methoxy-pyrimidin-2-yl)oxy]-3-methoxy-phthalide,
7-[(4-dimethylamino-6-methoxy-1,3,5-triazin-2-yl)oxy]-3-methyl-phthalide,
7-[(4-methoxy-6-methylamino-1,3,5-triazin-2-yl)oxy]-3-methyl-phthalide,
7-[(4-chloro-6-methylamino-1,3,5-triazin-2-yl)oxy]-3-methyl-phthalide,

7-[(4,6-dimethoxy-pyrimidin-2-yl)oxy]-3-hydroxy-phthalide,
3-cyanomethoxy-7-[(4,6-dimethoxy-pyrimidin-2-yl)oxy]-phthalide,
7-[(4,6-dimethoxy-pyrimidin-2-yl)oxy]-3-(methoxycarbonyl-methoxy)-phthalide,
3-ethoxycarbonylmethyl-7-[(4,6-dimethoxy-pyrimidin-2-yl)oxy]-phthalide,
7-[(4,6-dimethoxy-pyrimidin-2-yl)oxy]-3-methyl-2-benzothiophen-1(3H)-one,
7-[(4,6-dimethoxy-pyrimidin-2-yl)oxy]-3-methyl-isobenzofuran-1(3H)-thione and
7-[(4,6-dimethoxy-1,3,5-triazin-2-yl)oxy]-3-methyl-isobenzofuran-1(3H)-thione,
and formulation adjuvants.

**18.** A process for the preparation of a compound of the general formula

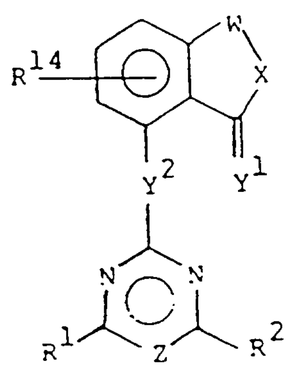

I

wherein

| | |
|---|---|
| W | is one of the divalent groups a) - d) |

a) 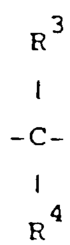 b) 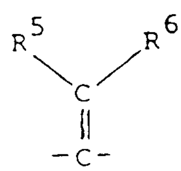 c) 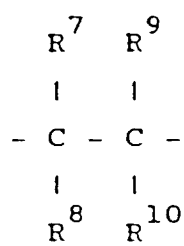

d) 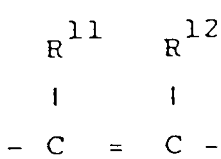

| | |
|---|---|
| X, $Y^1$ and $Y^2$ | are each oxygen or sulfur, |
| Z | is $CR^{13}$ or nitrogen, |
| $R^1$ | is hydrogen, fluorine, chlorine, $C_{1-3}$alkyl, halomethyl, methoxymethyl, $C_{1-3}$alkoxy, difluoromethoxy or methylthio, |
| $R^2$ | is methyl, $C_{1-2}$alkoxy, $C_{1-2}$fluoroalkoxy, $C_{1-2}$alkylamino, di($C_{1-2}$alkyl)amino or N-methoxymethylamino, |
| $R^3$ | is hydrogen, fluorine, chlorine, bromine, $C_{1-6}$alkyl, $C_{2-3}$alkenyl, $C_{2-3}$alkynyl; $C_{1-6}$alkyl substituted by halogen, hydroxy, methoxy, ethoxy, nitro, cyano, vinyl, ethynyl, carboxy, $C_{2-5}$alkoxycarbonyl, or by phenyl that may in turn be substituted by methoxy; $C_{2-3}$alkenyl, $C_{2-3}$alkynyl, phenyl, or phenyl substituted by fluorine, chlorine, methyl, methoxy or by trifluoromethyl; hydroxy, $C_{1-6}$alkoxy, or $C_{1-6}$-alkoxy substituted by halogen, vinyl, ethynyl, cyclopropyl, phenyl, $C_{1-2}$al- |

koxy, $C_{1-2}$alkylthio, cyano, carboxy, $C_{2-5}$alkoxycarbonyl, carbamoyl, N-($C_{1-2}$alkyl)carbamoyl, N,N-di($C_{1-2}$alkyl)carbamoyl or by $C_{3-5}$alkylideneiminooxy; $C_{1-6}$alkylthio, phenoxy, phenylthio, cyano, thiocyano, formyl, carboxy, $C_{2-5}$alkoxycarbonyl, carbamoyl, formyloxy, $C_{2-5}$-alkanoyloxy, $C_{2-5}$alkoxycarbonyloxy, $C_{2-3}$alkylcarbamoyloxy, di($C_{1-2}$alkyl)carbamoyloxy or di($C_{1-2}$-alkoxy)phosphonyl,

$R^4$ is hydrogen, $C_{1-6}$alkyl or trifluoromethyl,

$R^5$ is hydrogen, $C_{1-6}$alkyl, phenyl, or phenyl substituted by fluorine, chlorine, methyl, methoxy or by trifluoromethyl;

$R^6$ is hydrogen or methyl,

$R^7$, $R^8$ and $R^9$ each independently of the others is hydrogen or $C_{1-3}$alkyl,

$R^{10}$ is hydrogen or $C_{1-3}$alkoxy,

$R^{11}$ and $R^{12}$ each independently of the other is hydrogen or $C_{1-3}$alkyl,

$R^{13}$ is hydrogen, fluorine, chlorine or methyl

and

$R^{14}$ is hydrogen, halogen, $C_{1-2}$alkyl or $C_{1-2}$alkoxy,

which comprises reacting a compound of the general formula

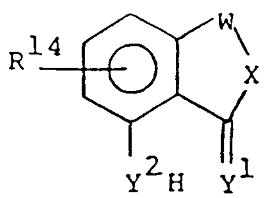

II,

wherein W, X, $Y^1$, $Y^2$ and $R^{14}$ are as defined above, with a compound of the general formula

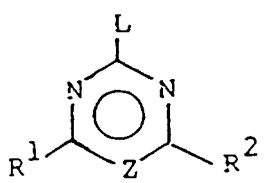

III,

wherein $R^1$, $R^2$ and Z are as defined above and L is a leaving group.

**19.** A process according to claim 18, which is used to prepare a compound of formula I wherein W is a group a) and $R^3$ is hydrogen, fluorine, chlorine, bromine, $C_{1-6}$-alkyl, $C_{2-3}$alkenyl, $C_{2-3}$alkynyl; $C_{1-6}$alkyl substituted by halogen, hydroxy, methoxy, ethoxy, nitro, cyano, vinyl, ethynyl, carboxy, $C_{2-5}$alkoxycarbonyl or by phenyl that may in turn be substituted by methoxy; $C_{2-3}$alkenyl, $C_{2-3}$alkynyl, phenyl, or phenyl substituted by fluorine, chlorine, methyl, methoxy or by trifluoromethyl; hydroxy, $C_{1-6}$alkoxy, $C_{1-6}$alkylthio, phenoxy, phenylthio, cyano or $C_{2-5}$alkoxycarbonyl and $R^4$ is hydrogen or $C_{1-6}$alkyl, or $R^3$ is hydrogen, fluorine, chlorine, bromine, $C_{1-6}$alkyl, hydroxy, $C_{1-6}$alkoxy, $C_{1-6}$alkylthio, phenoxy, phenylthio or cyano and $R^4$ is trifluoromethyl; or W is a group b), c) or d) wherein $R^5$, $R^6$, $R^7$, $R^8$, $R^9$, $R^{10}$, $R^{11}$ and $R^{12}$ are as defined in claim 19; and X is oxygen, $Y^1$, $Y^2$ and Z are as defined in claim 19, and $R^1$ is fluorine, chlorine, $C_{1-3}$alkyl, fluoromethyl, methoxymethyl, $C_{1-3}$alkoxy, difluoromethoxy or methylthio, $R^2$ is methyl, $C_{1-2}$alkoxy or $C_{1-2}$fluoroalkoxy, $R^{13}$ is hydrogen, fluorine, chlorine or methyl, and $R^{14}$ is hydrogen.

**20.** A method of controlling weeds and of regulating plant growth which comprises treating the crop to be protected against weeds and/or the weeds and the plants with an effective amount of a compound according to any one of claims 1, 3 to 6, 10, 11 and 14 or of a composition according to claim 15 or 17.

**21.** A method of controlling weeds which comprises treating the crop to be protected against weeds and/or the weeds with an effective amount of a compound according to any one of claims 2, 7 to 9, 12 and 13 or of a composition according to claim 16.

22. The use of a compound according to any one of claims 1, 3 to 6, 10, 11 and 14 or of a composition according to claim 15 or 17 for controlling weeds or for regulating plant growth.

23. The use of a compound according to any one of claims 2, 7 to 9, 12 and 13 or of a composition according to claim 16 for controlling weeds.

**Revendications**

1. Composés de formule générale

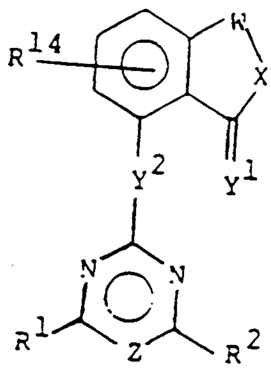

I

où

| | |
|---|---|
| W | représente un des groupes bivalents a) à d) |

a) 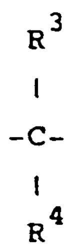

b) 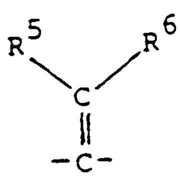

c) 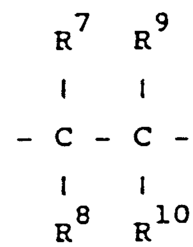

d) 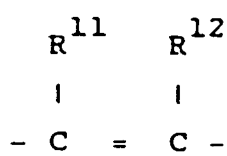

| | |
|---|---|
| X, $Y^1$ et $Y^2$ | représentent l'oxygène ou le soufre, |
| Z | $CR^{13}$ ou l'azote, |
| $R^1$ | l'hydrogène, le fluor, le chlore, un alkyle en $C_{1-3}$, un halogénométhyle, le méthoxyméthyle, un alcoxy en $C_{1-3}$, le difluorométhoxy ou le méthylthio, |
| $R^2$ | représente le méthyle, un alcoxy en $C_{1-2}$, un fluoroalcoxy en $C_{1-2}$, un alkyle en $C_{1-2}$amino, un di(alkyle en $C_{1-2}$)amino ou le N-méthoxyméthylamino, |
| $R^3$ | représente l'hydrogène, le fluor, le chlore, le brome, un alkyle en $C_{1-6}$, un alcényle en $C_{2-3}$, un alcynyle en $C_{2-3}$, un alkyle en $C_{1-6}$ substitué par un halogène, un hydroxy, le méthoxy, l'éthoxy, le nitro, le cyano, le vinyle, l'éthynyle, le carboxy, un alcoxy en $C_{2-5}$carbonyle, un phényle, ledit phényle pouvant être à son tour substitué par un méthoxy; un alcényle en $C_{2-3}$, un alcynyle en $C_{2-3}$; le phényle ou un phényle substitué par le fluor, le chlore, le méthyle, le méthoxy ou le trifluorométhyle; l'hydroxy, un alcoxy en $C_{1-6}$ ou un alcoxy en $C_{1-6}$ substitué par un halogène, le vinyle, l'éthynyle, le cyclopropyle, le phényle, un alcoxy en $C_{1-2}$thio, |

le cyano, le carboxy, un alcoxy en $C_{2-5}$carbonyle, le carbamoyle, un N-(alkyle en $C_{1-2}$)carbamoyle, un N,N-di(alkyle en $C_{1-2}$)carbamoyle ou un alkylidène en $C_{3-5}$iminooxy, un alkyle en $C_{1-6}$thio, le phénoxy, le phénylthio, le cyano, le thiocyanato, le formyle, le carboxy, un alcoxy en $C_{2-5}$carbonyle, le carbamoyle, le formyloxy, un alcanoyle en $C_{2-5}$oxy, un alcoxy en $C_{2-5}$carbonyloxy, un alkyle en $C_{2-3}$carbamoyloxy, un di(alkyle en $C_{1-2}$)carbamoyloxy ou un di(alcoxy en $C_{1-2}$)-phosphonyle,

$R^4$ représente l'hydrogène, un alkyle en $C_{1-6}$ ou le trifluorométhyle,

$R^5$ représente l'hydrogène, un alkyle en $C_{1-6}$, le phényle ou un phényle substitué par le fluor, le chlore, le méthyle, le méthoxy ou le trifluorométhyle,

$R^6$ l'hydrogène ou le méthyle,

$R^7$, $R^8$ et $R^9$ représentent, indépendamment les uns des autres, l'hydrogène ou un alkyle en $C_{1-3}$,

$R^{10}$ représente l'hydrogène ou un alcoxy en $C_{1-3}$,

$R^{11}$ et $R^{12}$ représentent, indépendamment l'un de l'autre, l'hydrogène ou un alkyle en $C_{1-3}$,

$R^{13}$ l'hydrogène, le fluor, le chlore ou le méthyle

et

$R^{14}$ l'hydrogène, un halogène, un alkyle en $C_{1-2}$ ou un alcoxy en $C_{1-2}$.

**2.** Composés selon la revendication 1 où W représente un groupe a) et $R^3$ représente l'hydrogène, le fluor, le chlore, le brome, un alkyle en $C_{1-6}$, un alcényle en $C_{2-3}$, un alcynyle en $C_{2-3}$, un alkyle en $C_{1-6}$ substitué par un halogène, un hydroxy, le méthoxy, l'éthoxy, le nitro, le cyano, le vinyle, l'éthynyle, le carboxy, un alcoxy en $C_{2-5}$carbonyle, un phényle, ledit phényle pouvant être à son tour substitué par un méthoxy; un alcényle en $C_{2-3}$, un alcynyle en $C_{2-3}$; le phényle ou un phényle substitué par le fluor, le chlore, le méthyle, le méthoxy ou le trifluorométhyle; un hydroxy, un alcoxy en $C_{1-6}$, un alkyle en $C_{1-6}$thio, un phénoxy, un phénylthio, le cyano ou un alcoxy en $C_{2-5}$carbonyle et $R^4$ représente l'hydrogène ou un alkyle en $C_{1-6}$ ou $R^3$ représente l'nydrogène, le fluor, le chlore, le brome, un alkyle en $C_{1-6}$, un hydroxy, un alcoxy en $C_{1-6}$, un alkyle en $C_{1-6}$thio, un phénoxy, un pnénylthio ou le cyano et $R^4$ représente le trifluorométhyle; ou W représente un groupe b), c) ou d) dans lesquels $R^5$, $R^6$, $R^7$, $R^8$, $R^9$, $R^{10}$, $R^{11}$ et $R^{12}$ ont les significations données dans la revendication 1; et X représente l'oxygène,
$R^{13}$ représente l'hydrogène, le fluor, le chlore ou le méthyle, $Y^1$, $Y^2$ et Z ont les signifirations données dans la revendication 1 et $R^1$ représente le fluor, le chlore, un alkyle en $C_{1-3}$, le fluorométhyle, le méthoxyméthyle, un alcoxy en $C_{1-3}$, le difluorométhoxy ou le méthylthio, $R^2$ représente le méthyle, un alcoxy en $C_{1-2}$ ou un fluoroalcoxy en $C_{1-2}$ et $R^{14}$ représente l'hydrogène.

**3.** Composés selon la revendication 1, où W représente un groupe a) dans lequel $R^3$ représente l'nydrogène, le vinyle, l'éthynyle, l'hydroxy, un alcoxy en $C_{1-4}$, un alcoxy en $C_{1-2}$ substitué par un halogène, le vinyle, l'éthynyle, un alcoxy en $C_{1-2}$, le cyano, le carboxyméthyle ou un alcoxy en $C_{2-3}$carbonylméthyle, un alkyle en $C_{1-2}$thio, le cyano, le carboxy, un alcoxy en $C_{2-3}$carbonyle ou le carbamoyle et $R^4$ représente l'hydrogène ou un alkyle en $C_{1-4}$.

**4.** Composés selon la revendication 1, où W représente un groupe b) dans lequel $R^5$ représente l'hydrogène ou un alkyle en $C_{1-3}$ et $R^6$ représente l'hydrogène.

**5.** Composés selon la revendication 1, où W représente un groupe c) dans lequel $R^7$, $R^8$ et $R^9$ représentent l'hydrogène ou le méthyle.

**6.** Composés selon la revendication 1, où W représente un groupe d) dans lequel $R^{11}$ et $R^{12}$ représentent l'hydrogène ou le méthyle.

**7.** Composés selon l'une des revendications 1 à 6, où X et $Y^1$ représentent l'oxygène.

**8.** Composés selon l'une des revendications 1 à 7, où $Y^2$ représente l'oxygène.

**9.** Composés selon l'une des revendications 1 à 8, où Z représente CH ou l'azote.

**10.** Composés selon l'une des revendications 1 à 9, où $R^1$ représente l'hydrogène, le chlore, le méthyle, le méthoxy ou le difluorométhoxy et $R^2$ représente le méthoxy, l'éthoxy, le méthylamino, le diméthylamino ou le N-méthoxyméthylamino.

**11.** Composés selon l'une des revendications 1 à 10, où au moins l'un des radicaux $R^1$ et $R^2$ représente le méthoxy.

**12.** Composés selon l'une des revendications 1 à 11, où $R^{14}$ représente l'hydrogène.

**13.** Composés selon la revendication 1, choisis dans le groupe
le 7-[(4,6-diméthoxy-pyrimidin-2-yl)oxy]-3-méthyl-phtalide,
le 7-[(4,6-diméthoxy-pyrimidin-2-yl)oxy]-phtalide,
le 3-éthyl-7-[(4,6-diméthoxy-pyrimidin-2-yl)oxy]-phtalide,
le 7-[(4,6-diméthoxy-pyrimidin-2-yl)oxy]-3-isopropyl-phtalide,
le 7-[(4,6-diméthoxy-pyrimidin-2-yl)oxy]-3-méthoxy-phtalide,
le 7-[(4-méthoxy-6-méthyl-pyrimidin-2-yl)oxy]-3-méthyl-phtalide,
le 7-[(4,6-diméthoxy-1,3,5-triazin-2-yl)oxy]-3-méthyl-phtalide,
le (Z)-3-éthylidène-7-[(4,6-diméthoxy-pyrimidin-2-yl)oxy]-phtalide,
la 8-[(4,6-diméthoxy-pyrimidin-2-yl)oxy]-3-méthyl-isochroman-1-one,
le 7-[(4,6-diméthoxy-pyrimidin-2-yl)thio]-3-méthyl-phtalide,
le 3-éthoxy-7-[(4,6-diméthoxy-pyrimidin-2-yl)oxy]-phtalide,
le 7-[(4,6-diméthoxy-pyrimidin-2-yl)oxy]-3-méthoxy-3-méthyl-phtalide,
le 7-[(4-chloro-6-méthoxy-pyrimidin-2-yl)oxy]-3-méthyl-phtalide,
le 7-[(4-éthoxy-6-méthoxy-pyrimidin-2-yl)oxy]-3-méthyl-phtalide,
le 7-[(4,6-diméthoxy-1,3,5-triazin-2-yl)thio]-3-méthyl-phtalide,
le 7-[(4-méthoxy-6-méthyl-1,3,5-triazin-2-yl)oxy]-3-méthyl-phtalide,
le 3-éthyl-7-[(4,6-diméthoxy-1,3,5-triazin-2-yl)oxy]-phtalide,
la 8-[(4,6-diméthoxy-pyrimidin-2-yl)oxy]-3,4-diméthyl-isochroman-2-one,
le 7-[(4,6-diméthoxy-pyrimidin-2-yl)oxy]-3-méthylthio-phtalide,
le 7-[(4,6-diméthoxy-pyrimidin-2-yl)oxy]-3-vinyl-phtalide et
le 3-cyano-7-[(4,6-diméthoxy-pyrimidin-2-yl)oxy]-phtalide.

**14.** Composés selon la revendication 1, choisis dans le groupe
le 7-[(4,6-diméthoxy-pyrimidin-2-yl)oxy]-3,6-diméthyl-phtalide,
le 3-carbamoyl-7-[(4,6-diméthoxy-pyrimidin-2-yl)oxy]-phtalide,
le 3-(2-chloroéthoxy)-7-[(4,6-diméthoxy-pyrimidin-2-yl)oxy]-phtalide,
le 7-[(4,6-diméthoxy-pyrimidin-2-yl)oxy]-3-propargyloxy-phtalide,
le 7-[(4,6-diméthoxy-pyrimidin-2-yl)oxy]-3-(n-propoxy)-phtalide,
le 7-[(4,6-diméthoxy-pyrimidin-2-yl)oxy]-3-(2-méthoxy-éthoxy)-phtalide,
le 7-[(4-méthoxy-pyrimidin-2-yl)oxy]-3-méthyl-phtalide,
le 7-[(4-chloro-6-méthoxy-pyrimidin-2-yl)oxy]-3-méthoxy-phtalide,
le 7-[(4-diméthylamino-6-méthoxy-1,3,5-triazin-2-yl)oxy]-3-méthyl-phtalide,
le 7-[(4-méthoxy-6-méthylamino-1,3,5-triazin-2-yl)oxy]-3-méthyl-phtalide,
le 7-[(4-chloro-6-méthylamino-1,3,5-triazin-2-yl)oxy]-3-méthyl-phtalide,
le 7-[(4,6-diméthoxy-primidin-2-yl)oxy]-3-hydroxy-phtalide,
le 3-cyanométhoxy-7-[(4,6-diméthoxy-pyrimidin-2-yl)oxy]-phtalide,
le 7-[(4,6-diméthoxy-pyrimidin-2-yl)oxy]-3-(méthoxycarbonylméthoxy)-phtalide,
le 3-éthoxycarbonylméthyl-7-[(4,6-diméthoxy-pyrimidin-2-yl)oxy]-phtalide,
la 7-[(4,6-diméthoxy-pyrimidin-2-yl)oxy]-3-méthyl-2-benzothiophén-1(3H)-one,
la 7-[(4,6-diméthoxy-pyrimidin-2-yl)oxy]3-méthylisobenzofuranne-1(3H)-thione et
la 7-[(4,6-diméthoxy-1,3,5-triazin-2-yl)oxy]-3-méthylisobenzofuranne-1(3H)-thione.

**15.** Composition pour lutter contre les mauvaises herbes et composition pour réguler la croissance des plantes, caractérisées en ce qu'elles contiennent une quantité efficace d'au moins un composé de formule générale

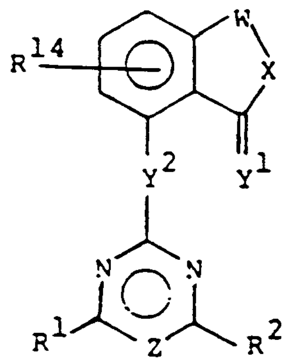

I

où

W représente un des groupes bivalents a) à d)

a) 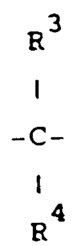

b) 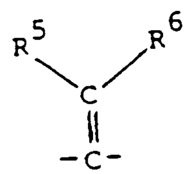

c) 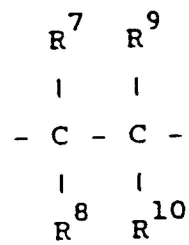

d) 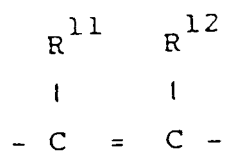

| | |
|---|---|
| X, $Y^1$ et $Y^2$ | représentent l'oxygène ou le soufre, |
| Z | $CR^{13}$ ou l'azote, |
| $R^1$ | l'hydrogène, le fluor, le chlore. un alkyle en $C_{1-3}$, un halogénométhyle, le méthoxyméthyle, un alcoxy en $C_{1-3}$, le difluorométhoxy ou le méthylthio, |
| $R^2$ | représente le méthyle, un alcoxy en $C_{1-2}$, un fluoroalcoxy en $C_{1-2}$, un alkyle en $C_{1-2}$amino, un di(alkyle en $C_{1-2}$)amino ou le N-méthoxyméthylamino, |
| $R^3$ | représente l'hydrogène, le fluor, le chlore, le brome, un alkyle en $C_{1-6}$, un alcényle en $C_{2-3}$, un alcynyle en $C_{2-3}$, un alkyle en $C_{1-6}$ substitué par un halogène, un hydroxy, le méthoxy, l'éthoxy, le nitro, le cyano, le vinyle, l'éthynyle, le carboxy, un alcoxy en $C_{2-5}$carbonyle, un phényle, ledit phényle pouvant être à son tour substitué par un méthoxy; un alcényle en $C_{2-3}$, un alcynyle en $C_{2-3}$; le phényle ou un phényle substitué par le fluor, le chlore, le méthyle, le méthoxy ou le trifluorométhyle; l'hydroxy, un alcoxy en $C_{1-6}$ ou un alcoxy en $C_{1-6}$ substitué par un halogène, le vinyle, l'éthynyle, le cyclopropyle, le phényle, un alcoxy en $C_{1-2}$, un alkyle en $C_{1-2}$thio, le cyano, le carboxy, un alcoxy en $C_{2-5}$carbonyle, le carbamoyle, un N-(alkyle en $C_{1-2}$)carbamoyle, un N,N-di(alkyle en $C_{1-2}$)carbamoyle ou un alkylidène en $C_{3-5}$iminooxy, un alkyle en $C_{1-6}$thio, le phénoxy, le phénylthio, le cyano, le thiocyanato, le formyle, le carboxy, un alcoxy en $C_{2-5}$carbonyle, le carbamoyle, le formyloxy, un alcanoyle en $C_{2-5}$oxy, un alcoxy en $C_{2-5}$carbonyloxy, un alkyle en $C_{2-3}$carbamoyloxy, un di(alkyle en $C_{1-2}$)carbamoyloxy ou un di(alcoxy en $C_{1-2}$)phosphonyle, |
| $R^4$ | représente l'hydrogène, un alkyle en $C_{1-6}$ ou le trifluorométhyle, |
| $R^5$ | représente l'hydrogène, un alkyle en $C_{1-6}$, le phényle ou un phényle substitué par le fluor, le chlore, le méthyle, le méthoxy ou le trifluorométhyle, |

| | |
|---|---|
| $R^6$ | l'hydrogène ou le méthyle, |
| $R^7$, $R^8$ et $R^9$ | représentent, indépendamment les uns des autres, l'hydrogène ou un alkyle en $C_{1-3}$, |
| $R^{10}$ | représente l'hydrogène ou un alcoxy en $C_{1-3}$, |
| $R^{11}$ et $R^{12}$ | représentent, indépendamment l'un de l'autre, l'hydrogène ou un alkyle en $C_{1-3}$, |
| $R^{13}$ | l'hydrogène, le fluor, le chlore ou le méthyle et |
| $R^{14}$ | l'hydrogène, un halogène, un alkyle en $C_{1-2}$ ou un alcoxy en $C_{1-2}$, |

ainsi que des adjuvants de formulation.

**16.** Composition pour lutter contre les mauvaises herbes, caractérisée en ce qu'elle contient une quantité efficace d'au moins l'un des composés choisi dans le groupe
le 7-[(4,6-diméthoxy-pyrimidin-2-yl)oxy]-3-méthyl-phtalide,
le 7-[(4,6-diméthoxy-pyrimidin-2-yl)oxy]-phtalide,
le 3-éthyl-7-[(4,6-diméthoxy-pyrimidin-2-yl)oxy]-phtalide,
le 7-[(4,6-diméthoxy-pyrimidin-2-yl)oxy]-3-isopropyl-phtalide,
le 7-[(4,6-diméthoxy-pyrimidin-2-yl)oxy]-3-méthoxy-phtalide,
le 7-[(4-méthoxy-6-méthyl-pyrimidin-2-yl)oxy]-3-méthyl-phtalide,
le 7-[(4,6-diméthoxy-1,3,5-triazin-2-yl)oxy]-3-méthyl-phtalide,
le (Z)-3-éthylidène-7-[(4,6-diméthoxy-pyrimidin-2-yl)oxy]-phtalide,
la 8-[(4,6-diméthoxy-pyrimidin-2-yl)oxy]-3-méthyl-isochroman-1-one,
le 7-[(4,6-diméthoxy-pyrimidin-2-yl)thio]-3-méthyl-phtalide,
le 3-éthoxy-7-[(4,6-diméthoxy-pyrimidin-2-yl)oxy]-phtalide,
le 7-[(4,6-diméthoxy-pyrimidin-2-yl)oxy]-3-méthoxy-3-méthyl-phtalide,
le 7-[(4-chloro-6-méthoxy-pyrimidin-2-yl)oxy]-3-méthyl-phtalide,
le 7-[(4-éthoxy-6-méthoxy-pyrimidin-2-yl)oxy]-3-méthyl-phtalide,
le 7-[(4,6-diméthoxy-1,3,(-triazin-2-yl)thio]-3-méthyl-phtalide,
le 7-[(4-méthoxy-6-méthyl-1,3,(-triazin-2-yl)oxy]-3-méthyl-phtalide,
le 3-éthyl-7-[(4,6-diméthoxy-1,3,5-triazin-2-yl)oxy]-phtalide,
la 8-[(4,6-diméthoxy-pyrimidin-2-yl)oxy]-3,4-diméthylisochroman-2-one,
le 7-[(4,6-diméthoxy-pyrimidin-2-yl)oxy]-3-méthylthio-phtalide,
le 7-[(4,6-diméthoxy-pyrimidin-2-yl)oxy]-3-vinyl-phtalide et le 3-cyano-7-[(4,6-diméthoxy-pyrimidin-2-yl)-oxy]-phtalide,
ainsi que des adjuvants de formulation.

**17.** Composition pour lutter contre les mauvaises herbes et composition pour réguler la croissance des plantes, caractérisées en ce qu'elles contiennent une quantité efficace d'au moins l'un des composés choisi dans le groupe
le 7-[(4,6-diméthoxy-pyrimidin-2-yl)oxy]-3,6-diméthyl-phtalide,
le 3-carbamoyl-7-[(4,6-diméthoxy-pyrimidin-2-yl)oxy]-phtalide,
le 3-(2-chloroéthoxy)-7-[(4,6-diméthoxy-pyrimidin-2-yl)-oxy]-phtalide,
le 7-[(4,6-diméthoxy-pyrimidin-2-yl)oxy]-3-propargyloxy-phtalide,
le 7-[(4,6-diméthoxy-pyrimidin-2-yl)oxy]-3-(n-propoxy)-phtalide,
le 7-[(4,6-diméthoxy-pyrimidin-2-yl)oxy]-3-(2-méthoxy-éthoxy)-phtalide,
le 7-[(4-méthoxy-pyrimidin-2-yl)oxy]-3-méthyl-phtalide,
le 7-[(4-chloro-6-méthoxy-pyrimidin-2-yl)oxy]-3-méthoxy-phtalide,
le 7-[(4-diméthylamino-6-méthoxy-1,3,5-triazin-2-yl)oxy]-3-méthyl-phtalide,
le 7-[(4-méthoxy-6-méthylamino-1,3,5-triazin-2-yl)oxy]-3-méthyl-phtalide,
le 7-[(4-chloro-6-méthylamino-1,3,5-triazin-2-yl)oxy]-3-méthyl-phtalide,
le 7-[(4,6-diméthoxy-pyrimidin-2-yl)oxy]-3-hydroxyphtalide,
le 3-cyanométhoxy-7-[(4,6-diméthoxy-pyrimidin-2-yl)oxy]-phtalide,
le 7-[(4,6-diméthoxy-pyrimidin-2-yl)oxy]-3-(méthoxy-carbonylméthoxy)-phtalide,
le 3-éthoxycarbonylméthyl-7-[(4,6-diméthoxy-pyrimidin-2-yl)oxy]-phtalide,
la 7-[(4,6-diméthoxy-pyrimidin-2-yl)oxy]-3-méthyl-2-benzothiophén-1(3H)-one,
la 7-[(4,6-diméthoxy-pyrimidin-2-yl)oxy]-3-méthylisobenzofuranne-1(3H)-thione et
la 7-[(4,6-diméthoxy-1,3,5-triazin-2-yl)oxy]-3-méthylisobenzofuranne-1(3H)-thione,
ainsi que des adjuvants de formulation.

**18.** Procédé pour la préparation des composés de formule générale

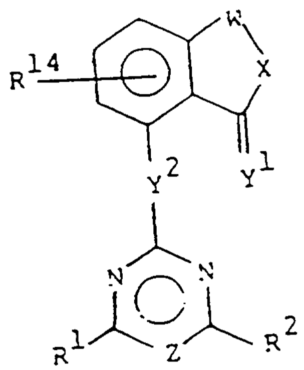

I

où

W représente un des groupes bivalents a) à d)

a) 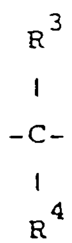

b) 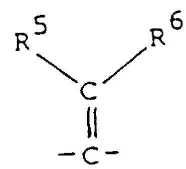

c) 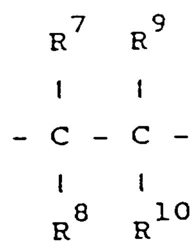

d) 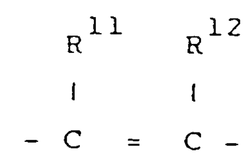

X, $Y^1$ et $Y^2$ représentent l'oxygène ou le soufre,

Z $CR^{13}$ ou l'azote,

$R^1$ l'hydrogéne, le fluor, le chlore, un alkyle en $C_{1-3}$, un halogénométhyle, le méthoxyméthyle, un alcoxy en $C_{1-3}$, le difluorométhoxy ou le méthylthio,

$R^2$ représente le méthyle, un alcoxy en $C_{1-2}$, un fluoroalcoxy en $C_{1-2}$, un alkyle en $C_{1-2}$amino, un di(alkyle en $C_{1-2}$)amino ou le N-méthoxyméthylamino,

$R^3$ représente l'hydrogène, le fluor, le chlore, le brome, un alkyle en $C_{1-6}$, un alcényle en $C_{2-3}$, un alcynyle en $C_{2-3}$, un alkyle en $C_{1-6}$ substitué par un halogène, un hydroxy, le méthoxy, l'éthoxy, le nitro, le cyano, le vinyle, l'éthynyle, le carboxy, un alcoxy en $C_{2-5}$carbonyle, un phényle, ledit phényle pouvant être à son tour substitué par un méthoxy; un alcényle en $C_{2-3}$, un alcynyle en $C_{2-3}$; le phényle ou un phényle substitué par le fluor, le chlore, le méthyle, le méthoxy ou le trifluorométhyle; l'hydroxy, un alcoxy en $C_{1-6}$ ou un alcoxy en $C_{1-6}$ substitué par un halogène, le vinyle, l'éthynyle, le cyclopropyle, le phényle, un alcoxy en $C_{1-2}$, un alkyle en $C_{1-2}$thio, le cyano, le carboxy, un alcoxy en $C_{2-5}$carbonyle, le carbamoyle, un N-(alkyle en $C_{1-2}$)carbamoyle, un N,N-di(alkyle en $C_{1-2}$)carbamoyle ou un alkylidène en $C_{3-5}$iminooxy, un alkyle en $C_{1-6}$thio, le phénoxy, le phénylthio, le cyano, le thiocyanato, le formyle, le carboxy, un alcoxy en $C_{2-5}$carbonyle, le carbamoyle, le formyloxy, un alcanoyle en $C_{2-5}$oxy, un alcoxy en $C_{2-5}$carbonyloxy, un alkyle en $C_{2-3}$carbamoyloxy, un di(alkyle en $C_{1-2}$)carbamoyloxy ou un di(alcoxy en $C_{1-2}$)-phosphonyle,

$R^4$ représente l'hydrogène, un alkyle en $C_{1-6}$ ou le trifluorométhyle,

$R^5$ représente l'hydrogène, un alkyle en $C_{1-6}$, le phényle ou un phényle substitué par le fluor, le chlore, le méthyle, le méthoxy ou le trifluorométhyle,

caractérisé en ce que l'on fait réagir un composé de formule générale

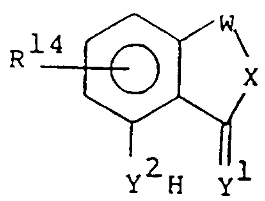

II

où W, X, $Y^1$, $Y^2$ et $R^{14}$ ont les significations données ci-dessus, avec un composé de formule générale

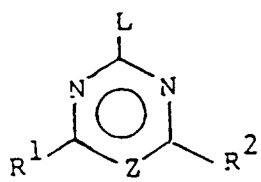

III

où $R^1$, $R^2$ et Z ont les significations données ci-dessus et L représente un groupe partant.

**19.** Procédé selon la revendication 18, caractérisé en ce qu'il sert à la préparation d'un composé de formule I où W représente un groupe a) et $R^3$ représente l'hydrogène, le fluor, le chlore, le brome, un alkyle en $C_{1-6}$, un alcényle en $C_{2-3}$, un alcynyle en $C_{2-3}$, un alkyle en $C_{1-6}$ substitué par un halogène, un hydroxy, le méthoxy, l'éthoxy, le nitro, le cyano, le vinyle, l'éthynyle, le carboxy, un alcoxy en $C_{2-5}$carbonyle, un phényle, ledit phényle pouvant être à son tour substitué par un méthoxy; un alcényle en $C_{2-3}$, un alcynyle en $C_{2-3}$; le phényle ou un phényle substitué par le fluor, le chlore, le méthyle, le méthoxy ou le trifluorométhyle, un hydroxy, un alcoxy en $C_{1-6}$, un alkyle en $C_{1-6}$thio, un phénoxy, un phénylthio, le cyano ou un alcoxy en $C_{2-5}$carbonyle et $R^4$ représente l'hydrogène ou un alkyle en $C_{1-6}$ ou $R^3$ représente l'hydrogène, le fluor, le chlore, le brome, un alkyle en $C_{1-6}$, un hydroxy, un alcoxy en $C_{1-6}$, un alkyle en $C_{1-6}$thio, un phénoxy, un phénylthio ou le cyano et $R^4$ représente le trifluorométhyle; ou W représente un groupe b), c) ou d) dans lesquels $R^5$, $R^6$, $R^7$, $R^8$, $R^9$, $R^{10}$, $R^{11}$ et $R^{12}$ ont les significations données dans la revendication 19 ; et X représente l'oxygène, $Y^1$, $Y^2$ et Z ont les significations données dans la revendication 19 et $R^1$ représente le fluor, le chlore, un alkyle en $C_{1-3}$, le fluorométhyle, le méthoxyméthyle, un alcoxy en $C_{1-3}$, le difluorométhoxy ou le méthylthio, $R^2$ représente le méthyle, un alcoxy en $C_{1-2}$ ou un fluoroalcoxy en $C_{1-2}$, $R^{13}$ représente le fluor, le chlore ou le méthyle et $R^{14}$ représente l'hydrogène.

**20.** Procédé pour lutter contre les mauvaises herbes et pour réguler la croissance des plantes, caractérisé en ce que l'on traite le produit à protéger contre les mauvaises herbes et/ou les mauvaises herbes ou les plantes avec une quantité efficace d'un composé selon l'une des revendications 1, 3 à 6, 10, 11 et 14 ou d'une composition selon la revendication 15 ou 17.

**21.** Procédé pour lutter contre les mauvaises herbes, caractérisé en ce que l'on traite le produit à protéger contre les mauvaises herbes et/ou les mauvaises herbes avec une quantité efficace d'un composé selon l'une des revendications 2, 7 à 9, 12 et 13 ou d'une composition selon la revendication 16.

**22.** Utilisation d'un composé selon l'une des revendications 1, 3 à 6, 10, 11 et 14 ou d'une composition selon la revendication 15 ou 17, pour lutter contre les mauvaises herbes ou pour réguler la croissance des plantes.

**23.** Utilisation d'un composé selon l'une des revendications 2, 7 à 9, 12 et 13 ou d'une composition selon la revendication 16, pour lutter contre les mauvaises herbes.